# EUROPEAN PATENT SPECIFICATION

(11) **EP 4 153 181 B1**
(45) Date of publication and mention of the grant of the patent: **29.07.2026**
(21) Application number: 21734578.4
(22) Date of filing: 20.05.2021
(51) Int. Cl.: A61K 31/553, A61K 45/00, A61K 9/00, A61P 31/18, A61K 9/10, A61K 47/14, A61K 9/107

(54) **PHARMACEUTICAL COMPOSITIONS COMPRISING BICTEGRAVIR**
PHARMAZEUTISCHE ZUSAMMENSETZUNGEN MIT BICTEGRAVIR
COMPOSITIONS PHARMACEUTIQUES CONTENANT DU BICTÉGRAVIR

(30) Priority: 21.05.2020 US 202063028277 P
(43) Date of publication of application: 29.03.2023
(73) Proprietor: GILEAD SCIENCES, INC., Foster City, California 94404 (US)
(72) Inventor: DE LA PAZ, Liliana, Fremont, California 94538 (US); NEJATI, Elham, Foster City, California 94404 (US); SHIH, Hui-Wen, Foster City, California 94404 (US); SUBRAMANIAN, Raju, Foster City, California 94404 (US); TANG, Huiqin, Foster City, California 94404 (US)
(74) Representative: Carpmaels & Ransford LLP
(86) International application number: PCT/US2021/033433
(87) International publication number: WO 2021/236944

(56) References cited:
- WO-A1-2020/061163
- ZHOU TIAN ET AL: "Optimizing the preparation and stability of decorated antiretroviral drug nanocrystals", NANOMEDICINE, vol. 13, no. 8, 19 March 2018 (2018-03-19), GB, pages 871 - 885, XP055837046, ISSN: 1743-5889, Retrieved from the Internet <URL:https://www.ncbi.nlm.nih.gov/pmc/articles/PMC5992566/pdf/nnm-13-871.pdf> DOI: 10.2217/nnm-2017-0381
- MANDAL SUBHRA ET AL: "A potential long-acting bictegravir loaded nano-drug delivery system for HIV-1 infection: A proof-of-concept study", ANTIVIRAL RESEARCH, vol. 167, 1 July 2019 (2019-07-01), NL, pages 83 - 88, XP055837105, ISSN: 0166-3542, Retrieved from the Internet <URL:https://www.ncbi.nlm.nih.gov/pmc/articles/PMC7263829/pdf/nihms-1585294.pdf> DOI: 10.1016/j.antiviral.2019.04.007
- KETAN HIPPALGAONKAR ET AL: "Injectable Lipid Emulsions-Advancements, Opportunities and Challenges", AAPS PHARMSCITECH, vol. 11, no. 4, 26 October 2010 (2010-10-26), pages 1526 - 1540, XP055202451, DOI: 10.1208/s12249-010-9526-5
- JOUYBAN ABOLGHASEM ET AL: "Review of pharmaceutical applications of N-methyl-2-pyrrolidone", vol. 13, no. 4, 1 January 2010 (2010-01-01), pages 524 - 535, XP008165552, ISSN: 1482-1826, Retrieved from the Internet <URL:http://ejournals.library.ualberta.ca/index.php/JPPS/article/view/8257/7452> DOI: 10.18433/J3P306
- SOUGATA PRAMANICK ET AL: "Excipient Selection In Parenteral Formulation Development", PHARMA TIMES, vol. 45, no. 3, 1 March 2013 (2013-03-01), pages 65 - 77, XP055449954

## Description

### FIELD

The present invention provides pharmaceutical compositions consisting of:

| **Component** | **% w/w** |
|---|---|
| bictegravir, free acid | 18-28 |
| povidone K-12 | 1.0-2.0 |
| polysorbate 20 | 0.3-0.5 |
| sodium chloride | 0.5-0.7 |
| sodium phosphate buffer | 0.1-0.3 |
| water | 70-80. |

The present invention also provides said pharmaceutical compositions for use in methods of treating or preventing HIV in a subject comprising administering to the subject a therapeutically effective amount of said pharmaceutical composition by injection, optionally in combination with one or more other therapeutic agents.

### BACKGROUND

HIV-1 infection is a life threatening and serious disease of major public health significance, with approximately 38 million people infected worldwide and approximately 22 million taking antiretroviral (ARV) treatment (Joint United Nations Programme on HIV/AIDS (UNAIDS). UNAIDS Data. Geneva, Switzerland. 2018). Standard of care for the treatment of HIV-1 infection uses combination ARV therapy (ART) to suppress viral replication to below detectable limits, allow cluster determinant 4 (CD4) cell counts to increase, and stop disease progression. For ART-naive, HIV-infected patients, current treatment guidelines suggest that initial therapy consist of 2 nucleos(t)ide reverse transcriptase inhibitors (N[t]RTIs) and preferably an integrase strand-transfer inhibitor (INSTI) (Department of Health and Human Services (DHHS), Panel on Antiretroviral Therapy and Medical Management of HIV-Infected Children. www.aidsinfo.nih.gov/guidelines. May 22. 2018; Gunthard HF, et al. Antiretroviral Drugs for Treatment and Prevention of HIV Infection in Adults: 2016 Recommendations of the International Antiviral Society-USA Panel. JAMA 2016;316 (2):191-210; The Department of Health and Human Services (DHHS) Panel on Antiretroviral Guidelines for Adults and Adolescents; www.aidsinfo.nih.gov/ContentFiles/AdultandAdolescentGL 25 October 2018).

Virologically suppressed, HIV-infected patients can benefit by switching from their current regimen to improve safety or tolerability or to simplify the regimen (European AIDS Clinical Society (EACS). The EACS Guidelines Version 10. November. 2019; The Department of Health and Human Services (DHHS) Panel on Antiretroviral Guidelines for Adults and Adolescents).

While current combination ARV therapy for the treatment of HIV-1 infection is efficacious and well tolerated, these agents need to be taken every day and require adherence to minimize the emergence of drug-resistant variants. As a result, "treatment fatigue" can occur, defined as "decreased desire and motivation to maintain vigilance in adhering to a treatment regimen" among people living with HIV (PLWH) prescribed chronic or life-long treatment (Claborn KR, et al., A Systematic Review Of Treatment Fatigue Among HIV-Infected Patients Prescribed Antiretroviral Therapy. Psychology, health & medicine 2015;20 (3):1-11), which can lead to nonadherence and treatment failure. As such, there remains a medical need for ARVs that can be administered less frequently (i.e., long-acting drug products), thereby providing an alternative treatment option for PLWH.

Mandal et al.: "A potential long-acting bictegravir loaded nano-drug delivery system for HIV-1 infection: A proof of concept study" investigates the HIV prevention potency of a long-acting BIC nano-formulation aimed to improve adherence.

WO 2020/061163 A1 discloses methods of preventing HIV in a subject, comprising administering to the subject a therapeutically effective amount of bictegravir, or a pharmaceutically acceptable salt thereof, optionally in combination with one or more additional therapeutic agents. Methods of reducing the risk of acquiring HIV (e.g. HIV-1 and/or HIV-2) are also provided.

Hippalgaonkar et al.: "Injectable Lipid Emulsions-Advancements, Opportunities and Challenges" provides a broad overview of recent advancements in the field of novel lipids, opportunities for intravenous drug delivery, and challenges associated with injectable lipid emulsions.

Jouyban et al.: "Review of pharmaceutical applications of N-Methyl-2-Pyrrolidone" discusses N-Methyl-2-Pyrrolidone's physicochemical characteristics, its applications - especially in the pharmaceutical sciences, pharmacokinetics and toxicity.

### SUMMARY

The present invention provides pharmaceutical compositions consisting of:

| **Component** | **% w/w** |
|---|---|
| bictegravir, free acid | 18-28 |
| povidone K-12 | 1.0-2.0 |
| polysorbate 20 | 0.3-0.5 |
| sodium chloride | 0.5-0.7 |
| sodium phosphate buffer | 0.1-0.3 |
| water | 70-80. |

The pharmaceutical compositions may be used in methods of treating or preventing HIV in a subject comprising administering to the subject a therapeutically effective amount of said pharmaceutical composition by injection, optionally in combination with one or more other therapeutic agents.

In particular, the pharmaceutical compositions described herein are long acting formulations comprising bictegravir.

Described herein are methods for the preparation of long acting formulations of bictegravir and uses of the long acting formulations as therapeutic or prophylactic agents.

### BRIEF DESCRIPTION OF THE DRAWINGS

**FIG. 1** shows the pharmacokinetics of bictegravir (BIC) following subcutaneous administration of NMP solution formulation to rat (30 mg/kg), dog (10 mg/kg), and monkey (100 mg) (Mean ± SD, n = 3) (see Example 1).
**FIG. 2** shows the pharmacokinetics of BIC following subcutaneous administration of PVP suspension formulation to rat (30 mg/kg), dog (100 mg), and monkey (100 mg) (Mean ± SD, n = 3) (see Example 1).
**FIG. 3** shows the pharmacokinetics of BIC following subcutaneous administration of HPMC suspension formulation to rat (30 mg/kg), dog (100 mg/kg), and monkey (100 mg) (Mean ± SD, n = 3) (see Example 1).
**FIG. 4** shows the pharmacokinetics of BIC following subcutaneous administration of Miglyol suspension formulation to rat (53.5 mg/kg) and monkey (446 mg) (Mean ± SD, n = 3 or 4) (see Example 1).
**FIG. 5** shows the high level schema of a Ph 1 clinical study to evaluate BIC LAI formulations in healthy human subjects.

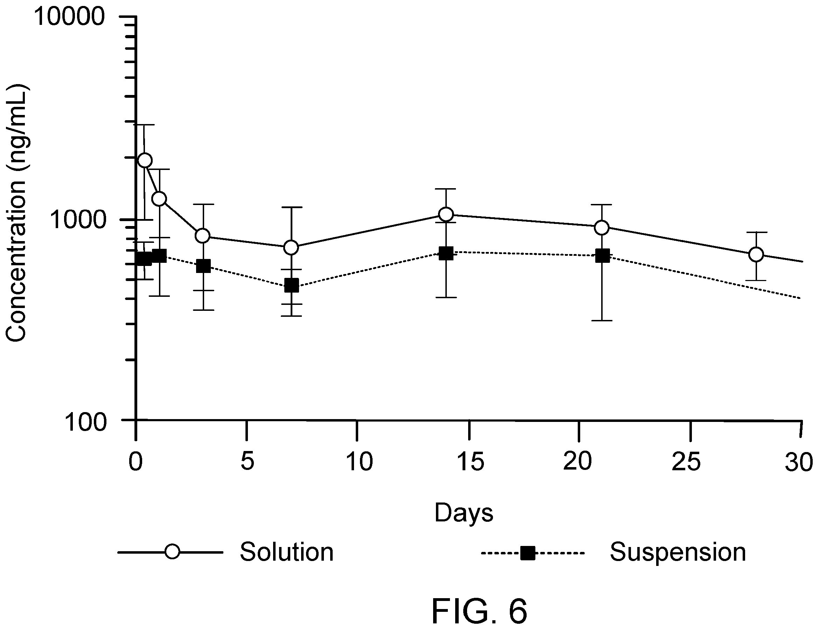

**FIG. 6** shows the plasma concentration of BIC administered as aqueous PVP suspension or NMP solution over 30 days post dose to cynomolgus monkey (see Example 1).

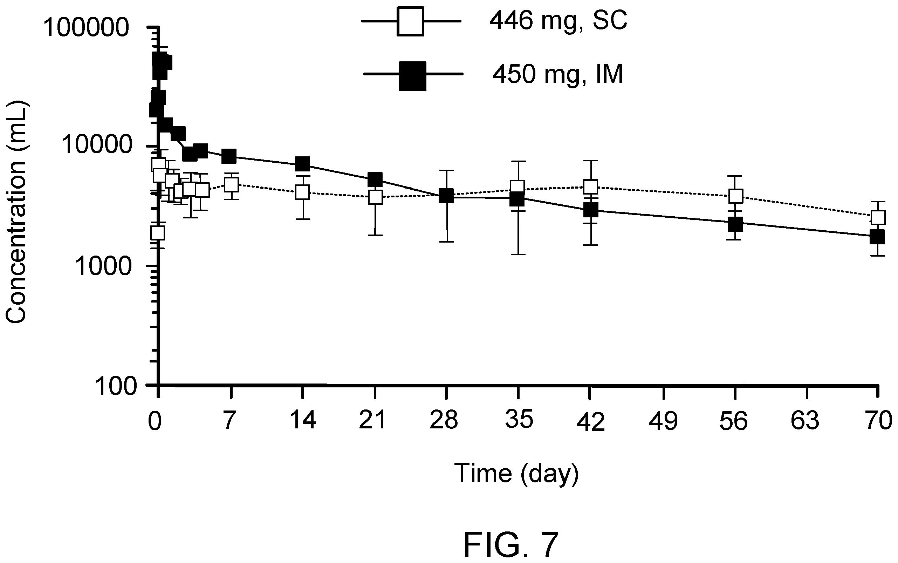

**FIG. 7** shows the plasma pharmacokinetics of BIC administered as Miglyol microsuspension over 70 days post dose subcutaneously or intramuscularly to cynomolgus monkey (see Example 1).
**FIG. 8** shows the thermodynamic solubility profiles for BIC sodium and BIC free acid forms I and III in aqueous media as a function of pH.
**FIG. 9** shows the dissolution profile for BIC free acid Form I, BIC free acid Form III, and BIC sodium in 0.01N HCl at pH of 2.
**FIG. 10** shows the pharmacokinetics of BIC free acid form III solution at 300 mg/mL; BIC free acid form III suspension at 250 mg/mL; and BIC sodium salt nanosuspension at 300 mg/mL following subcutaneous administration in a rat.
**FIG. 11** shows the pharmacokinetics of BIC free acid form III as oil suspensions in Miglyol 812 or sesame oil at 250 mg/mL following subcutaneous administration in a rat.
**FIG. 12** shows the XRPD pattern for (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form IX.
**FIG. 13** shows the DSC for (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form IX.
**FIG. 14** shows the XRPD pattern for (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form X.
**FIG. 15** shows the DSC for (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form X.
**FIG. 16** shows the DSC for (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form X isolated by heating (2R,5S,13aR)-7,9-dioxo-10-((2,4,6-trifluorobenzyl)carbamoyl)-2,3,4,5,7,9,13,13a-octahydro-2,5-methanopyrido[1',2':4,5]pyrazino[2,1-b][1,3]oxazepin-8-olate Free Acid Form IX to 210 °C.
**FIG. 17** shows the *in vitro* dissolution profile curves of BIC FA Form III PLGA formulations.
**FIG. 18** shows the pharmacokinetics of BIC administered as PLGA and HPC solid dispersion formulations to dog (100 mg), NMP solution formulation (300 mg), and PVP suspension formulation (250 mg).

### DETAILED DESCRIPTION

The present invention relates to pharmaceutical compositions consisting of:

| **Component** | **% w/w** |
|---|---|
| bictegravir, free acid | 18-28 |
| povidone K-12 | 1.0-2.0 |
| polysorbate 20 | 0.3-0.5 |
| sodium chloride | 0.5-0.7 |
| sodium phosphate buffer | 0.1-0.3 |
| water | 70-80 |

(hereinafter refer to as the 'pharmaceutical compositions'). The pharmaceutical compositions may be used in a method of treating or preventing an HIV infection (e.g., HIV-1 and/or HIV-2) in a subject (e.g., a human) by administering to the subject a therapeutically effective amount of a pharmaceutical composition. In some embodiments, a pharmaceutical composition is administered as a monotherapy (i.e., in the absence of an additional therapeutic agent). In some embodiments, a pharmaceutical composition is administered in combination with one or more other therapeutic agents, such as anti-HIV agents. In some embodiments, a pharmaceutical composition is administered as a long acting injectable.

### I. Definitions

Unless the context requires otherwise, throughout the present description and claims, the word "comprise" and variations thereof, such as, "comprises" and "comprising" are to be construed in an open, inclusive sense, that is as "including, but not limited to".

Reference throughout this specification to "one embodiment" or "an embodiment" means that a particular feature, structure or characteristic described in connection with the embodiment is included in at least one embodiment described herein. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

The d₉₀ values referred herein describe the size where ninety percent of particles in a sample have a smaller particle size than the specified d₉₀ value. For example, a d₉₀ of about 4 µm, means that 90% of the particles in the sample are smaller than 4 µm. Likewise, any d₅₀ values specified herein describe the size such that that 50% of particles in the sample are smaller than the specified d₅₀ value. Similarly, any d₁₀ values listed herein are the size at which 10% of the particles in the sample have a size smaller than this value.

As used herein, "bictegravir" or "BIC" each refer to the integrase inhibitor drug compound (2R,5S,13aR)-8-hydroxy-7,9-dioxo-N-(2,4,6-trifluorobenzyl)-2,3,4,5,7,9,13,13a-octahydro-2,5-methanopyrido[1',2':4,5]pyrazino[2,1-b][1,3]oxazepine-10-carboxamide (IUPAC name) represented by the below structure (Formula (I)). Bictegravir is described in U.S. Patent No.: 9,216,996.

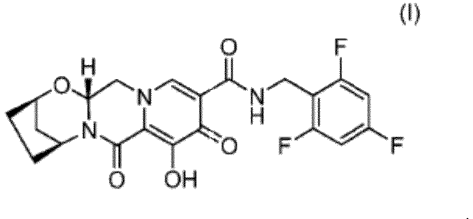

"Pharmaceutically acceptable" refers to compounds, salts, compositions, dosage forms and other materials which are useful in preparing a pharmaceutical composition that is suitable for veterinary or human pharmaceutical use.

"Pharmaceutically acceptable excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" refers to a salt of a compound that is pharmaceutically acceptable and that possesses (or can be converted to a form that possesses) the desired pharmacological activity of the parent compound. Such salts include acid addition salts formed with inorganic acids, and salts formed when an acidic proton present in the parent compound is replaced by either a metal ion, e.g., an alkali metal ion, an alkaline earth ion, or an aluminum ion; or coordinates with an organic base such as diethanolamine, triethanolamine, N-methylglucamine and the like. Also included in this definition are ammonium and substituted or quaternized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, Table 38-5.

"Treatment" or "treating" is an approach for obtaining beneficial or desired results including clinical results. Beneficial or desired clinical results may include one or more of the following: a) inhibiting the disease or condition (e.g., decreasing one or more symptoms resulting from the disease or condition, and/or diminishing the extent of the disease or condition); b) slowing or arresting the development of one or more clinical symptoms associated with the disease or condition (e.g., stabilizing the disease or condition, preventing or delaying the worsening or progression of the disease or condition, and/or preventing or delaying the spread (e.g., metastasis) of the disease or condition); and/or c) relieving the disease, that is, causing the regression of clinical symptoms (e.g., ameliorating the disease state, providing partial or total remission of the disease or condition, enhancing effect of another medication, delaying the progression of the disease, increasing the quality of life, and/or prolonging survival).

As used herein, the terms "prevention" or "preventing" refers to the administration of a compound, composition, or pharmaceutically salt according to the present disclosure pre- or post-exposure of the human to the virus but before the appearance of symptoms of the disease, and/or prior to the detection of the virus in the blood. The terms also refer to prevention of the appearance of symptoms of the disease and/or to prevent the virus from reaching detectible levels in the blood. The term includes both pre-exposure prophylaxis (PrEP), as well as post-exposure prophylaxis (PEP) and event driven or "on demand" prophylaxis. The term also refers to prevention of perinatal transmission of HIV from mother to baby, by administration to the mother before giving birth and to the child within the first days of life. The term also refers to prevention of transmission of HIV through blood transfusion.

"Subject" refers to an animal, such as a mammal (e.g., a human), that has been or will be the object of treatment, observation or experiment. The methods described herein may be useful in human therapy and/or veterinary applications. In some embodiments, the subject is a mammal. In one embodiment, the subject is a human.

"Solvate" as used herein refers to an aggregate that comprises one or more molecules of a compound described herein with one or more molecules of solvent. The solvent may be water, in which case the solvate may be a hydrate. Alternatively, the solvent may be an organic solvent. Examples of organic solvents include, but are not limited to methanol, ethanol, acetonitrile, and dichloromethane.

The term "therapeutically effective amount" or "effective amount" of a composition or a compound or pharmaceutically acceptable salts, isomer, or a mixture thereof, described herein means an amount sufficient to effect treatment when administered to a subject, to provide a therapeutic benefit such as amelioration of symptoms or slowing of disease progression. For example, a therapeutically effective amount may be an amount sufficient to decrease a symptom of a disease or condition responsive to HIV activity. The therapeutically effective amount may vary depending on the subject, and the disease or condition being treated, the weight and age of the subject, the severity of the disease or condition, and the manner of administering, which can readily be determined by one of ordinary skill in the art.

Reference to "about" a value or parameter herein includes (and describes) embodiments that are directed to that value or parameter *per se.* In certain embodiments, the term "about" includes the indicated amount ± 10%. In other embodiments, the term "about" includes the indicated amount ± 5%. In certain other embodiments, the term "about" includes the indicated amount ± 1%. Also, the term "about X" includes description of "X".

Examples of isotopes that can be incorporated into the described compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ³⁶Cl, ¹²³I, and ¹²⁵I, respectively. These radiolabeled compounds could be useful to help determine or measure the effectiveness of the compounds, by characterizing, for example, the site or mode of action, or binding affinity to pharmacologically important site of action. Certain isotopically-labeled salts and/or co-crystals of tenofovir alafenamide, for example, those incorporating a radioactive isotope, are useful in drug and/or substrate tissue distribution studies. The radioactive isotopes tritium, i.e., ³H, and carbon-14, i.e., ¹⁴C, are particularly useful for this purpose in view of their ease of incorporation and ready means of detection.

Substitution with heavier isotopes such as deuterium, i.e., ²H, may afford certain therapeutic advantages resulting from greater metabolic stability. For example, *in vivo* half-life may increase or dosage requirements may be reduced. Thus, heavier isotopes may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled salts and/or co-crystals of bictegravir can generally be prepared by conventional techniques known to those skilled in the art.
Crystalline forms may be characterized by the interlattice plane intervals determined by an X-ray powder diffraction pattern (XRPD). Unless otherwise stated, XRPD patterns referred to herein were conducted on a diffractometer (PANanalytical XPERT-PRO, PANanalytical B.V., Almelo, Netherlands) using copper radiation (Cu Kα, λ = 1.5418 Å). Samples were prepared for analysis by depositing the powdered sample in the center of an aluminum holder equipped with a zero background plate. The generator was operated at a voltage of 45 kV and amperage of 40 mA. Slits used were Soller 0.02 rad., antiscatter 1.0°, and divergence. The sample rotation speed was 2 sec. Scans were performed from 2 to 40° 2θ during 15 min with a step size of 0.0167° 2θ. Data analysis was performed by X'Pert Highscore version 2.2c (PANalytical B.V., Almelo, Netherlands) and X'Pert data viewer version 1.2d (PANalytical B.V., Almelo, Netherlands).

### II. Bictegravir (BIC) Drug Substance

The pharmaceutical compositions described herein utilize bictegravir drug substance, wherein bictegravir is in the form of a free acid. The BIC drug substance can be crystalline, amorphous, or a combination thereof. In some embodiments, the BIC drug substance is crystalline. In some embodiments, the BIC drug substance is amorphous. The BIC drug substance may exhibit different properties in the solid form vs. in a solvent or solution as is used in the pharmaceutical compositions provided herein.
. In some embodiments, the bictegravir free acid is in an amorphous form. In some embodiments, the bictegravir free acid is in a crystalline form. Certain crystalline forms of bictegravir free acid are disclosed in U.S. Patent Nos. 9,682,084 and 10,098,886.

In certain embodiments, the BIC drug substance is the crystalline form of bictegravir free acid, wherein the crystalline form of bictegravir free acid is Form I. In some embodiments, Form I of bictegravir free acid has an XRPD pattern comprising peaks at about 13.1°, 16.2°, and 20.6° 2θ ± 0.2° 2θ. In some embodiments, Form I of bictegravir free acid has an XRPD pattern further comprising peaks at about 25.0° and 27.4° 2θ ± 0.2° 2θ. In some embodiments, Form I of bictegravir free acid has an XRPD pattern further comprising peaks at about 9.3° and 10.5° 2θ ± 0.2° 2θ. In some embodiments, Form I of bictegravir free acid has an XRPD pattern further comprising peaks at about 11.4°, 13.9°, 17.4°, and 22.3° 2θ ± 0.2° 2θ.

In certain embodiments, the BIC drug substance is the crystalline form of bictegravir free acid, wherein the crystalline form of bictegravir free acid is Form II. In some embodiments, Form II of bictegravir free acid has an XRPD pattern comprising peaks at about 6.6°, 10.6°, and 12.5° 2θ ± 0.2° 2θ. In some embodiments, Form II of bictegravir free acid has an XRPD pattern further comprising peaks at about 16.2° and 21.4° 2θ ± 0.2° 2θ. In some embodiments, Form II of bictegravir free acid has an XRPD pattern further comprising peaks at about 14.3°, 23.5°, and 25.6° 2θ ± 0.2° 2θ.

In certain embodiments, the BIC drug substance is the crystalline form of bictegravir free acid, wherein the crystalline form of bictegravir free acid is Form III. In some embodiments, Form III of bictegravir free acid has an XRPD pattern comprising peaks at about 9.6°, 14.0°, and 18.5° 2θ ± 0.2° 2θ. In some embodiments, Form III of bictegravir free acid has an XRPD pattern further comprising peaks at about 20.0° and 22.5° 2θ ± 0.2° 2θ. In some embodiments, Form III of bictegravir free acid has an XRPD pattern further comprising peaks at about 12.1° and 16.2° 2θ ± 0.2° 2θ. In some embodiments, Form III of bictegravir free acid has an XRPD pattern further comprising peaks at about 25.0°, 27.0°, and 29.0° 2θ ± 0.2° 2θ.

In certain embodiments, the BIC drug substance is the crystalline form of bictegravir free acid, wherein the crystalline form of bictegravir free acid is Form IV. In some embodiments, Form IV of bictegravir free acid has an XRPD pattern comprising peaks at about 6.2°, 16.3°, and 22.3° 2θ ± 0.2° 2θ. In some embodiments, Form IV of bictegravir free acid has an XRPD pattern further comprising peaks at about 22.7° and 25.8° 2θ ± 0.2° 2θ. In some embodiments, Form IV of bictegravir free acid has an XRPD pattern further comprising peaks at about 8.6° and 13.2° 2θ ± 0.2° 2θ. In some embodiments, Form IV of bictegravir free acid has an XRPD pattern further comprising peaks at about 18.7°, 20.0°, and 27.7° 2θ ± 0.2° 2θ.

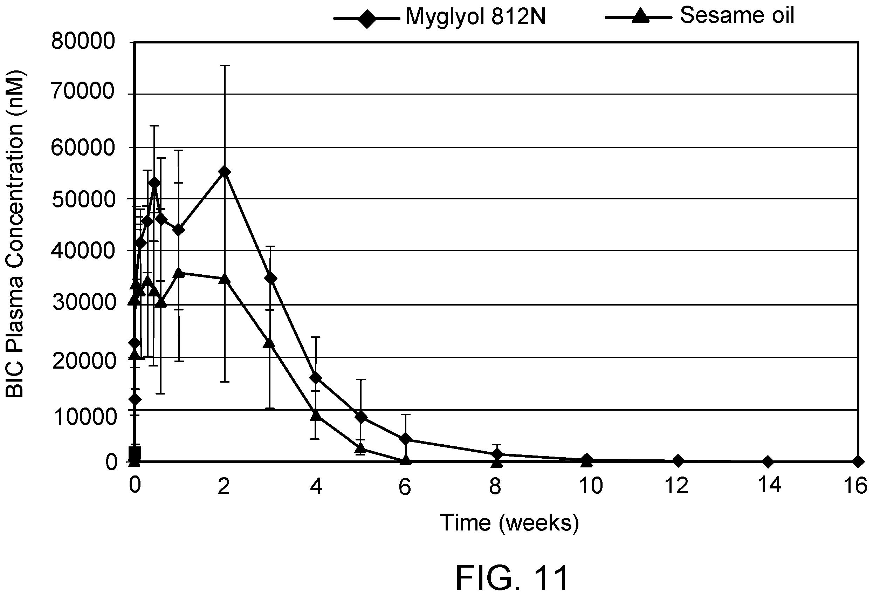

In certain embodiments, the BIC drug substance is the crystalline form of bictegravir free acid, wherein the crystalline form of bictegravir free acid is Form IX. In some embodiments, as shown in FIG. 11, Form IX of bictegravir free acid has an XRPD pattern comprising at least one peak selected from about 6.5°, about 6.7°, about 14.3°, about 14.4°, about 16.7°, or about 28.3° 2θ ± 0.2° 2θ. In some embodiments, Form IX of bictegravir free acid has an XRPD pattern comprising at least two peaks selected from about 6.5°, about 6.7°, about 14.3°, about 14.4°, about 16.7°, or about 28.3° 2θ ± 0.2° 2θ. In some embodiments, Form IX of bictegravir free acid has an XRPD pattern comprising at least three peaks selected from about 6.5°, about 6.7°, about 14.3°, about 14.4°, about 16.7°, or about 28.3° 2θ ± 0.2° 2θ. In some embodiments, Form IX of bictegravir free acid has an XRPD pattern comprising at least one peak selected from about 6.5°, about 6.7°, about 10.6°, about 10.9°, about 12.5°, about 14.3°, about 14.4°, about 16.7°, about 28.3°, or about 29.7° 2θ ± 0.2° 2θ. In some embodiments, Form IX of bictegravir free acid has an XRPD pattern comprising at least two peaks selected from about 6.5°, about 6.7°, about 10.6°, about 10.9°, about 12.5°, about 14.3°, about 14.4°, about 16.7°, about 28.3°, or about 29.7° 2θ ± 0.2° 2θ. In some embodiments, Form IX of bictegravir free acid has an XRPD pattern comprising at least three peaks selected from about 6.5°, about 6.7°, about 10.6°, about 10.9°, about 12.5°, about 14.3°, about 14.4°, about 16.7°, about 28.3°, or about 29.7° 2θ ± 0.2° 2θ. In some embodiments, Form IX of bictegravir free acid has an XRPD pattern comprising at least four peaks selected from about 6.5°, about 6.7°, about 10.6°, about 10.9°, about 12.5°, about 14.3°, about 14.4°, about 16.7°, about 28.3°, or about 29.7° 2θ ± 0.2° 2θ. In some embodiments, Form IX of bictegravir free acid has an XRPD pattern substantially as shown in FIG. 11.

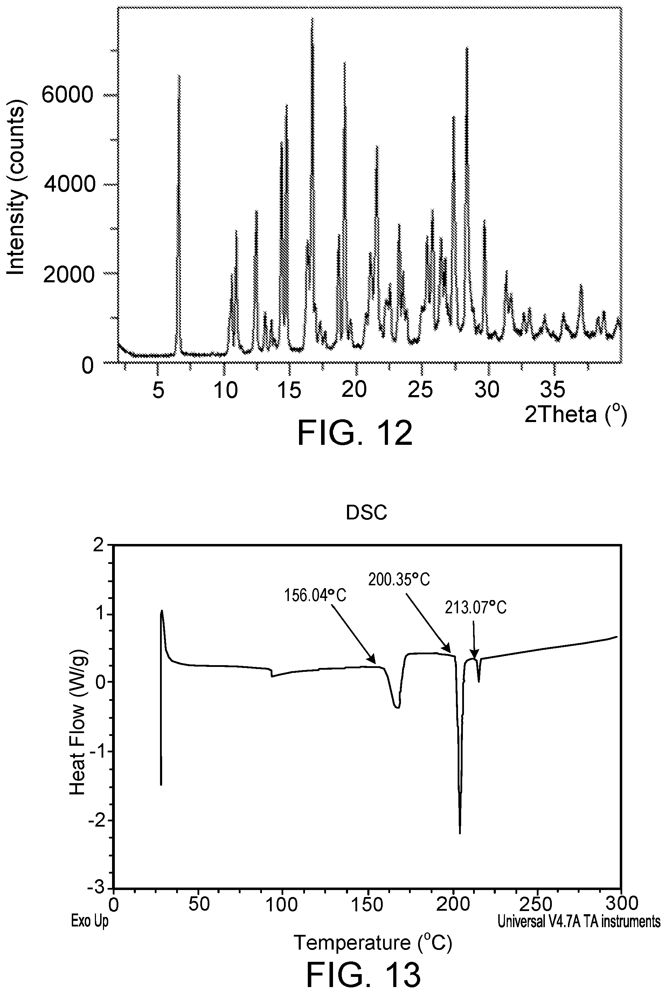

In certain embodiments, Form IX of bictegravir free acid has a DSC thermogram comprising an endothermic event with onset at about 156 °C, an endothermic event at about 200 °C, and an endothermic event at about 213 °C. In certain embodiments, Form IX of bictegravir free acid has a DSC thermogram substantially as shown in FIG. 12. In certain embodiments, Form IX of bictegravir free acid has a TGA graph substantially as shown in FIG. 13.

In certain embodiments, the BIC drug substance is the crystalline form of bictegravir free acid, wherein the crystalline form of bictegravir free acid is Form X. In some embodiments, Form X of bictegravir free acid has an XRPD pattern comprising at least one peak selected from about 5.3°, about 10.6°, about 14.3°, about 15.9° or about 22.1° 2θ ± 0.2° 2θ. In some embodiments, Form X of bictegravir free acid has an XRPD pattern comprising at least two peaks selected from about 5.3°, about 10.6°, about 14.3°, about 15.9° and about 22.1° 20 ± 0.2° 2θ. In some embodiments, Form X of bictegravir free acid has an XRPD pattern comprising at least three peaks selected from about 5.3°, about 10.6°, about 14.3°, about 15.9° and about 22.1° 2θ ± 0.2° 2θ. In some embodiments, Form X of bictegravir free acid has an XRPD pattern comprising at least one peak selected from about 5.3°, about 10.6°, about 14.3°, about 15.9°, about 22.1°, about 17.9°, about 18.6°, about 18.9°, about 19.5°, about 23.5°, about 24.1°, or about 24.6° 2θ ± 0.2° 2θ. In some embodiments, Form X of bictegravir free acid has an XRPD pattern comprising at least two peaks selected from about 5.3°, about 10.6°, about 14.3°, about 15.9°, about 22.1°, about 17.9°, about 18.6°, about 18.9°, about 19.5°, about 23.5°, about 24.1°, and about 24.6° 2θ ± 0.2° 2θ. In some embodiments, Form X of bictegravir free acid has an XRPD pattern comprising at least three peaks selected from about 5.3°, about 10.6°, about 14.3°, about 15.9°, about 22.1°, about 17.9°, about 18.6°, about 18.9°, about 19.5°, about 23.5°, about 24.1°, and about 24.6° 2θ ± 0.2° 2θ. In some embodiments, Form X of bictegravir free acid has an XRPD pattern comprising at least four peaks selected from about 5.3°, about 10.6°, about 14.3°, about 15.9°, about 22.1°, about 17.9°, about 18.6°, about 18.9°, about 19.5°, about 23.5°, about 24.1°, and about 24.6° 2θ ± 0.2° 2θ.

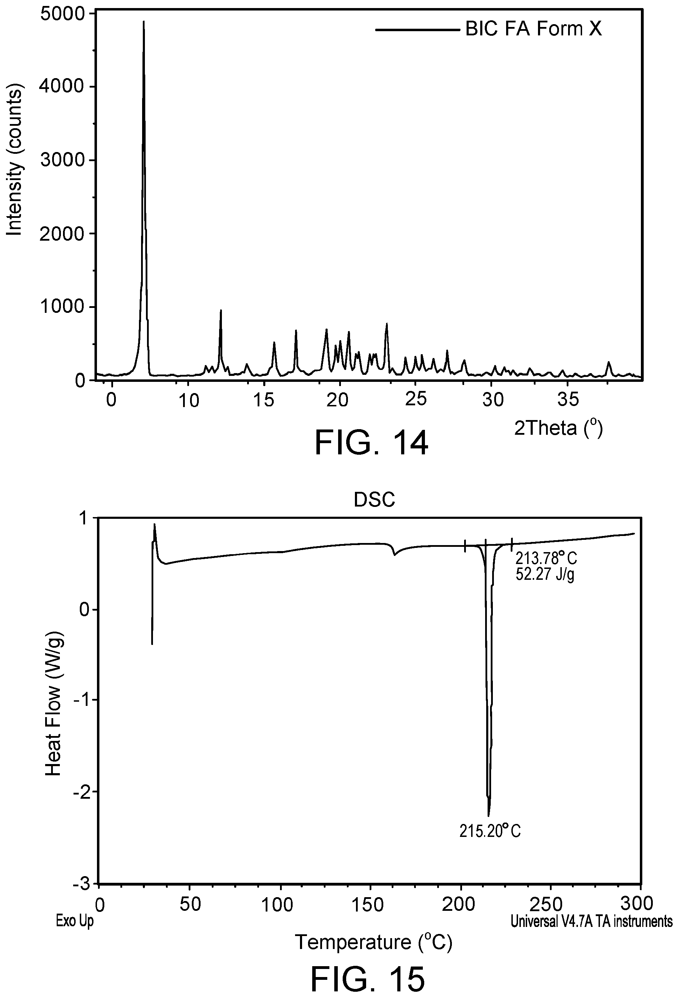

In certain embodiments, Form X of bictegravir free acid has a DSC thermogram having an endotherm at about 213 °C. In certain embodiments, Form X of bictegravir free acid has a DSC thermogram substantially as shown in FIG. 15.

Form X of bictegravir free acid can be prepared by heating bictegravir free acid Form IX (e.g., to over 200 C, for example, to about 210 °C).

The term "substantially as shown in" when referring, for example, to an XRPD pattern, a DSC thermogram, or a TGA graph includes a pattern, thermogram or graph that is not necessarily identical to those depicted herein, but that falls within the limits of experimental error or deviations when considered by one of ordinary skill in the art.

Bictegravir can be present in the pharmaceutical compositions described herein in solvated and/or unsolvated form, and references to "bictegravir", or "bictegravir drug substance" comprise the solvated and unsolvated forms and mixtures thereof. As used herein, and in absence of a specific reference to a solvate of bictegravir, any dosages, whether expressed in, e.g., milligrams or as % by weight, should be taken as referring to the amount of bictegravir, i.e., the amount of:

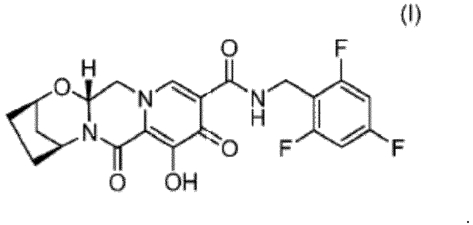

Therefore, for example, a reference to "500 mg bictegravir, or a pharmaceutically acceptable salt thereof" means an amount of bictegravir, or a pharmaceutically acceptable salt thereof which provides the same amount of bictegravir as 500 mg of bictegravir free acid.

Bictegravir can be prepared using a crystallization process in one or more suitable solvents. In some embodiments, bictegravir is crystallized from isopropyl alcohol (IPA). In some embodiments, bictegravir is crystallized from a mixture of water and N-methyl-2-pyrrolidone (NMP). In some embodiments, the BIC drug substance is micronized (i.e., a micronized drug substance). Micronization is the process of reducing the particle size of a bulk solid material (e.g., a pharmaceutical drug substance) to micron or submicron level. Micronization of pharmaceutical bulk solids can be achieved via methods including, for example, milling or grinding.

In some embodiments, the particle size of the BIC drug substance is controlled by milling, e.g., jet milling (fluid energy milling), bead milling, dry milling spiral milling, or high shear wet milling (HSWM). In an embodiment, the particle size of the BIC drug substance is controlled by high shear wet milling. High shear wet milling can be either integrated into the crystallization process or performed post crystallization. In some embodiments, the HSWM is performed at a single speed of about 3,000 rpm to about 20,000 rpm; at about 3,000 rpm, about 4,000 rpm, about 5,000 rpm, about 6,000 rpm, about 7,000 rpm, about 8,000 rpm, about 9,000 rpm, about 10,000 rpm, about 11,000 rpm, about 12,000 rpm, about 13,000 rpm, about 14,000 rpm, about 15,000 rpm, or about 16,000 rpm. In an embodiment, the HSWM is performed at a single speed of 4,200 rpm. In an embodiment, the HSWM is performed at a single speed of 5,500 rpm. In an embodiment, the HSWM is performed at a single speed of 8,000 rpm. In an embodiment, the HSWM is performed at a single speed of 12,000 rpm. In an embodiment, the HSWM is performed at a single speed of 16,000 rpm. In some embodiments, the wet-milled granulated particles are passed through a comil upon drying. In some embodiments, the size of the comil screen is about 0.032 inches to about 0.250 inches. In an embodiment, the size of the comil screen is about 0.032 inches. In an embodiment, the size of the comil screen is about 0.250 inches. In some embodiments, the wet-milled granulated particles are dried in a tumble dryer. In some embodiments, the wet-milled granulated particles are dried in an agitated filter/dryer. In some embodiments, the wet-milled granulated particles are dried in a vacuum oven dryer.

In an embodiment, the particle size of the BIC drug substance is controlled by jet milling. In an embodiment, the particle size of the BIC drug substance is controlled by loop style jet milling. In an embodiment, the particle size of the BIC drug substance is controlled by a Model 0202 Jet-O-Mizer (JOM) loop style jet mill system. In an embodiment, the particle size of the BIC drug substance is controlled by spiral style jet milling.

In an embodiment, the particle size of the BIC drug substance is controlled by bead milling. In some embodiments, the BIC drug substance is sieved after milling to reduce agglomerates which may help reduce the risk of clogging and improve flowability of the milled BIC powder. In some embodiments, the milled BIC powder is not sieved prior to vial filling.

The particle size distribution of a solid material can be measured using a variety of analytical characterization methods known to those skilled in the art, including, for example, sieving, laser light diffraction, quasi-elastic light scattering, centrifugal sedimentation-optical, electrical resistance zone sensing, microelectrophoresis, light microscopy, and scanning electron microscopy, etc. In some embodiments, the particle size distribution of the BIC drug substance is measured using light microscopy. In some embodiments, the particle size distribution of the BIC drug substance is measured using laser light diffraction. The particle size distribution of a solid material can be represented by the d₉₀, d₅₀ and d₁₀ values as defined herein. In some embodiments, the BIC drug substance has a particle size distribution with a d₉₀ value less than about 120 µm. For example, the BIC drug substance has a particle size distribution with a d₉₀ value less than about 115 µm, about 110 µm, about 105 µm, about 100 µm, about 95 µm, about 90 µm, about 85 µm, about 80 µm, about 70 µm, about 65 µm, about 60 µm, about 55 µm, about 50 µm, about 45 µm, about 40 µm, about 35 µm, about 30 µm, about 25 µm, about 20 µm, about 15 µm, about 10 µm, about 9 µm, about 8 µm, about 7 µm, about 6 µm, about 5 µm, about 4 µm, about 3 µm, about 2 µm, or about 1 µm. In some embodiments, the has a particle size distribution with a d₉₀ value from about 1 µm to about 120 µm, for example, about 1 µm to about 115 µm, about 1 µm to about 110 µm, about 1 µm to about 105 µm, about 1 µm to about 100 µm, about 1 µm to about 95 µm, about 1 µm to about 90 µm, about 1 µm to about 85 µm, about 1 µm to about 80 µm, about 1 µm to about 75 µm, about 1 µm to about 70 µm, about 1 µm to about 65 µm, about 1 µm to about 60 µm, about 1 µm to about 55 µm, about 1 µm to about 50 µm, about 1 µm to about 45 µm, about 1 µm to about 40 µm, about 1 µm to about 35 µm, about 1 µm to about 30 µm, about 1 µm to about 25 µm, about 1 µm to about 20 µm, about 1 µm to about 15 µm, about 1 µm to about 10 µm, 1 µm to about 9 µm, 1 µm to about 8 µm, 1 µm to about 7 µm, 1 µm to about 6 µm, 1 µm to about 5 µm, 1 µm to about 4 µm, 1 µm to about 3 µm, or 1 µm to 2 µm. In some embodiments, the BIC drug substance has a particle size distribution with a d₉₀ value of ≤ about 30 µm. In some embodiments, the BIC drug substance has a particle size distribution with a d₉₀ value of ≤ about 20 µm. In some embodiments, the BIC drug substance has a particle size distribution with a d₉₀ value of ≤ about 15 µm. In some embodiments, the BIC drug substance has a particle size distribution with a d₉₀ value of ≤ about 10 µm. In some embodiments, the BIC drug substance has a particle size distribution with a d₉₀ value from about 25 µm to about 30 µm. In some embodiments, the BIC drug substance has a particle size distribution with a d₉₀ value from about 20 µm to about 25 µm. In some embodiments, the BIC drug substance has a particle size distribution with a d₉₀ value from about 15 µm to about 20 µm. In some embodiments, the BIC drug substance has a particle size distribution with a d₉₀ value from about 10 µm to about 15 µm. In some embodiments, the BIC drug substance has a particle size distribution with a d₉₀ value from about 1 µm to about 10 µm. In some embodiments, the BIC drug substance has a particle size distribution with a d₉₀ value from about 1 µm to about 5 µm. In some embodiments, the BIC drug substance has a particle size distribution with a d₉₀ value of about 20 µm. In some embodiments, the BIC drug substance has a particle size distribution with a d₉₀ value of about 19 µm. In some embodiments, the BIC drug substance has a particle size distribution with a d₉₀ value of about 18 µm. In some embodiments, the BIC drug substance has a particle size distribution with a d₉₀ value of about 17 µm. In some embodiments, the BIC drug substance has a particle size distribution with a d₉₀ value of about 16 µm. In some embodiments, the BIC drug substance has a particle size distribution with a d₉₀ value of about 15 µm. In some embodiments, the BIC drug substance has a particle size distribution with a d₉₀ value of about 14 µm. In some embodiments, the BIC drug substance has a particle size distribution with a d₉₀ value of about 13 µm. In some embodiments, the BIC drug substance has a particle size distribution with a d₉₀ value of about 12 µm. In some embodiments, the BIC drug substance has a particle size distribution with a d₉₀ value of about 11 µm. In some embodiments, the BIC drug substance has a particle size distribution with a d₉₀ value of about 10 µm. In some embodiments, the BIC drug substance has a particle size distribution with a d₉₀ value of about 4 µm.

In some embodiments, the BIC drug substance has a particle size distribution with a d₉₀ value from about 1 µm to about 90 µm.

In some embodiments, the BIC drug substance has a particle size distribution with a d₉₀ value from about 15 µm to about 70 µm.

In some embodiments, the BIC drug substance has a particle size distribution with a d₉₀ value from about 5 µm to about 30 µm.

In some embodiments, the BIC drug substance has a particle size distribution with a d₉₀ value less than about 20 µm.

In some embodiments, the amount of the BIC drug substance present is about 23% of the total pharmaceutical composition weight.

In some embodiments, the BIC drug substance is a crystalline form of bictegravir free acid and the amount of the BIC drug substance present is about 18% to about 28% of the total pharmaceutical composition weight. In some embodiments, the BIC drug substance is a crystalline form of bictegravir free acid and the amount of the BIC drug substance present is about 23% of the total pharmaceutical composition weight. In some embodiments, the BIC drug substance is crystalline form III of bictegravir free acid and the amount of the BIC drug substance present is about 18% to about 28% of the total pharmaceutical composition weight In some embodiments, the BIC drug substance is crystalline form III of bictegravir free acid and the amount of the BIC drug substance present is about 23% of the total pharmaceutical composition weight.

### III. Pharmaceutical Compositions Comprising Bictegravir (BIC) Drug Substance

The pharmaceutical compositions described herein are generally physically and/or chemically stable. As such the pharmaceutical compositions described herein can be stored at room temperature or refrigerated conditions for an extended period of time without significant degradation and/or change in physical form. In some embodiments, the pharmaceutical compositions can be stored at room temperature for at least one month, for example at least two months, at least three months, at least four months, at least five months, at least six months, at least one year, or at least two years.

The pharmaceutical compositions described herein are suitable for injection. As such, the pharmaceutical compositions provided herein can be formulated as a suspension or solution. In some embodiments, the pharmaceutical composition is formulated as a nanosuspension. In some embodiments, the pharmaceutical composition is formulated as a microsuspension. As defined herein, a nanosuspension is a biphasic, colloidal dispersion of submicron drug particles typically of less than 1 µm in diameter. As defined herein, a microsuspension is a dispersion of drug particles typically of between about 1 µm to about 200 µm in diameter.

In some embodiments, the pharmaceutical composition provided herein is formulated as a solid dispersion formulation. Pharmaceutical solid dispersion formulations consist of a drug dispersed in one or more hydrophilic carriers, which can increase the surface area of the drug-carrier particles and lead to enhanced drug solubility and dissolution rate. Examples of hydrophilic carriers used in the preparation of pharmaceutical solid dispersion formulations include crystalline carriers (e.g., urea and sugars such as D-mannitol, D-fructose, D-maltose), polymeric carriers (e.g., poly(lactic-co-glycolic acid) (PLGA), Povidone (PVP), polyethylene glycol (PEG), polymethacrylates, hydroxypropylmethylcellulose (HPMC), and hydroxypropylcellulose (HPC)), surfactants (e.g., poloxamers, sodium lauryl sulfate (SLS), Gelucire^{®}, and polysorbates (Tween^{®})), and mixtures thereof. Pharmaceutical solid dispersion formulations can be prepared using methods known to those skilled in the art, for example, using the kneading method, a melting method (hot-melt extrusion, melt agglomeration), a solvent evaporation method (spray drying, lyophilization, super-critical fluid, co-precipitation, electrospinning), or a melt evaporation method. Depending upon the hydrophilic carrier and the method used in the preparation of the solid dispersion formulation, the drug can exist as amorphous or crystalline solids dispersed within the formulation. Examples of analytical characterization of solid dispersion formulations include X-ray powder diffraction analysis (XRPD), differential-scanning calorimetry (DSC), thermogravimetric analysis (TGA), particle size analysis (PSD), scanning electron microscopy (SEM), and dissolution testing.

In some embodiments, the solid dispersion formulation includes amorphous bictegravir free acid. In some embodiments, the solid dispersion formulation includes crystalline bictegravir free acid. In some embodiments, the solid dispersion formulation includes bictegravir free acid crystalline Form III.

In some embodiments, the pharmaceutical compositions described herein are administered parenterally. In some embodiments, the pharmaceutical compositions described herein are administered parenterally via injection. In some embodiments, the pharmaceutical compositions for injection described herein are administered subcutaneously or intramuscularly. In some embodiments, the pharmaceutical compositions for injection described herein are administered subcutaneously. In some embodiments, the pharmaceutical compositions for injection described herein are administered intramuscularly.

In some embodiments, the pharmaceutical compositions described herein are administered via injection using an injection device. In some embodiments, the injection device is or includes a syringe, which can be employed manually, or as part of a syringe-containing injection device. A wide variety of injection devices can be used, including, but not limited to, a handheld or wearable autoinjector, a handheld or wearable manual injector, an on-body injector, a syrette, a jet injector, or a pen injector, each of which can be reusable or disposable.

In some embodiments, the pharmaceutical compositions provided herein can be administered with a syringe suitable for administration of the compound. In some embodiments, the syringe is disposable. In some embodiments, the syringe is reusable. In some embodiments, the syringe is pre-filled with the pharmaceutical composition.

In some embodiments, the pharmaceutical compositions provided herein can be administered with an auto-injector comprising a syringe. In some embodiments, the syringe is disposable. In some embodiments, the syringe is reusable. In some embodiments, the syringe is pre-filled with any of the pharmaceutical compositions provided herein.

In some embodiments, the concentration of bictegravir in the pharmaceutical composition for injection is from about 100 mg/mL to about 1000 mg/mL, including, e.g., about 200 mg/mL to about 1000 mg/mL, about 300 mg/mL to about 1000 mg/mL, about 200 mg/mL to about 500 mg/mL, about 300 mg/mL to about 500 mg/mL, and about 400 mg/mL to about 500 mg/mL. In some embodiments, the concentration of bictegravir in the pharmaceutical composition for injection is about 100 mg/mL, about 125 mg/mL, about 150 mg/mL, about 175 mg/mL, about 200 mg/mL, about 225 mg/mL, about 250 mg/mL, about 275 mg/mL, about 300 mg/mL, about 325 mg/mL, about 350 mg/mL, about 375 mg/mL, about 400 mg/mL, about 425 mg/mL, about 450 mg/mL, about 475 mg/mL, about 500 mg/mL, about 525 mg/mL, about 550 mg/mL, about 575 mg/mL, about 600 mg/mL, about 625 mg/mL, about 650 mg/mL, about 675 mg/mL, about 700 mg/mL, about 725 mg/mL, about 750 mg/mL, about 775 mg/mL, about 800 mg/mL, about 825 mg/mL, about 850 mg/mL, about 875 mg/mL, about 900 mg/mL, about 925 mg/mL, about 950 mg/mL, about 975 mg/mL, or about 1000 mg/mL.

In some embodiments, the concentration of bictegravir in the pharmaceutical composition for injection is from about 225 mg/mL to about 275 mg/mL. In some embodiments, the concentration of bictegravir in the pharmaceutical composition for injection is about 250 mg/mL.

In some embodiments, the concentration of bictegravir in the pharmaceutical composition for injection is from about 300 mg/mL to about 500 mg/mL. In some embodiments, the concentration of bictegravir in the pharmaceutical composition for injection is about 400 mg/mL. In some embodiments, the concentration of bictegravir in the pharmaceutical composition for injection is about 450 mg/mL.

In some embodiments, the concentration of bictegravir in the pharmaceutical composition for injection is from about 200 mg/mL to about 400 mg/mL. In some embodiments, the concentration of bictegravir in the pharmaceutical composition for injection is about 300 mg/mL.

The volume of the pharmaceutical composition for injection administered per administration (i.e., one dose of the pharmaceutical composition for injection) is about 0.1 to about 5 mL. In some embodiments, the volume of the suspension for injection administered per administration is about 0.1 mL to about 4.5 mL, about 0.1 mL to about 4.0 mL, about 0.1 mL to about 3.5 mL, about 0.1 mL to about 3.0 mL, about 0.1 mL to about 2.5 mL, about 0.1 mL to about 2.0 mL, about 0.1 mL to about 1.5 mL, about 0.1 mL to about 1.0 mL, about 0.1 mL to about 0.5 mL, about 0.5 mL to about 5.0 mL, about 0.5 mL to about 4.5 mL, about 0.5 mL to about 4.0 mL, about 0.5 mL to about 3.5 mL, about 0.5 mL to about 3.0 mL, about 0.5 mL to about 2.5 mL, about 0.5 mL to about 2.0 mL, about 0.5 mL to about 1.5 mL, about 0.5 mL to about 1.0 mL, about 1.0 mL to about 5.0 mL, about 1.0 mL to about 4.5 mL, about 1.0 mL to about 4.0 mL, about 1.0 mL to about 3.5 mL, about 1.0 mL to about 3.0 mL, about 1.0 mL to about 2.5 mL, about 1.0 mL to about 2.0 mL, about 1.0 mL to about 1.5 mL, about 1.5 mL to about 5.0 mL, about 1.5 mL to about 4.5 mL, about 1.5 mL to about 4.0 mL, about 1.5 mL to about 3.5 mL, about 1.5 mL to about 3.0 mL, about 1.5 mL to about 2.5 mL, about 1.5 mL to about 2.0 mL, about 2.0 mL to about 5.0 mL, about 2.0 mL to about 4.5 mL, about 2.0 mL to about 4.0 mL, about 2.0 mL to about 3.5 mL, about 2.0 mL to about 3.0 mL, about 2.0 mL to about 2.5 mL, about 2.5 mL to about 5.0 mL, about 2.5 mL to about 4.5 mL, about 2.5 mL to about 4.0 mL, about 2.5 mL to about 3.5 mL, about 2.5 mL to about 3.0 mL, about 3.0 mL to about 5.0 mL, about 3.0 mL to about 4.5 mL, about 3.0 mL to about 4.0 mL, about 3.0 mL to about 3.5 mL, about 3.5 mL to about 5.0 mL, about 3.5 mL to about 4.5 mL, about 3.5 mL to about 4.0 mL, about 4.0 mL to about 5.0 mL, about 4.0 mL to about 4.5 mL, or about 4.5 mL to about 5.0 mL.

In some embodiments, the volume of the pharmaceutical composition for injection administered is about 1.5 mL to about 3.5 mL, for example about 1.5 mL, about 1.6 mL, about 1.7 mL, about 1.8 mL, about 1.9 mL, about 2.0 mL, about 2.1 mL, about 2.2 mL, about 2.3 mL, about 2.4 mL, about 2.5 mL, about 2.6 mL, about 2.7 mL, about 2.8 mL, about 2.9 mL, about 3.0 mL, about 3.1 mL, about 3.2 mL, about 3.3 mL, about 3.4 mL, or about 3.5 mL.

In some embodiments, the volume of the pharmaceutical composition for injection administered is about 0.5 mL to about 2.5 mL. In some embodiments, the volume of the pharmaceutical composition for injection administered is about 0.8 mL to about 1.2 mL. In some embodiments, the volume of the pharmaceutical composition administered by injection is about 1.0 mL.

In some embodiments, the volume of the pharmaceutical composition for injection administered is about 1.0 mL to about 3.0 mL. In some embodiments, the volume of the pharmaceutical composition for injection administered is about 1.8 mL to about 2.2 mL. In some embodiments, the volume of the pharmaceutical composition administered by injection is about 2.0 mL.

In some embodiments, the volume of the pharmaceutical composition for injection administered is about 1.5 mL to about 3.5 mL. **In** some embodiments, the volume of the pharmaceutical composition for injection administered is about 2.0 mL to about 3.0 mL. In some embodiments, the volume of the pharmaceutical composition administered by injection is about 2.5 mL.

In some embodiments, the pharmaceutical compositions described herein are evaluated for syringeability and injectability. As defined herein, syringeability refers to the ability of the pharmaceutical composition to pass easily through a hypodermic needle on injection, whereas injectability refers to the performance of the pharmaceutical composition during injection, as measured by parameters such as glide force (measured in Newton, or N) and viscosity (measured in centipoise, or cP), using suitable methods known in the art. In some embodiments, the pharmaceutical composition exhibits a maximum glide force of about 20 N to about 40 N when measured using a 22G ETW (extra thin wall) hypodermic needle. In some embodiments, the pharmaceutical composition exhibits a maximum glide force of about 20 N to about 35 N when measured using a 22G ETW hypodermic needle. In some embodiments, the pharmaceutical composition exhibits a maximum glide force of about 24 N to about 30 N when measured using a 22G ETW hypodermic needle. In some embodiments, the estimated viscosity of the pharmaceutical composition is about 300 cP to about 600 cP. In some embodiments, the estimated viscosity of the pharmaceutical composition is about 300 cP to about 400 cP. In some embodiments, the estimated viscosity of the pharmaceutical composition is about 400 cP to about 500 cP. In some embodiments, the estimated viscosity of the pharmaceutical composition is about 500 cP to about 600 cP.

The amount of povidone K12 in the pharmaceutical composition may vary. In some embodiments, the amount of povidone K12 in the pharmaceutical composition is about 1.0% w/w.

In some embodiments, the amount of povidone K12 in the pharmaceutical composition is about 1.5% w/w.

The amount of Tween 20 in the pharmaceutical composition may vary. In some embodiments, the amount of Tween 20 in the pharmaceutical composition is about 0.4 % w/w.

In some embodiments, the sodium phosphate buffer comprises sodium phosphate monobasic, sodium phosphate dibasic, and/or sodium chloride. In some embodiments, the sodium phosphate buffer comprises sodium phosphate monobasic and sodium phosphate dibasic. In some embodiments, the amount of the sodium phosphate buffer in the pharmaceutical composition is about 0.1%, about 0.2%, and about 0.3%. In some embodiments, the amount of the sodium phosphate buffer in the pharmaceutical composition is the amount of buffer required to maintain a pH of between about 7 and 7.8 (e.g., 7.4).

In some embodiments, the pharmaceutical composition has a pH of from about 7.0 to about 7.8. In some embodiments, the pharmaceutical composition has a pH of about 7.4.

In some embodiments, the pharmaceutical composition consists of:

| **Component** | **% w/w** |
|---|---|
| bictegravir, free acid | 23.0 |
| povidone K-12 | 1.5 |
| polysorbate 20 | 0.4 |
| sodium chloride | 0.6 |
| sodium phosphate buffer | 0.2 |
| water | 74.3 |

In some embodiments, the pharmaceutical composition consists of:

| **Component** | **% w/w** |
|---|---|
| bictegravir, free acid | 23.6 |
| povidone K-12 | 1.5 |
| polysorbate 20 | 0.4 |
| sodium chloride | 0.6 |
| sodium phosphate buffer | 0.2 |
| water | 73.7 |

In some embodiments, the pharmaceutical compositions and their individual components are sterilized to remove endotoxin and/or bioburden using one or more suitable methods known in the art, for example, by autoclaving, dry heating, Gamma radiation, infrared radiation, ultraviolet (UV) radiation, processing with high-velocity electrons (E-beam), or sterile filtration. In some embodiments, the pharmaceutical compositions are sterilized using gamma radiation. In some embodiments, the pharmaceutical compositions are sterilized using sterile filtration. In some embodiments, the one or more pharmaceutically acceptable excipients are sterilized using gamma radiation. In some embodiments, the pharmaceutically acceptable excipients are sterilized using sterile filtration. In some embodiments, the Miglyol 812N vehicle is sterilized using gamma radiation. In some embodiments, the Miglyol 812N vehicle is sterilized using sterile filtration.

Any of the pharmaceutical compositions disclosed herein can be administered by injection. In some embodiments, the pharmaceutical composition is for subcutaneous (SC) or intramuscular (IM) administration. Site reactions to injections and infusions are common adverse events (AE), which are any unfavorable and unintended sign, symptom, or disease temporally associated with the use of a medical treatment or procedure regardless of whether it is considered related to the medical treatment or procedure. Injection site reactions (ISR) associated with SC or IM administration of a pharmaceutical composition can be measured by monitoring the following parameters: injection site pain or tenderness, injection site erythema or redness, injection site induration or swelling, and injection site pruritus, on a scale of Grade 1 (mild) to Grade 4 (potentially life-threatening) based on the severity of the symptoms (Division of AIDS (DAIDS) Table for Grading the Severity of Adult and Pediatric Adverse Events, Corrected Version 2.1, July 2017, page 24).

Any of the pharmaceutical compositions provided herein can be for use in a method for treating or preventing an HIV infection, comprising administering to the human a therapeutically effective amount of any one of the disclosed pharmaceutical compositions. In some embodiments, the pharmaceutical composition is administered not more frequently than once every four weeks (Q4W). In some embodiments, the pharmaceutical composition is administered once every four weeks (Q4W). In some embodiments, the pharmaceutical composition is administered once every eight weeks (Q8W). In some embodiments, the pharmaceutical composition is administered once every twelve weeks (Q12W).

In some embodiments, the therapeutically effective amount of the pharmaceutical composition, is the amount of the pharmaceutical composition comprising from about 100 mg to about 2500 mg of bictegravir. In some embodiments, the pharmaceutical composition comprises of from about 200 mg to about 1000 mg of bictegravir. In some embodiments, the pharmaceutical composition comprises of from about 400 mg to about 600 mg of bictegravir. In some embodiments, the pharmaceutical composition comprises about 500 mg of bictegravir.

### IV. Pharmaceutical compositions for use in methods of treatment

Provided herein are pharmaceutical compositions for use in methods for treating or preventing an HIV infection in a human, comprising administering to the human a therapeutically effective amount of the pharmaceutical composition, wherein the pharmaceutical composition is administered by injection. Such pharmaceutical compositions (i.e., pharmaceutical formulations) can be long-acting meaning that the pharmaceutical formulations provided herein can maintain a therapeutically effective amount of bictegravir, or a pharmaceutically acceptable salt thereof, in the plasma and/or the PBMCs of the human. The bictegravir long-acting formulations can be administered to the human less frequently than, e.g., immediate release or short-acting forms.

Accordingly, provided herein are pharmaceutical compositions for use in a method for treating or preventing an HIV infection in a human, comprising administering to the human a therapeutically effective amount of any of the pharmaceutical compositions.

In some embodiments of the methods provided herein, the bictegravir is administered once every 1 week to about 6 months. In some embodiments, the bictegravir is administered at a frequency of about 1 week to about 5 months, about 1 week to about 4 months, about 1 week to about 3 months, about 1 week to about 2 months, about 1 week to about 1 month, about 1 week to about 4 weeks, about 1 week to about 3 weeks, about 1 week to about 2 weeks, about 2 weeks to 6 months, about 2 weeks to about 5 months, about 2 weeks to about 4 months, about 2 weeks to about 3 months, about 2 weeks to about 2 months, about 2 weeks to about 1 month, about 2 weeks to about 4 weeks, about 2 weeks to about 3 weeks, about 3 weeks to about 6 months, about 3 weeks to about 5 months, about 3 weeks to about 4 months, about 3 weeks to about 3 months, about 3 weeks to about 2 months, about 3 weeks to about 1 month, about 3 weeks to about 4 weeks, about 4 weeks to about 6 months, about 4 weeks to about 5 months, about 4 weeks to about 4 months, about 4 weeks to about 3 months, about 4 weeks to about 2 months, about 4 weeks to about 1 month, about 1 month to about 6 months, about 1 month to about 5 months, about 1 month to about 4 months, about 1 month to about 3 months, about 1 month to about 3 months, about 2 months to about 6 months, about 2 months to about 5 months, about 2 months to about 4 months, about 2 months to about 3 months, about 2 months to about 3 months, about 3 months to about 6 months, about 3 months to about 5 months, about 3 months to about 4 months, about 4 months to about 6 months, about 3 months to about 5 months, or about 5 months to about 6 months.

In some embodiments, the bictegravir is administered once every 28 days. In some embodiments, the bictegravir is administered once every 1 month. In some embodiments the bictegravir is administered at a frequency of once every month or less. In some embodiments, the bictegravir is administered at a frequency of once every two months or less. In some embodiments, the bictegravir is administered at a frequency of once every three months or less.

In some embodiments of the methods provided herein, the bictegravir is administered not more frequently than once every four weeks (Q4W).

In some embodiments, the bictegravir is administered once every four weeks (Q4W). In some embodiments, the bictegravir is administered once every five weeks (Q5W). In some embodiments, the bictegravir is administered once every six weeks (Q6W). In some embodiments, the bictegravir is administered once every seven weeks (Q7W). In some embodiments, the bictegravir is administered once every eight weeks (Q8W). In some embodiments, the bictegravir is administered once every nine weeks (Q9W). In some embodiments, the bictegravir is administered once every ten weeks (Q10W). In some embodiments, the bictegravir is administered once every eleven weeks (Q11W). In some embodiments, the bictegravir is administered once every twelve weeks (Q12W). In some embodiments, the bictegravir is administered once every fourteen weeks (Q14W). In some embodiments, the bictegravir is administered once every sixteen weeks (Q16W).

In some embodiments, the method comprises administering to the human of from about 100 mg to about 2500 mg of bictegravir. In some embodiments, the method comprises administering to the human of from about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg. about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, or about 100 mg to about 200 mg.

In some embodiments, the method comprises administering to the human of from about 200 mg to about 1000 mg of bictegravir. In some embodiments, the method comprises administering to the human of from about 200 mg to about 900 mg of bictegravir. In some embodiments, the method comprises administering to the human of from about 200 mg to about 800 mg of bictegravir. In some embodiments, the method comprises administering to the human of from about 200 mg to about 700 mg of bictegravir. In some embodiments, the method comprises administering to the human of from about 200 mg to about 600 mg of bictegravir. In some embodiments, the method comprises administering to the human of from about 200 mg to about 500 mg of bictegravir.

In some embodiments, the method comprises administering to the human of from about 400 mg to about 600 mg of bictegravir. In some embodiments, the method comprises administering to the human of from about 450 mg to about 550 mg of bictegravir. In some embodiments, the method comprises administering to the human of from about 550 mg to about 600 mg of bictegravir. In some embodiments, the method comprises administering to the human of from about 500 mg to about 600 mg of bictegravir. In some embodiments, the method comprises administering to the human of from about 500 mg to about 550 mg of bictegravir.

In some embodiments, the method comprises administering to the human about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1125 mg, about 1150 mg, about 1175 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg, or about 2500 mg of bictegravir.

In some embodiments, the method comprises administering to the human about 250 mg of bictegravir.

In some embodiments, the method comprises administering to the human about 250 mg of bictegravir free acid.

In some embodiments, the method comprises administering to the human about 250 mg of a crystalline form of bictegravir free acid.

In some embodiments, the method comprises administering to the human about 250 mg of crystalline Form III of bictegravir free acid.

In some embodiments, the method comprises administering to the human about 251 mg of bictegravir.

In some embodiments, the method comprises administering to the human about 251 mg of bictegravir free acid.

In some embodiments, the method comprises administering to the human about 251 mg of a crystalline form of bictegravir free acid.

In some embodiments, the method comprises administering to the human about 251 mg of crystalline Form III of bictegravir free acid.

In some embodiments, the method comprises administering to the human about 900 mg of bictegravir.

In some embodiments, the method comprises administering to the human about 892 mg of bictegravir.

In some embodiments, the method comprises administering to the human about 600 mg of bictegravir.

In some embodiments, the method comprises administering to the human about 575 mg of bictegravir.

In some embodiments, the method comprises administering to the human about 550 mg of bictegravir.

In some embodiments, the method comprises administering to the human about 525 mg of bictegravir.

In some embodiments, the method comprises administering to the human about 500 mg of bictegravir.

In some embodiments, the method comprises administering to the human about 475 mg of bictegravir. In some embodiments, the method comprises administering to the human about 450 mg of bictegravir.

In some embodiments, the method comprises administering to the human about 446 mg of bictegravir.

In some embodiments, the method comprises administering to the human about 425 mg of bictegravir.

In some embodiments, the method comprises administering to the human about 400 mg of bictegravir.

In some embodiments, the method comprises administering to the human about 375 mg of bictegravir.

In some embodiments, the method comprises administering to the human about 300 mg of bictegravir.

In some embodiments, the method comprises administering to the human about 250 mg of bictegravir.

In some embodiments, the method comprises administering to the human about 150 mg of bictegravir.

In some embodiments, the method comprises administering to the human of from about 100 mg to about 2500 mg of bictegravir once every four weeks (Q4W). In some embodiments, the method comprises administering to the human of from about 100 mg to about 2500 mg, about 100 mg to about 2400 mg, about 100 mg to about 2300 mg, about 100 mg to about 2200 mg, about 100 mg to about 2100 mg, about 100 mg to about 2000 mg, about 100 mg to about 1900 mg. about 100 mg to about 1800 mg, about 100 mg to about 1700 mg, about 100 mg to about 1600 mg, about 100 mg to about 1500 mg, about 100 mg to about 1400 mg, about 100 mg to about 1300 mg, about 100 mg to about 1200 mg, about 100 mg to about 1100 mg, about 100 mg to about 1000 mg, about 100 mg to about 900 mg, about 100 mg to about 800 mg, about 100 mg to about 700 mg, about 100 mg to about 600 mg, about 100 mg to about 500 mg, about 100 mg to about 400 mg, about 100 mg to about 300 mg, or about 100 mg to about 200 mg once every four weeks (Q4W).

In some embodiments, the method comprises administering to the human of from about 200 mg to about 1000 mg of bictegravir once every four weeks (Q4W). In some embodiments, the method comprises administering to the human of from about 200 mg to about 900 mg of bictegravir once every four weeks (Q4W). In some embodiments, the method comprises administering to the human of from about 200 mg to about 800 mg of bictegravir once every four weeks (Q4W). In some embodiments, the method comprises administering to the human of from about 200 mg to about 700 mg of bictegravir once every four weeks (Q4W). In some embodiments, the method comprises administering to the human of from about 200 mg to about 600 mg of bictegravir once every four weeks (Q4W). In some embodiments, the method comprises administering to the human of from about 400 mg to about 600 mg of bictegravir once every four weeks (Q4W). In some embodiments, the method comprises administering to the human of from about 450 mg to about 550 mg of bictegravir once every four weeks (Q4W).

In some embodiments, the method comprises administering to the human of about 450 mg of bictegravir once every four weeks (Q4W).

In some embodiments, the method comprises administering to the human of about 475 mg of bictegravir once every four weeks (Q4W).

In some embodiments, the method comprises administering to the human of about 500 mg of bictegravir once every four weeks (Q4W).

In some embodiments, the method comprises administering to the human of about 525 mg of bictegravir once every four weeks (Q4W).

In some embodiments, the method comprises administering to the human of about 550 mg of bictegravir once every four weeks (Q4W).

In some embodiments, the method comprises administering to the human of about 575 mg of bictegravir once every four weeks (Q4W).

In some embodiments, the method comprises administering to the human of about 600 mg of bictegravir once every four weeks (Q4W).

In some embodiments, the method comprises administering to the human of about 450 mg of bictegravir free acid once every four weeks (Q4W).

In some embodiments, the method comprises administering to the human of about 475 mg of bictegravir free acid once every four weeks (Q4W).

In some embodiments, the method comprises administering to the human of about 500 mg of bictegravir free acid once every four weeks (Q4W).

In some embodiments, the method comprises administering to the human of about 525 mg of bictegravir free acid once every four weeks (Q4W).

In some embodiments, the method comprises administering to the human of about 550 mg of bictegravir free acid once every four weeks (Q4W).

In some embodiments, the method comprises administering to the human of about 575 mg of bictegravir free acid once every four weeks (Q4W).

In some embodiments, the method comprises administering to the human of about 600 mg of bictegravir free acid once every four weeks (Q4W).

In some embodiments, the method comprises administering to the human of about 450 mg of bictegravir free acid crystalline Form III once every four weeks (Q4W).

In some embodiments, the method comprises administering to the human of about 475 mg of bictegravir free acid crystalline Form III once every four weeks (Q4W).

In some embodiments, the method comprises administering to the human of about 500 mg of bictegravir free acid crystalline Form III once every four weeks (Q4W).

In some embodiments, the method comprises administering to the human of about 525 mg of bictegravir free acid crystalline Form III once every four weeks (Q4W).

In some embodiments, the method comprises administering to the human of about 550 mg of bictegravir free acid crystalline Form III once every four weeks (Q4W).

In some embodiments, the method comprises administering to the human of about 575 mg of bictegravir free acid crystalline Form III once every four weeks (Q4W).

In some embodiments, the method comprises administering to the human of about 600 mg of bictegravir free acid crystalline Form III once every four weeks (Q4W).

In some embodiments, the method comprising administering bictegravir to the human results in a release rate (kᵣₑₗₑₐₛₑ) of bictegravir of about 0.0005 1/h to about 0.005 1/h, 0.0005 1/h to about 0.004 1/h, about 0.001 1/h to about 0.003 1/h, about 0.002 1/h to about 0.004 1/h, about 0.003 1/h to about 0.004 1/h, about 0.0005 1/h to about 0.001 1/h, about 0.0005 1/h to about 0.0015 1/h, about 0.0005 1/h to about 0.002 1/h, about 0.001 1/h to about 0.0015 1/h, about 0.0015 1/h to about 0.002 1/h, about 0.002 1/h to about 0.0025 1/h, about 0.0025 1/h to about 0.003 1/h, about 0.0005 1/h to about 0.0006 1/h, about 0.0006 1/h to about 0.0007 1/h, about 0.0007 1/h to about 0.0008 1/h, about 0.0008 1/h to about 0.0009 1/h, about 0.0009 1/h to about 0.001 1/h, and about 0.001 1/h to about 0.0015 1/h.

In some embodiments, the method comprising administering bictegravir to the human results in a release rate (kᵣₑₗₑₐₛₑ) of bictegravir of about 0.0005 1/h or less, about 0.00055 1/h or less, about 0.0006 1/h or less, about 0.00065 1/h or less, about 0.0007 1/h or less, about 0.00075 1/h or less, about 0.0008 1/h or less, about 0.00085 1/h or less, about 0.0009 1/h or less, about 0.00095 1/h or less, about 0.001 1/h or less, about 0.0015 1/h or less, about 0.002 1/h or less, about 0.0025 1/h or less, about 0.003 1/h or less, or about 0.0035 1/h or less.

In some embodiments, the method comprising administering bictegravir to the human results in a release rate (kᵣₑₗₑₐₛₑ) of bictegravir of about 0.0007 1/h. In some embodiments, the method comprising administering bictegravir to the human results in a release rate (kᵣₑₗₑₐₛₑ) of bictegravir of about 0.0008 1/h.

In some embodiments, the method comprising administering bictegravir to the human results in a mean peak plasma concentration (Cₘₐₓ) of bictegravir of about 0.2 µM to about 2.0 µM, about 0.5 µM to about 2.0 µM, about 1.0 µM to about 2.0 µM, about 1.5 µM to about 2.0 µM, about 1.0 µM to about 1.8 µM, or about 1.0 µM to about 1.5 µM.

In some embodiments, the method comprising administering bictegravir to the human results in a mean peak plasma concentration (Cₘₐₓ) of bictegravir of about 1.5 µM. In some embodiments, the method comprising administering bictegravir to the human results in a mean peak plasma concentration (Cₘₐₓ) of bictegravir of about 1.1 µM.

In some embodiments, the method comprising administering bictegravir to the human results in a trough concentration of bictegravir of about 1.0 µM to about 3.0 µM, about 0.5 µM to about 2.0 µM, about 1.0 µM to about 2.0 µM, about 1.5 µM to about 2.0 µM, about 2.0 µM to about 2.5 µM, or about 2.5 µM to about 3.0 µM following repeat administration of BIC once every 4 weeks (Q4W). In some embodiments, the method comprising administering bictegravir to the human results in a trough concentration of bictegravir of at least about 1.0 µM, about 1.2 µM, about 1.4 µM, about 1.6 µM, about 1.8 µM, about 2.0 µM, about 2.2 µM, about 2.4 µM, about 2.6 µM, about 2.8 µM, or about 3.0 µM following repeat administration of BIC once every 4 weeks (Q4W). In some embodiments, the method comprising administering bictegravir to the human results in a trough concentration of bictegravir of at least about 1.8 µM following repeat administration of BIC once every 4 weeks (Q4W).

In some embodiments of the methods provided herein, the human is infected with HIV.

In some embodiments of the methods provided herein, the human has an HIV-1 RNA copy number of less than about 50 copies/mL (i.e., is virologically suppressed).

In some embodiments, the HIV infection is HIV-1 infection characterized by HIV-1 mutant resistance to one or more antiviral medications. In some embodiments, the HIV infection is an HIV-1 infection characterized by HIV-1 mutant resistance to two or more antiretroviral medications. In some embodiments, the HIV infection is an HIV-1 infection characterized by HIV-1 mutant resistance to three or more antiretroviral medications.

In some embodiments, the HIV-1 mutant is resistant to a protease inhibitor (PI), a nucleoside or nucleotide reverse transcriptase inhibitor (NRTI), a non-nucleoside or non-nucleotide reverse transcriptase inhibitor (NNRTI), or an integrase strand transfer inhibitor (INSTI). In certain embodiments, the HIV-1 mutant resistant to a protease inhibitor is selected from I50V, 184V/L90M, G48V/V82A/L90M, and G48V/V82S. In certain embodiments, the HIV-1 mutant resistant to a nucleoside or nucleotide reverse transcriptase inhibitor is selected from K65R, M184V, and 6TAMs. In certain embodiments, the HIV-1 mutant resistant to a non-nucleoside or non-nucleotide reverse transcriptase inhibitor is selected from K103N, Y181C, Y188L, L100I/K103N, and K103N/Y181C. In certain embodiments, the HIV-1 mutant resistant to an integrase strand transfer inhibitor is selected from Y143R, E138K/Q148K, G140S/Q148R, E92Q/N155H, N155H/Q148R, and R263K/M50I.

In some embodiments, the human (or "patient") is infected with HIV-1 resistant to at least one antiretroviral medication. In some embodiments, the patient is infected with multidrug resistant HIV-1 which is resistant to at least one antiretroviral medication from each of two different classes of antiretroviral medications. In some embodiments, the patient is infected with multidrug resistant HIV-1 which is resistant to at least one antiretroviral medication from each of three different classes of antiretroviral medications. In some embodiments, the different classes of antiretroviral medications are selected from a nucleoside or nucleotide reverse transcriptase inhibitor (NRTI), a non-nucleoside or non-nucleotide reverse transcriptase inhibitor (NNRTI), a protease inhibitor (PI), and an integrase strand transfer inhibitor (INSTI).

In some embodiments, the NRTI is selected from emtricitabine, lamivudine (3TC), zidovudine (azidothymidine (AZT)), didanosine (ddI), dideoxyinosine, tenofovir, tenofovir alafenamide, tenofovir alafenamide hemifumarate, tenofovir disoproxil fumarate, stavudine (d4T), zalcitabine (dideoxycytidine, ddC), and abacavir.

In some embodiments, the NNRTI is selected from efavirenz, etravirine, rilpivirine, nevirapine, and delavirdine.

In some embodiments, the PI is selected from amprenavir, atazanavir, darunavir, fosamprenavir, indinavir, lopinavir, nelfinavir, ritonavir, saquinavir, and tipranavir.

In some embodiments, the INSTI is selected from raltegravir, elvitegravir, and dolutegravir.

In some embodiments of the method, the patient had been previously treated with at least one antiretroviral medication for at least 3 months, at least 6 months, at least 9 months, or at least 12 months.

In some embodiments, the patient failed a prior HIV treatment regimen including administration of at least one antiretroviral medication. In certain embodiments, the prior treatment regimen included administration of at least one antiretroviral medication from each of two different classes of antiretroviral medications. In certain embodiments, the prior treatment regimen included administration of at least one antiretroviral medication from each of three different classes of antiretroviral medications. In some embodiments, the different classes of antiretroviral medications are selected from a nucleoside reverse transcriptase inhibitor (NRTI), a non-nucleoside reverse transcriptase inhibitor (NNRTI), a protease inhibitor (PI), and an integrase strand transfer inhibitor (INSTI).

In some embodiments of the methods provided herein, the bictegravir is administered as a long-acting injectable pharmaceutical composition (e.g., a solution or suspension formulation).

In some embodiments of the methods provided herein, the bictegravir is administered as a solution formulation.

In some embodiments of the methods provided herein, the bictegravir is administered as a suspension formulation.

In some embodiments of the methods provided herein, the bictegravir is administered as an oil-based suspension formulation.

In some embodiments of the methods provided herein, the bictegravir is administered as an aqueous suspension formulation.

In some embodiments of the methods provided herein, the bictegravir is administered subcutaneously.

In some embodiments of the methods provided herein, the bictegravir is administered intramuscularly.

In some embodiments of the methods provided herein, the bictegravir is administered in combination with one or more additional therapeutic agents (e.g., one, two, three, or four additional therapeutic agents).

In certain embodiments, the present description provides pharmaceutical compositions for use in a method for treating an HIV infection, comprising administering to a patient in need thereof a therapeutically effective amount of a composition described herein, in combination with a therapeutically effective amount of one or more additional therapeutic agents.

Combination or co-administration of bictegravir with one or more additional therapeutic agents generally refers to simultaneous or sequential administration of bictegravir and one or more additional therapeutic agents, such that therapeutically effective of bictegravir and the one or more additional therapeutic agents are both present in the body of the patient. When administered sequentially, the combination may be administered in two or more administrations.

Co-administration includes administration of unit dosages of the compounds disclosed herein before or after administration of unit dosages of one or more additional therapeutic agents. For example, the compound disclosed herein may be administered within seconds, minutes, or hours of the administration of the one or more additional therapeutic agents. In some embodiments, a unit dose of a compound disclosed herein is administered first, followed within seconds or minutes by administration of a unit dose of one or more additional therapeutic agents. Alternatively, a unit dose of one or more additional therapeutic agents is administered first, followed by administration of a unit dose of a compound disclosed herein within seconds or minutes. In other embodiments, a unit dose of a compound disclosed herein is administered first, followed, after a period of hours (e.g., 1-12 hours), by administration of a unit dose of one or more additional therapeutic agents. In yet other embodiments, a unit dose of one or more additional therapeutic agents is administered first, followed, after a period of hours (e.g., 1-12 hours), by administration of a unit dose of a compound disclosed herein.

In some embodiments, the additional therapeutic agent may be an anti-HIV agent. In some instances, the additional therapeutic agent can be HIV protease inhibitors, HIV non-nucleoside or non-nucleotide inhibitors of reverse transcriptase, HIV nucleoside or nucleotide inhibitors of reverse transcriptase, HIV integrase inhibitors, HIV non-catalytic site (or allosteric) integrase inhibitors, HIV entry inhibitors, HIV maturation inhibitors, HIV capsid inhibitors, HIV Tat or Rev inhibitors, immunomodulators, immunotherapeutic agents, antibody-drug conjugates, gene modifiers, gene editors (such as CRISPR/Cas9, zinc finger nucleases, homing nucleases, synthetic nucleases, TALENs), cell therapies (such as chimeric antigen receptor T-cell, CAR-T, and engineered T-cell receptors, TCR-T, autologous T-cell therapies, engineered B cells), latency reversing agents, immune-based therapies, phosphatidylinositol 3-kinase (PI3K) inhibitors, HIV antibodies, bispecific antibodies and "antibody-like" therapeutic proteins, HIV p17 matrix protein inhibitors, IL-13 antagonists, peptidyl-prolyl cis-trans isomerase A modulators, protein disulfide isomerase inhibitors, complement C5a receptor antagonists, DNA methyltransferase inhibitor, Fatty acid synthase inhibitor, HIV vif gene modulators, Vif dimerization antagonists, HIV-1 viral infectivity factor inhibitors, HIV-1 Nef modulators, TNF alpha ligand inhibitors, HIV Nef inhibitors, Hck tyrosine kinase modulators, mixed lineage kinase-3 (MLK-3) inhibitors, HIV-1 splicing inhibitors, integrin antagonists, nucleoprotein inhibitors, splicing factor modulators, COMM domain containing protein 1 modulators, HIV ribonuclease H inhibitors, IFN antagonists, retrocyclin modulators, CDK-4 inhibitors, CDK-6 inhibitors, CDK-9 inhibitors, dendritic ICAM-3 grabbing nonintegrin 1 inhibitors, HIV GAG protein inhibitors, HIV POL protein inhibitors, Complement Factor H modulators, ubiquitin ligase inhibitors, deoxycytidine kinase inhibitors, cyclin dependent kinase inhibitors, proprotein convertase PC9 stimulators, ATP dependent RNA helicase DDX3X inhibitors, reverse transcriptase priming complex inhibitors, G6PD and NADH-oxidase inhibitors, mTOR complex 1 inhibitors, mTOR complex 2 inhibitors, P-Glycoprotein modulators, TAT protein inhibitors, Prolylendopeptidase inhibitors, Phospholipase A2 inhibitors, pharmacokinetic enhancers, HIV gene therapy, HIV vaccines, and combinations thereof.

In some embodiments, the additional therapeutic agent is selected from the group consisting of combination drugs for HIV, other drugs for treating HIV, HIV protease inhibitors, HIV reverse transcriptase inhibitors, HIV integrase inhibitors, HIV non-catalytic site (or allosteric) integrase inhibitors, HIV entry (fusion) inhibitors, HIV maturation inhibitors, latency reversing agents, capsid inhibitors, immune-based therapies, PI3K inhibitors, HIV antibodies, and bispecific antibodies, and "antibody-like" therapeutic proteins, and combinations thereof.

### Combination Drugs

In certain embodiments, the agents described herein are combined with an HIV combination drug. Examples of combination drugs that can be employed with an agent of this disclosure include ATRIPLA^{®} (efavirenz, tenofovir disoproxil fumarate, and emtricitabine); COMPLERA^{®} (EVIPLERA^{®}; rilpivirine, tenofovir disoproxil fumarate, and emtricitabine); STRIBILD^{®} (elvitegravir, cobicistat, tenofovir disoproxil fumarate, and emtricitabine); TRUVADA^{®} (tenofovir disoproxil fumarate and emtricitabine; TDF+FTC); DESCOVY^{®} (tenofovir alafenamide and emtricitabine); ODEFSEY^{®} (tenofovir alafenamide, emtricitabine, and rilpivirine); GENVOYA^{®} (tenofovir alafenamide, emtricitabine, cobicistat, and elvitegravir); darunavir, tenofovir alafenamide hemifumarate, emtricitabine, and cobicistat; efavirenz, lamivudine, and tenofovir disoproxil fumarate; lamivudine and tenofovir disoproxil fumarate; tenofovir and lamivudine; tenofovir alafenamide and emtricitabine; tenofovir alafenamide hemifumarate and emtricitabine; tenofovir alafenamide hemifumarate, emtricitabine, and rilpivirine; tenofovir alafenamide hemifumarate, emtricitabine, cobicistat, and elvitegravir; tenofovir analogs; COMBIVIR^{®} (zidovudine and lamivudine; AZT+3TC); EPZICOM^{®} (LIVEXA^{®}; abacavir sulfate and lamivudine; ABC+3TC); KALETRA^{®} (ALUVIA^{®}; lopinavir and ritonavir); TRIUMEQ^{®} (dolutegravir, abacavir, and lamivudine); BIKTARVY (bictegravir + emtricitabine + tenofovir alafenamide), DOVATO, TRIZIVIR^{®} (abacavir sulfate, zidovudine, and lamivudine; ABC+AZT+3TC); atazanavir and cobicistat; atazanavir sulfate and cobicistat; atazanavir sulfate and ritonavir; darunavir and cobicistat; dolutegravir and rilpivirine; dolutegravir and rilpivirine hydrochloride; dolutegravir, abacavir sulfate, and lamivudine; lamivudine, nevirapine, and zidovudine; raltegravir and lamivudine; doravirine, lamivudine, and tenofovir disoproxil fumarate; doravirine, lamivudine, and tenofovir disoproxil; dolutegravir + lamivudine, lamivudine + abacavir + zidovudine, lamivudine + abacavir, lamivudine + tenofovir disoproxil fumarate, lamivudine + zidovudine + nevirapine, lopinavir + ritonavir, lopinavir + ritonavir + abacavir + lamivudine, lopinavir + ritonavir + zidovudine + lamivudine, tenofovir + lamivudine, and tenofovir disoproxil fumarate + emtricitabine + rilpivirine hydrochloride, lopinavir, ritonavir, zidovudine and lamivudine; cabotegravir + rilpivirine; elpida (elsulfavirine; VM-1500; VM-1500A).

Examples of other drugs for treating HIV that can be combined with an agent of this disclosure include aspernigrin C, acemannan, alisporivir, BanLec, deferiprone, Gamimune, metenkefalin, naltrexone, Prolastin, REP 9, RPI-MN, VSSP, H1viral, SB-728-T, 1,5-dicaffeoylquinic acid, rHIV7-shl-TAR-CCR5RZ, AAV-eCD4-Ig gene therapy, MazF gene therapy, BlockAide, bevirimat derivatives, ABX-464, AG-1105, APH-0812, bryostatin analogs, BIT-225, CYT-107, CS-TATI-1, fluoro-beta-D-arabinose nucleic acid (FANA)-modified antisense oligonucleotides, FX-101, griffithsin, HGTV-43, HPH-116, HS-10234, hydroxychloroquine, IMB-10035, IMO-3100, IND-02, JL-18008, LADAVRU, MK-1376, MK-2048, MK-4250, MK-8507, MK-8558, MK-8591 (islatravir), NOV-205, OB-002H, ODE-Bn-TFV, M1-TFV, PA-1050040 (PA-040), PC-707, PGN-007, QF-036, S-648414, SCY-635, SB-9200, SCB-719, TR-452, TEV-90110, TEV-90112, TEV-90111, TEV-90113, RN-18, DIACC-1010, Fasnall, Immuglo, 2-CLIPS peptide, HRF-4467, thrombospondin analogs, TBL-1004HI, VG-1177, xl-081, rfhSP-D, [18F]-MC-225, URMC-099-C, RES-529, and VIR-576.

### HIV Protease Inhibitors

In certain embodiments, the agents described herein are combined with an HIV protease inhibitor. Examples of HIV protease inhibitors that can be combined with an agent of this disclosure include amprenavir, atazanavir, brecanavir, darunavir, fosamprenavir, fosamprenavir calcium, indinavir, indinavir sulfate, lopinavir, nelfinavir, nelfinavir mesylate, ritonavir, saquinavir, saquinavir mesylate, tipranavir, AEBL-2, DG-17, GS-1156, TMB-657 (PPL-100), T-169, BL-008, MK-8122, TMB-607, GRL-02031, and TMC-310911. Additional examples are compounds as disclosed in US 2018258097, and US2020030327.

### HIV Ribonuclease H Inhibitors

In certain embodiments, the agents described herein are combined with an HIV ribonuclease H inhibitor. Examples of HIV ribonuclease H inhibitors that can be combined with an agent of this disclosure include NSC-727447.

### HIV Nef Inhibitors

In certain embodiments, the agents described herein are combined with an HIV Nef inhibitor. Examples of HIV Nef inhibitors that can be combined with an agent of this disclosure include FP-1.

### HIV Reverse Transcriptase Inhibitors

In certain embodiments, the agents described herein are combined with a non-nucleoside or non-nucleotide inhibitor. Examples of HIV non-nucleoside or non-nucleotide inhibitors of reverse transcriptase that can be combined with an agent of this disclosure include dapivirine, delavirdine, delavirdine mesylate, doravirine, efavirenz, etravirine, lentinan, nevirapine, rilpivirine, ACC-007, ACC-008, AIC-292, F-18, KM-023, PC-1005, VM-1500A-LAI, PF-3450074, elsulfavirine (sustained release oral, HIV infection), elsulfavirine (long-acting injectable nanosuspension, HIV infection), and elsulfavirine (VM-1500).

### Long-Acting HIV drugs

In certain embodiments, the agents described herein are combined with a long-acting drug. Examples of drugs that are being developed as long acting regimens: cabotegravir LA, rilpivirine LA, cabotegravir LA + rilpivirine LA, elvitegravir (extended release), lenacapavir long acting, raltegravir long acting, darunavir long acting, any integrase LA, VM-1500A--LAI, VM-3500, maraviroc (LAI), T-1144, ODE-Bn-TFV, CP-112, S-648414, tenofovir implant, tenofovir long acting, tenofovir prodrug long acting, MK-8591 subdermal implant, long-acting dolutegravir, long acting raltegravir + lamivudine, transdermal devices that can deliver HIV drugs, such as transdermal tenofovir (WO2020092990).

### HIV Nucleoside or Nucleotide inhibitor

In certain embodiments, the agents described herein are combined with an HIV nucleoside or nucleotide inhibitor. Examples of HIV nucleoside or nucleotide inhibitors of reverse transcriptase that can be combined with an agent of this disclosure include adefovir, adefovir dipivoxil, azvudine, emtricitabine, tenofovir, tenofovir alafenamide, tenofovir alafenamide fumarate, tenofovir alafenamide hemifumarate, tenofovir disoproxil, tenofovir disoproxil fumarate, tenofovir octadecyloxyethyl ester (AGX-1009), tenofovir disoproxil hemifumarate, VIDEX^{®} and VIDEX EC^{®} (didanosine, ddl), abacavir, abacavir sulfate, alovudine, apricitabine, censavudine, didanosine, elvucitabine, festinavir, fosalvudine tidoxil, CMX-157, dapivirine, doravirine, etravirine, OCR-5753, tenofovir disoproxil orotate, fozivudine tidoxil, lamivudine, phosphazid, stavudine, zalcitabine, zidovudine, rovafovir etalafenamide (GS-9131), GS-9148, MK-8504, MK-8591, MK-8583, VM-2500 and KP-1461.

### HIV Integrase Inhibitors

In certain embodiments, the agents described herein are combined with an HIV integrase inhibitor. Examples of HIV integrase inhibitors that can be combined with an agent of this disclosure include elvitegravir, elvitegravir (extended-release microcapsules), curcumin, derivatives of curcumin, chicoric acid, derivatives of chicoric acid, 3,5-dicaffeoylquinic acid, derivatives of 3,5-dicaffeoylquinic acid, aurintricarboxylic acid, derivatives of aurintricarboxylic acid, caffeic acid phenethyl ester, derivatives of caffeic acid phenethyl ester, tyrphostin, derivatives of tyrphostin, quercetin, derivatives of quercetin, raltegravir, PEGylated raltegravir, dolutegravir, JTK-351, bictegravir, AVX-15567, cabotegravir (long-acting injectable), diketo quinolin-4-1 derivatives, integrase-LEDGF inhibitor, ledgins, M-522, M-532, MK-0536, NSC-310217, NSC-371056, NSC-48240, NSC-642710, NSC-699171, NSC-699172, NSC-699173, NSC-699174, stilbenedisulfonic acid, T169, STP-0404, VM-3500 and cabotegravir.

In certain embodiments, the agents described herein are combined with a HIV non-catalytic site, or allosteric, integrase inhibitor (NCINI). Examples of HIV non-catalytic site, or allosteric, integrase inhibitors (NCINI) that can be combined with an agent of this disclosure include CX-05045, CX-05168, and CX-14442.

### HIV Entry Inhibitors

In certain embodiments, the agents described herein are combined with an HIV entry inhibitor. Examples of HIV entry (fusion) inhibitors that can be combined with an agent of this disclosure include AAR-501, LBT-5001, cenicriviroc, CCR5 inhibitors, gp41 inhibitors, CD4 attachment inhibitors, gp120 inhibitors, gp160 inhibitors, and CXCR4 inhibitors.

In certain embodiments, the agents described herein are combined with a CCR5 inhibitor. Examples of CCR5 inhibitors that can be combined with an agent of this disclosure include aplaviroc, vicriviroc, maraviroc, maraviroc (long-acting injectable nanoemulsion), cenicriviroc, leronlimab (PRO-140), adaptavir (RAP-101), nifeviroc (TD-0232), anti-GP120/CD4 or CCR5 bispecific antibodies, B-07, MB-66, polypeptide C25P, TD-0680, thioraviroc and vMIP (Haimipu).

In certain embodiments, the agents described herein are combined with a gp41 inhibitor. Examples of gp41 inhibitors that can be combined with an agent of this disclosure include albuvirtide, enfuvirtide, griffithsin (gp41/gp120/gp160 inhibitor), BMS-986197, enfuvirtide biobetter, enfuvirtide biosimilar, HIV-1 fusion inhibitors (P26-Bapc), ITV-1, ITV-2, ITV-3, ITV-4, CPT-31, Cl3hmAb, PIE-12 trimer and sifuvirtide.

In certain embodiments, the agents are combined with a CD4 attachment inhibitor. Examples of CD4 attachment inhibitors that can be combined with an agent of this disclosure include ibalizumab and CADA analogs.

In certain embodiments, the agents described herein are combined with a gp120 inhibitor. Examples of gp120 inhibitors that can be combined with an agent of this disclosure include anti-HIV microbicide, Radha-108 (receptol) 3B3-PE38, BanLec, bentonite-based nanomedicine, fostemsavir tromethamine, IQP-0831, VVX-004, and BMS-663068.

In certain embodiments, the agents described herein are combined with a gp160 inhibitor. Examples of gp160 inhibitors that can be combined with an agent of this disclosure include fangchinoline.

In certain embodiments, the agent described herein are combined with a CXCR4 inhibitor. Examples of CXCR4 inhibitors that can be combined with an agent of this disclosure include plerixafor, ALT-1188, N15 peptide, and vMIP (Haimipu).

In certain embodiments, the agents described herein are combined with a HIV maturation inhibitor. Examples of HIV maturation inhibitors that can be combined with an agent of this disclosure include BMS-955176, GSK-3640254 and GSK-2838232.

### Latency Reversing Agents

In certain embodiments, the agents described herein are combined with a latency reversing agent (LRA). Examples of latency reversing agents that can be combined with an agent of this disclosure include toll-like receptor (TLR) agonists (including TLR7 agonists, e.g., GS-9620), histone deacetylase (HDAC) inhibitors, proteasome inhibitors such as velcade, protein kinase C (PKC) activators, Smyd2 inhibitors, BET-bromodomain 4 (BRD4) inhibitors, ionomycin, IAP antagonists (inhibitor of apoptosis proteins, such as APG-1387, LBW-242), SMAC mimetics (including TL32711, LCL161, GDC-0917, HGS1029, AT-406, Debio-1143), PMA, SAHA (suberanilohydroxamic acid, or suberoyl, anilide, and hydroxamic acid), NIZ-985, IL-15 modulating antibodies (including IL-15, IL-15 fusion proteins and IL-15 receptor agonists), JQ1, disulfiram, amphotericin B, and ubiquitin inhibitors such as largazole analogs, APH-0812, and GSK-343. Examples of PKC activators include indolactam, prostratin, ingenol B, and DAG-lactones.

### Histone Deacetylase (HDAC) Inhibitors

In various embodiments, the agents as described herein are combined with an inhibitor of a histone deacetylase, e.g., histone deacetylase 1, histone deacetylase 9 (HDAC9, HD7, HD7b, HD9, HDAC, HDAC7, HDAC7B, HDAC9B, HDAC9FL, HDRP, MITR; Gene ID: 9734). Examples of HDAC inhibitors include without limitation, abexinostat, ACY-241, AR-42, BEBT-908, belinostat, CKD-581, CS-055 (HBI-8000), CT-101, CUDC-907 (fimepinostat), entinostat, givinostat, mocetinostat, panobinostat, pracinostat, quisinostat (JNJ-26481585), resminostat, ricolinostat, romidepsin, SHP-141, TMB-ADC, valproic acid (VAL-001), vorinostat, tinostamustine, remetinostat, and entinostat.

In one embodiment, an agent disclosed herein, or a pharmaceutically acceptable salt thereof, is combined with one, two, three, four or more additional therapeutic agents selected from ATRIPLA^{®} (efavirenz, tenofovir disoproxil fumarate, and emtricitabine); COMPLERA^{®} (EVIPLERA^{®}; rilpivirine, tenofovir disoproxil fumarate, and emtricitabine); STRIBILD^{®} (elvitegravir, cobicistat, tenofovir disoproxil fumarate, and emtricitabine); TRUVADA^{®} (tenofovir disoproxil fumarate and emtricitabine; TDF +FTC); DESCOVY^{®} (tenofovir alafenamide and emtricitabine); ODEFSEY^{®} (tenofovir alafenamide, emtricitabine, and rilpivirine); GENVOYA^{®} (tenofovir alafenamide, emtricitabine, cobicistat, and elvitegravir); BIKTARVY (bictegravir + emtricitabine + tenofovir alafenamide), adefovir; adefovir dipivoxil; cobicistat; emtricitabine; tenofovir; tenofovir alafenamide and elvitegravir; tenofovir disoproxil; tenofovir disoproxil fumarate; tenofovir alafenamide; tenofovir alafenamide hemifumarate; TRIUMEQ^{®} (dolutegravir, abacavir, and lamivudine); dolutegravir, abacavir sulfate, and lamivudine; raltegravir; PEGylated raltegravir; raltegravir and lamivudine; maraviroc; tenofovir + emtricitabine + maraviroc, enfuvirtide; ALUVIA^{®} (KALETRA^{®}; lopinavir and ritonavir); COMBIVIR^{®} (zidovudine and lamivudine; AZT+3TC); EPZICOM^{®} (LIVEXA^{®}; abacavir sulfate and lamivudine; ABC+3TC); TRIZIVIR^{®} (abacavir sulfate, zidovudine, and lamivudine; ABC+AZT+3TC); rilpivirine; rilpivirine hydrochloride; atazanavir sulfate and cobicistat; atazanavir and cobicistat; darunavir and cobicistat; atazanavir; atazanavir sulfate; dolutegravir; elvitegravir; ritonavir; atazanavir sulfate and ritonavir; darunavir; lamivudine; prolastin; fosamprenavir; fosamprenavir calcium efavirenz; etravirine; nelfinavir; nelfinavir mesylate; interferon; didanosine; stavudine; indinavir; indinavir sulfate; tenofovir and lamivudine; zidovudine; nevirapine; saquinavir; saquinavir mesylate; aldesleukin; zalcitabine; tipranavir; amprenavir; delavirdine; delavirdine mesylate; Radha-108 (receptol); lamivudine and tenofovir disoproxil fumarate; efavirenz, lamivudine, and tenofovir disoproxil fumarate; phosphazid; lamivudine, nevirapine, and zidovudine; abacavir; and abacavir sulfate.

In some embodiments, an agent disclosed herein, or a pharmaceutical composition thereof, is combined with an HIV nucleoside or nucleotide inhibitor of reverse transcriptase and an HIV non-nucleoside inhibitor of reverse transcriptase. In another specific embodiment, an agent disclosed herein, or a pharmaceutical composition thereof, is combined with an HIV nucleoside or nucleotide inhibitor of reverse transcriptase, and an HIV protease inhibiting compound. In an additional embodiment, an agent disclosed herein, or a pharmaceutical composition thereof, is combined with an HIV nucleoside or nucleotide inhibitor of reverse transcriptase, an HIV non-nucleoside inhibitor of reverse transcriptase, and a pharmacokinetic enhancer. In certain embodiments, an agent disclosed herein, or a pharmaceutical composition thereof, is combined with at least one HIV nucleoside inhibitor of reverse transcriptase, an integrase inhibitor, and a pharmacokinetic enhancer. In another embodiment, an agent disclosed herein, or a pharmaceutical composition thereof, is combined with two HIV nucleoside or nucleotide inhibitors of reverse transcriptase.

In a certain embodiment, an agent disclosed herein, or a pharmaceutical composition thereof, is combined with abacavir sulfate, tenofovir, tenofovir disoproxil, tenofovir disoproxil fumarate, tenofovir disoproxil hemifumarate, tenofovir alafenamide, or tenofovir alafenamide hemifumarate.

In another embodiment, an agent disclosed herein, or a pharmaceutical composition thereof, is combined with tenofovir, tenofovir disoproxil, tenofovir disoproxil fumarate, tenofovir alafenamide, or tenofovir alafenamide hemifumarate.

In yet another embodiment, an agent disclosed herein, or a pharmaceutical composition thereof, is combined with a first additional therapeutic agent selected from the group consisting of abacavir sulfate, tenofovir, tenofovir disoproxil, tenofovir disoproxil fumarate, tenofovir alafenamide, and tenofovir alafenamide hemifumarate, and a second additional therapeutic agent selected from the group consisting of emtricitabine and lamivudine.

In another embodiment, an agent disclosed herein, or a pharmaceutical composition thereof, is combined with a first additional therapeutic agent selected from the group consisting of tenofovir, tenofovir disoproxil, tenofovir disoproxil fumarate, tenofovir alafenamide, and tenofovir alafenamide hemifumarate, and a second additional therapeutic agent, wherein the second additional therapeutic agent is emtricitabine.

### Capsid Inhibitor

In certain embodiments, the agents described herein are combined with a capsid inhibitor. Examples of capsid inhibitors that can be combined with an agent of this disclosure include capsid polymerization inhibitors or capsid disrupting compounds, HIV nucleocapsid p7 (NCp7) inhibitors such as azodicarbonamide, HIV p24 capsid protein inhibitors, GS-6207, GS-CA1, AVI-621, AVI-101, AVI-201, AVI-301, and AVI-CAN1-15 series, PF-3450074, and compounds described in this patent (GSK WO2019/087016).

In some embodiments, examples of capsid inhibitors include: 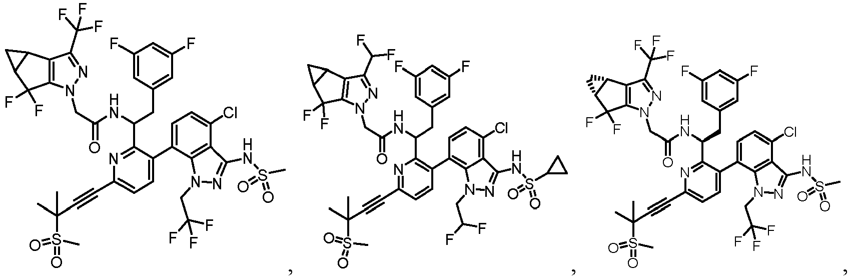 and 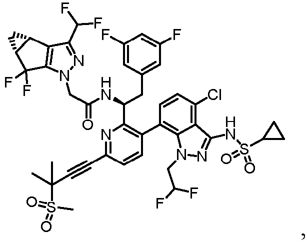 or a pharmaceutically acceptable salt thereof.

In some embodiments, the capsid inhibitor is selected from: 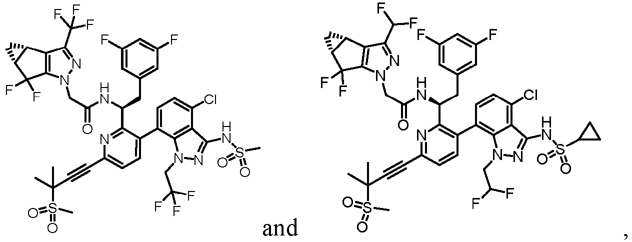 or a pharmaceutically acceptable salt thereof.

In some embodiments, the capsid inhibitor is: 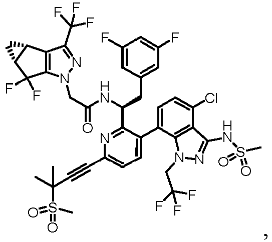 or a pharmaceutically acceptable salt thereof.

In some embodiments, the capsid inhibitor is: 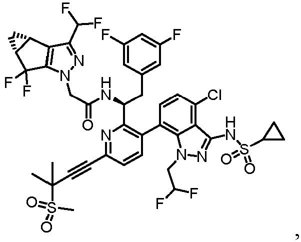 or a pharmaceutically acceptable salt thereof. In a specific embodiment, the additional therapeutic agents are each independently selected from HIV capsid inhibitors, HIV protease inhibitors, HIV non-nucleoside or non-nucleotide inhibitors of reverse transcriptase, HIV nucleoside or nucleotide inhibitors of reverse transcriptase, HIV integrase inhibitors, and HIV entry inhibitors (e.g., CCR5 inhibitors).

In some embodiments, at least one of the additional therapeutic agents is an HIV capsid inhibitor.

In some embodiments, the HIV capsid inhibitor is a compound of Formula (III), N-((S)-1-(3-(4-chloro-3-(methylsulfonamido)-1-(2,2,2-trifluoroethyl)-1H-indazol-7-yl)-6-(3-methyl-3-(methylsulfonyl)but-1-yn-1-yl)pyridin-2-yl)-2-(3,5-difluorophenyl)ethyl)-2-((3bS,4aR)-5,5-difluoro-3-(trifluoromethyl)-3b,4,4a,5-tetrahydro-1H-cyclopropa[3,4]cyclopenta[1,2-c]pyrazol-1-yl)acetamide, having the following structure: 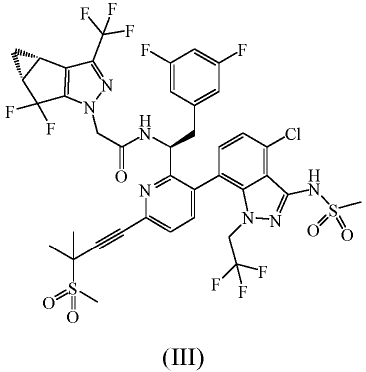 or a pharmaceutically acceptable salt thereof. A compound of Formula (III), or a pharmaceutically acceptable salt thereof, can be synthesized by the methods disclosed in US Patent No. 10071985 and US Patent Application Publication No. 2019-0300505.

In some embodiments, the compound of Formula (III) is a sodium salt. Crystalline forms of the compound of Formula (III) sodium salt are disclosed in US Patent Application Publication No. 2019-0084963.

In some embodiments, the compound of Formula (III) is a salt form selected from a potassium salt, methanesulfonic acid salt, ethanesulfonic acid salt, benzenesulfonic acid salt, hydrochloric acid salt, and sulfuric acid salt. These salt forms, as well as crystalline forms of the same, are disclosed in US Patent Application Publication No. 2019-0084963.

In some embodiments, the compound of Formula (III) is a choline salt. The choline salt of the compound of Formula (III) and crystalline forms thereof are disclosed in US Patent Application Publication No. 2019-0083478.

Formulations comprising a compound of Formula (III), or a pharmaceutically acceptable salt thereof, are disclosed in US Patent Application Publication No. 2020-0038389.

### Immune Checkpoint Modulators

In various embodiments, the agents as described herein, are combined with one or more blockers or inhibitors of inhibitory immune checkpoint proteins or receptors and/or with one or more stimulators, activators or agonists of one or more stimulatory immune checkpoint proteins or receptors. Blockade or inhibition of inhibitory immune checkpoints can positively regulate T-cell or NK cell activation and prevent immune escape of infected cells. Activation or stimulation of stimulatory immune check points can augment the effect of immune checkpoint inhibitors in infective therapeutics. In various embodiments, the immune checkpoint proteins or receptors regulate T cell responses (e.g., reviewed in Xu, et al., J Exp Clin Cancer Res. (2018) 37:110). In various embodiments, the immune checkpoint proteins or receptors regulate NK cell responses (e.g., reviewed in Davis, et al., Semin Immunol. (2017) 31:64-75 and Chiossone, et al., Nat Rev Immunol. (2018) 18(11):671-688).

Examples of immune checkpoint proteins or receptors include without limitation CD27, CD70; CD40, CD40LG; CD47, CD48 (SLAMF2), transmembrane and immunoglobulin domain containing 2 (TMIGD2, CD28H), CD84 (LY9B, SLAMF5), CD96, CD160, MS4A1 (CD20), CD244 (SLAMF4); CD276 (B7H3); V-set domain containing T cell activation inhibitor 1 (VTCN1, B7H4); V-set immunoregulatory receptor (VSIR, B7H5, VISTA); immunoglobulin superfamily member 11 (IGSF11, VSIG3); natural killer cell cytotoxicity receptor 3 ligand 1 (NCR3LG1, B7H6); HERV-H LTR-associating 2 (HHLA2, B7H7); inducible T cell costimulator (ICOS, CD278); inducible T cell costimulator ligand (ICOSLG, B7H2); TNF receptor superfamily member 4 (TNFRSF4, OX40); TNF superfamily member 4 (TNFSF4, OX40L); TNFRSF8 (CD30), TNFSF8 (CD30L); TNFRSF10A (CD261, DR4, TRAILR1), TNFRSF9 (CD137), TNFSF9 (CD137L); TNFRSF10B (CD262, DR5, TRAILR2), TNFRSF10 (TRAIL); TNFRSF14 (HVEM, CD270), TNFSF14 (HVEML); CD272 (B and T lymphocyte associated (BTLA)); TNFRSF17 (BCMA, CD269), TNFSF13B (BAFF); TNFRSF18 (GITR), TNFSF18 (GITRL); MHC class I polypeptide-related sequence A (MICA); MHC class I polypeptide-related sequence B (MICB); CD274 (CD274, PDL1, PD-L1); programmed cell death 1 (PDCD1, PD1, PD-1); cytotoxic T-lymphocyte associated protein 4 (CTLA4, CD152); CD80 (B7-1), CD28; nectin cell adhesion molecule 2 (NECTIN2, CD112); CD226 (DNAM-1); Poliovirus receptor (PVR) cell adhesion molecule (PVR, CD155); PVR related immunoglobulin domain containing (PVRIG, CD112R); T cell immunoreceptor with Ig and ITIM domains (TIGIT); T cell immunoglobulin and mucin domain containing 4 (TIMD4; TIM4); hepatitis A virus cellular receptor 2 (HAVCR2, TIMD3, TIM3); galectin 9 (LGALS9); lymphocyte activating 3 (LAG3, CD223); signaling lymphocytic activation molecule family member 1 (SLAMF1, SLAM, CD150); lymphocyte antigen 9 (LY9, CD229, SLAMF3); SLAM family member 6 (SLAMF6, CD352); SLAM family member 7 (SLAMF7, CD319); UL16 binding protein 1 (ULBP1); UL16 binding protein 2 (ULBP2); UL16 binding protein 3 (ULBP3); retinoic acid early transcript 1E (RAET1E; ULBP4); retinoic acid early transcript 1G (RAET1G; ULBP5); retinoic acid early transcript 1L (RAET1L; ULBP6); lymphocyte activating 3 (CD223); killer cell immunoglobulin like receptor, three Ig domains and long cytoplasmic tail 1 (KIR, CD158E1); killer cell lectin like receptor C1 (KLRC1, NKG2A, CD159A); killer cell lectin like receptor K1 (KLRK1, NKG2D, CD314); killer cell lectin like receptor C2 (KLRC2, CD159c, NKG2C); killer cell lectin like receptor C3 (KLRC3, NKG2E); killer cell lectin like receptor C4 (KLRC4, NKG2F); killer cell immunoglobulin like receptor, two Ig domains and long cytoplasmic tail 1 (KIR2DL1); killer cell immunoglobulin like receptor, two Ig domains and long cytoplasmic tail 2 (KIR2DL2); killer cell immunoglobulin like receptor, two Ig domains and long cytoplasmic tail 3 (KIR2DL3); killer cell immunoglobulin like receptor, three Ig domains and long cytoplasmic tail 1 (KIR3DL1); killer cell lectin like receptor D1 (KLRD1); and SLAM family member 7 (SLAMF7).

In various embodiments, the agents described herein, are combined with one or more blockers or inhibitors of one or more T-cell inhibitory immune checkpoint proteins or receptors. Illustrative T-cell inhibitory immune checkpoint proteins or receptors include without limitation CD274 (CD274, PDL1, PD-L1); programmed cell death 1 ligand 2 (PDCD1LG2, PD-L2, CD273); programmed cell death 1 (PDCD1, PD1, PD-1); cytotoxic T-lymphocyte associated protein 4 (CTLA4, CD152); CD276 (B7H3); V-set domain containing T cell activation inhibitor 1 (VTCN1, B7H4); V-set immunoregulatory receptor (VSIR, B7H5, VISTA); immunoglobulin superfamily member 11 (IGSF11, VSIG3); TNFRSF14 (HVEM, CD270), TNFSF14 (HVEML); CD272 (B and T lymphocyte associated (BTLA)); PVR related immunoglobulin domain containing (PVRIG, CD112R); T cell immunoreceptor with Ig and ITIM domains (TIGIT); lymphocyte activating 3 (LAG3, CD223); hepatitis A virus cellular receptor 2 (HAVCR2, TIMD3, TIM3); galectin 9 (LGALS9); killer cell immunoglobulin like receptor, three Ig domains and long cytoplasmic tail 1 (KIR, CD158E1); killer cell immunoglobulin like receptor, two Ig domains and long cytoplasmic tail 1 (KIR2DL1); killer cell immunoglobulin like receptor, two Ig domains and long cytoplasmic tail 2 (KIR2DL2); killer cell immunoglobulin like receptor, two Ig domains and long cytoplasmic tail 3 (KIR2DL3); and killer cell immunoglobulin like receptor, three Ig domains and long cytoplasmic tail 1 (KIR3DL1). In various embodiments, the agents, as described herein, are combined with one or more agonist or activators of one or more T-cell stimulatory immune checkpoint proteins or receptors. Illustrative T-cell stimulatory immune checkpoint proteins or receptors include without limitation CD27, CD70; CD40, CD40LG; inducible T cell costimulator (ICOS, CD278); inducible T cell costimulator ligand (ICOSLG, B7H2); TNF receptor superfamily member 4 (TNFRSF4, OX40); TNF superfamily member 4 (TNFSF4, OX40L); TNFRSF9 (CD137), TNFSF9 (CD137L); TNFRSF18 (GITR), TNFSF18 (GITRL); CD80 (B7-1), CD28; nectin cell adhesion molecule 2 (NECTIN2, CD112); CD226 (DNAM-1); CD244 (2B4, SLAMF4), Poliovirus receptor (PVR) cell adhesion molecule (PVR, CD155). *See, e.g.,* Xu, et al., J Exp Clin Cancer Res. (2018) 37:110.

In various embodiments, the agents as described herein, are combined with one or more blockers or inhibitors of one or more NK-cell inhibitory immune checkpoint proteins or receptors. Illustrative NK-cell inhibitory immune checkpoint proteins or receptors include without limitation killer cell immunoglobulin like receptor, three Ig domains and long cytoplasmic tail 1 (KIR, CD158E1); killer cell immunoglobulin like receptor, two Ig domains and long cytoplasmic tail 1 (KIR2DL1); killer cell immunoglobulin like receptor, two Ig domains and long cytoplasmic tail 2 (KIR2DL2); killer cell immunoglobulin like receptor, two Ig domains and long cytoplasmic tail 3 (KIR2DL3); killer cell immunoglobulin like receptor, three Ig domains and long cytoplasmic tail 1 (KIR3DL1); killer cell lectin like receptor C1 (KLRC1, NKG2A, CD159A); and killer cell lectin like receptor D1 (KLRD1, CD94). In various embodiments, the agents as described herein, are combined with one or more agonist or activators of one or more NK-cell stimulatory immune checkpoint proteins or receptors. Illustrative NK-cell stimulatory immune checkpoint proteins or receptors include without limitation CD16, CD226 (DNAM-1); CD244 (2B4, SLAMF4); killer cell lectin like receptor K1 (KLRK1, NKG2D, CD314); SLAM family member 7 (SLAMF7). *See, e.g.,* Davis, et al., Semin Immunol. (2017) 31:64-75; Fang, et al., Semin Immunol. (2017) 31:37-54; and Chiossone, et al., Nat Rev Immunol. (2018) 18(11):671-688.

In some embodiments, the one or more immune checkpoint inhibitors comprises a proteinaceous (e.g., antibody or fragment thereof, or antibody mimetic) inhibitor of PD-L1 (CD274), PD-1 (PDCD1) or CTLA4. In some embodiments, the one or more immune checkpoint inhibitors comprises a small organic molecule inhibitor of PD-L1 (CD274), PD-1 (PDCD1) or CTLA4. In some embodiments, the small molecule inhibitor of CD274 or PDCD1 is selected from the group consisting of GS-4224, GS-4416, INCB086550 and MAX10181. In some embodiments, the small molecule inhibitor of CTLA4 comprises BPI-002.

Examples of inhibitors of CTLA4 that can be co-administered include without limitation ipilimumab, tremelimumab, BMS-986218, AGEN1181, AGEN1884, BMS-986249, MK-1308, REGN-4659, ADU-1604, CS-1002, BCD-145, APL-509, JS-007, BA-3071, ONC-392, AGEN-2041, JHL-1155, KN-044, CG-0161, ATOR-1144, PBI-5D3H5, BPI-002, as well as multi-specific inhibitors FPT-155 (CTLA4/PD-L1/CD28), PF-06936308 (PD-1/CTLA4), MGD-019 (PD-1/CTLA4), KN-046 (PD-1/CTLA4), MEDI-5752 (CTLA4/PD-1), XmAb-20717 (PD-1/CTLA4), and AK-104 (CTLA4/PD-1).

Examples of inhibitors of PD-L1 (CD274) or PD-1 (PDCD1) that can be co-administered include without limitation pembrolizumab, nivolumab, cemiplimab, pidilizumab, AMP-224, MEDI0680 (AMP-514), spartalizumab, atezolizumab, avelumab, durvalumab, BMS-936559, CK-301, PF-06801591, BGB-A317 (tislelizumab), GLS-010 (WBP-3055), AK-103 (HX-008), AK-105, CS-1003, HLX-10, MGA-012, BI-754091, AGEN-2034, JS-001 (toripalimab), JNJ-63723283, genolimzumab (CBT-501), LZM-009, BCD-100, LY-3300054, SHR-1201, SHR-1210 (camrelizumab), Sym-021, ABBV-181(budigalimab), PD1-PIK, BAT-1306, (MSB0010718C), CX-072, CBT-502, TSR-042 (dostarlimab), MSB-2311, JTX-4014, BGB-A333, SHR-1316, CS-1001 (WBP-3155, KN-035, IBI-308 (sintilimab), HLX-20, KL-A167, STI-A1014, STI-A1015 (IMC-001), BCD-135, FAZ-053, TQB-2450, MDX1105-01, GS-4224, GS-4416, INCB086550, MAX10181, as well as multi-specific inhibitors FPT-155 (CTLA4/PD-L1/CD28), PF-06936308 (PD-1/CTLA4), MGD-013 (PD-1/LAG-3), FS-118 (LAG-3/PD-L1) MGD-019 (PD-1/CTLA4), KN-046 (PD-1/CTLA4), MEDI-5752 (CTLA4/PD-1), RO-7121661 (PD-1/TIM-3), XmAb-20717 (PD-1/CTLA4), AK-104 (CTLA4/PD-1), M7824 (PD-L1/TGFB-EC domain), CA-170 (PD-L1/VISTA), CDX-527 (CD27/PD-L1), LY-3415244 (TIM3/PDL1), and INBRX-105 (4-1BB/PDL1).

In various embodiments, the agents as described herein are combined with anti-TIGIT antibodies, such as BMS-986207, RG-6058, AGEN-1307.

### TNF Receptor Superfamily (TNFRSF) Member Agonists or Activators

In various embodiments, the agents as described herein are combined with an agonist of one or more TNF receptor superfamily (TNFRSF) members, e.g., an agonist of one or more of TNFRSF1A (NCBI Gene ID: 7132), TNFRSF1B (NCBI Gene ID: 7133), TNFRSF4 (OX40, CD134; NCBI Gene ID: 7293), TNFRSF5 (CD40; NCBI Gene ID: 958), TNFRSF6 (FAS, NCBI Gene ID: 355), TNFRSF7 (CD27, NCBI Gene ID: 939), TNFRSF8 (CD30, NCBI Gene ID: 943), TNFRSF9 (4-1BB, CD137, NCBI Gene ID: 3604), TNFRSF10A (CD261, DR4, TRAILR1, NCBI Gene ID: 8797), TNFRSF10B (CD262, DR5, TRAILR2, NCBI Gene ID: 8795), TNFRSF10C (CD263, TRAILR3, NCBI Gene ID: 8794), TNFRSF10D (CD264, TRAILR4, NCBI Gene ID: 8793), TNFRSF11A (CD265, RANK, NCBI Gene ID: 8792), TNFRSF11B (NCBI Gene ID: 4982), TNFRSF12A (CD266, NCBI Gene ID: 51330), TNFRSF13B (CD267, NCBI Gene ID: 23495), TNFRSF13C (CD268, NCBI Gene ID: 115650), TNFRSF16 (NGFR, CD271, NCBI Gene ID: 4804), TNFRSF17 (BCMA, CD269, NCBI Gene ID: 608), TNFRSF18 (GITR, CD357, NCBI Gene ID: 8784), TNFRSF19 (NCBI Gene ID: 55504), TNFRSF21 (CD358, DR6, NCBI Gene ID: 27242), and TNFRSF25 (DR3, NCBI Gene ID: 8718).

Example anti-TNFRSF4 (OX40) antibodies that can be co-administered include without limitation, MEDI6469, MEDI6383, MEDI0562 (tavolixizumab), MOXR0916, PF-04518600, RG-7888, GSK-3174998, INCAGN1949, BMS-986178, GBR-8383, ABBV-368, and those described in WO2016179517, WO2017096179, WO2017096182, WO2017096281, and WO2018089628.

Example anti-TNFRSF5 (CD40) antibodies that can be co-administered include without limitation RG7876, SEA-CD40, APX-005M and ABBV-428.

In some embodiments, the anti-TNFRSF7 (CD27) antibody varlilumab (CDX-1127) is co-administered.

Example anti-TNFRSF9 (4-1BB, CD137) antibodies that can be co-administered include without limitation urelumab, utomilumab (PF-05082566), AGEN2373 and ADG-106.

Example anti-TNFRSF18 (GITR) antibodies that can be co-administered include without limitation, MEDI1873, FPA-154, INCAGN-1876, TRX-518, BMS-986156, MK-1248, GWN-323, and those described in WO2017096179, WO2017096276, WO2017096189, and WO2018089628. In some embodiments, an antibody, or fragment thereof, co-targeting TNFRSF4 (OX40) and TNFRSF18 (GITR) is co-administered. Such antibodies are described, e.g., in WO2017096179 and WO2018089628.

### Bi-and Tri-Specific Natural Killer (NK)-Cell Engagers

In various embodiments, the agents as described herein, are combined with a bi-specific NK-cell engager (BiKE) or a tri-specific NK-cell engager (TriKE) (e.g., not having an Fc) or bi-specific antibody (e.g., having an Fc) against an NK cell activating receptor, e.g., CD16A, C-type lectin receptors (CD94/NKG2C, NKG2D, NKG2E/H and NKG2F), natural cytotoxicity receptors (NKp30, NKp44 and NKp46), killer cell C-type lectin-like receptor (NKp65, NKp80), Fc receptor FcγR (which mediates antibody-dependent cell cytotoxicity), SLAM family receptors (e.g., 2B4, SLAM6 and SLAM7), killer cell immunoglobulin-like receptors (KIR) (KIR-2DS and KIR-3DS), DNAM-1 and CD137 (41BB). As appropriate, the anti-CD16 binding bi-specific molecules may or may not have an Fc. Illustrative bi-specific NK-cell engagers that can be co-administered target CD16 and one or more HIV-associated antigens as described herein. BiKEs and TriKEs are described, e.g., in Felices, et al., Methods Mol Biol. (2016) 1441:333-346; Fang, et al., Semin Immunol. (2017) 31:37-54. Examples of a trispecific NK cell engager (TRiKE) include OXS-3550, and CD16-IL-15-B7H3 TriKe.

### Indoleamine-pyrrole-2,3-dioxygenase (IDO1) inhibitors

In various embodiments, the agents as described herein, are combined with an inhibitor of indoleamine 2,3-dioxygenase 1 (IDO1; NCBI Gene ID: 3620). Examples of IDO1 inhibitors include without limitation, BLV-0801, epacadostat, F-001287, GBV-1012, GBV-1028, GDC-0919, indoximod, NKTR-218, NLG-919-based vaccine, PF-06840003, pyranonaphthoquinone derivatives (SN-35837), resminostat, SBLK-200802, BMS-986205, and shIDO-ST, EOS-200271, KHK-2455, LY-3381916.

### Toll-Like Receptor (TLR) Agonists

In various embodiments, the agents as described herein, are combined with an agonist of a toll-like receptor (TLR), e.g., an agonist of TLR1 (NCBI Gene ID: 7096), TLR2 (NCBI Gene ID: 7097), TLR3 (NCBI Gene ID: 7098), TLR4 (NCBI Gene ID: 7099), TLR5 (NCBI Gene ID: 7100), TLR6 (NCBI Gene ID: 10333), TLR7 (NCBI Gene ID: 51284), TLR8 (NCBI Gene ID: 51311), TLR9 (NCBI Gene ID: 54106), and/or TLR10 (NCBI Gene ID: 81793). Example TLR7 agonists that can be co-administered include without limitation AL-034, DSP-0509, GS-9620 (vesatolimod), vesatolimod analogs, LHC-165, TMX-101 (imiquimod), GSK-2245035, resiquimod, DSR-6434, DSP-3025, IMO-4200, MCT-465, MEDI-9197, 3M-051, SB-9922, 3M-052, Limtop, TMX-30X, TMX-202, RG-7863, RG-7854, RG-7795, and the compounds disclosed in US20100143301 (Gilead Sciences), US20110098248 (Gilead Sciences), and US20090047249 (Gilead Sciences), US20140045849 (Janssen), US20140073642 (Janssen), WO2014/056953 (Janssen), WO2014/076221 (Janssen), WO2014/128189 (Janssen), US20140350031 (Janssen), WO2014/023813 (Janssen), US20080234251 (Array Biopharma), US20080306050 (Array Biopharma), US20100029585 (Ventirx Pharma), US20110092485 (Ventirx Pharma), US20110118235 (Ventirx Pharma), US20120082658 (Ventirx Pharma), US20120219615 (Ventirx Pharma), US20140066432 (Ventirx Pharma), US20140088085 (Ventirx Pharma), US20140275167 (Novira Therapeutics), and US20130251673 (Novira Therapeutics). An TLR7/TLR8 agonist that can be co-administered is NKTR-262, telratolimod and BDB-001. Example TLR8 agonists that can be co-administered include without limitation E-6887, IMO-4200, IMO-8400, IMO-9200, MCT-465, MEDI-9197, motolimod, resiquimod, GS-9688, VTX-1463, VTX-763, 3M-051, 3M-052, and the compounds disclosed in US20140045849 (Janssen), US20140073642 (Janssen), WO2014/056953 (Janssen), WO2014/076221 (Janssen), WO2014/128189 (Janssen), US20140350031 (Janssen), WO2014/023813 (Janssen), US20080234251 (Array Biopharma), US20080306050 (Array Biopharma), US20100029585 (Ventirx Pharma), US20110092485 (Ventirx Pharma), US20110118235 (Ventirx Pharma), US20120082658 (Ventirx Pharma), US20120219615 (Ventirx Pharma), US20140066432 (Ventirx Pharma), US20140088085 (Ventirx Pharma), US20140275167 (Novira Therapeutics), and US20130251673 (Novira Therapeutics). Example TLR9 agonists that can be co-administered include without limitation AST-008, cobitolimod, CMP-001, IMO-2055, IMO-2125, litenimod, MGN-1601, BB-001, BB-006, IMO-3100, IMO-8400, IR-103, IMO-9200, agatolimod, DIMS-9054, DV-1079, DV-1179, AZD-1419, lefitolimod (MGN-1703), CYT-003, CYT-003-QbG10, tilsotolimod and PUL-042. Examples of TLR3 agonist include rintatolimod, poly-ICLC, RIBOXXON^{®}, Apoxxim, RIBOXXIM^{®}, IPH-33, MCT-465, MCT-475, and ND-1.1. Examples of TLR4 agonist include G-100, and GSK-1795091.

### CDK Inhibitors or Antagonists

In some embodiments, the agents described herein are combined with an inhibitor or antagonist of CDK. In some embodiments, the CDK inhibitor or antagonist is selected from the group consisting of VS2-370.

### STING agonists, RIG-I and NOD2 modulators

In some embodiments, the agents described herein are combined with a stimulator of interferon genes (STING). In some embodiments, the STING receptor agonist or activator is selected from the group consisting of ADU-S100 (MIW-815), SB-11285, MK-1454, SR-8291, AdVCA0848, GSK-532, SYN-STING, MSA-1, SR-8291, 5,6-dimethylxanthenone-4-acetic acid (DMXAA), cyclic-GAMP (cGAMP) and cyclic-di-AMP. In some embodiments, the agents described herein are combined with a RIG-I modulator such as RGT-100, or NOD2 modulator, such as SB-9200, and IR-103.

### LAG-3 and TIM-3 inhibitors

In certain embodiments, the agents as described herein are combined with an anti-TIM-3 antibody, such as TSR-022, LY-3321367, MBG-453, INCAGN-2390.

In certain embodiments, the antibodies or antigen-binding fragments described herein are combined with an anti LAG-3 (Lymphocyte-activation) antibody, such as relatlimab (ONO-4482), LAG-525, MK-4280, REGN-3767, INCAGN2385.

### Interleukin agonists

In certain embodiments, the agents described herein are combined with an interleukin agonist, such as IL-2, IL-7, IL-15, IL-10, IL-12 agonists; examples of IL-2 agonists such as proleukin (aldesleukin, IL-2); pegylated IL-2 (eg NKTR-214); modified variants of IL-2 (eg THOR-707), bempegaldesleukin, AIC-284, ALKS-4230, CUI-101, Neo-2/15; examples of IL-15 agonists, such as ALT-803, NKTR-255, and hetIL-15, interleukin-15/Fc fusion protein, AM-0015, NIZ-985, SO-C101, IL-15 Synthorin (pegylated Il-15), P-22339, and a IL-15 -PD-1 fusion protein N-809; examples of IL-7 include CYT-107.

Examples of additional immune-based therapies that can be combined with an agent of this disclosure include interferon alfa; interferon alfa-2b; interferon alfa-n3; pegylated interferon alfa; interferon gamma; Flt3 agonists such as CDX-301 and GS-3583; gepon; normferon, peginterferon alfa-2a, peginterferon alfa-2b, RPI-MN.

### Phosphatidylinositol 3-kinase (PI3K) Inhibitors

In certain embodiments, the agents described herein are combined with a PI3K inhibitor. Examples of PI3K inhibitors that can be combined with an agent of this disclosure include idelalisib, alpelisib, buparlisib, CAI orotate, copanlisib, duvelisib, gedatolisib, neratinib, panulisib, perifosine, pictilisib, pilaralisib, puquitinib mesylate, rigosertib, rigosertib sodium, sonolisib, taselisib, AMG-319, AZD-8186, BAY-1082439, CLR-1401, CLR-457, CUDC-907, DS-7423, EN-3342, GSK-2126458, GSK-2269577, GSK-2636771, INCB-040093, LY-3023414, MLN-1117, PQR-309, RG-7666, RP-6530, RV-1729, SAR-245409, SAR-260301, SF-1126, TGR-1202, UCB-5857, VS-5584, XL-765, and ZSTK-474.

### alpha-4/beta-7 antagonists

In certain embodiments, the agents described herein are combined with an alpha-4/beta-7 antagonist. Examples of Integrin alpha-4/beta-7 antagonists that can be combined with an agent of this disclosure include PTG-100, TRK-170, abrilumab, etrolizumab, carotegrast methyl, and vedolizumab.

### HIV targeting Antibodies

Examples of HIV antibodies, bispecific antibodies, and "antibody-like" therapeutic proteins that can be combined with an agent of this disclosure include DARTs^{®}, DUOBODIES^{®}, BITES^{®}, XmAbs^{®}, TandAbs^{®}, Fab derivatives, bNAbs (broadly neutralizing HIV-1 antibodies), TMB-360, and those targeting HIV gp120 or gp41, antibody-Recruiting Molecules targeting HIV, anti-CD63 monoclonal antibodies, anti-GB virus C antibodies, anti-GP120/CD4, gp120 bispecific monoclonal antibody, CCR5 bispecific antibodies, anti-Nef single domain antibodies, anti-Rev antibody, camelid derived anti-CD18 antibodies, camelid-derived anti-ICAM-1 antibodies, DCVax-001, gp140 targeted antibodies, gp41-based HIV therapeutic antibodies, human recombinant mAbs (PGT-121), PGT121.414.LS, ibalizumab, Immuglo, MB-66, VRC-HIVMAB091-00-AB.

Various bNAbs are known in the art and may be used in this invention. Examples include, but are not limited to, those described in U.S. Patent No. 8673307, 9,493,549, 9,783,594, WO2014/063059, WO2012/158948, WO2015/117008, and PCT/US2015/41272, and WO2017/096221, including antibodies 12A12, 12A21, NIH45-46, bANC131, 8ANC134, IB2530, INC9, 8ANC195. 8ANC196, 10-259, 10-303, 10-410, 10- 847, 10-996, 10-1074, 10-1121, 10-1130, 10-1146, 10-1341, 10-1369, and 10-1074GM. Additional examples include those described in Klein et al., Nature, 492(7427): 118-22 (2012), Horwitz et al., Proc Natl Acad Sci U S A, 110(41): 16538-43 (2013), Scheid, et al., Science, 333: 1633-1637 (2011), Scheid, et al., Nature, 458:636-640 (2009), Eroshkin et al, Nucleic Acids Res., 42 (Database issue):Dl 133-9 (2014), Mascola et al., Immunol Rev., 254(l):225-44 (2013), such as 2F5, 4E10, M66.6, CAP206-CH12, 10E81 (all of which bind the MPER of gp41); PG9, PG16, CH01-04 (all of which bind V1V2-glycan), 2G12 (which binds to outer domain glycan); b12, HJ16, CH103-106, VRC01-03, VRC-PG04, 04b, VRC-CH30-34, 3BNC62, 3BNC89, 3BNC91, 3BNC95, 3BNC104, 3BNC176, and 8ANC131 (all of which bind to the CD4 binding site).

Additional broadly neutralizing antibodies which can be used as a second therapeutic agent in a combination therapy are described, e.g., in U.S. Patent Nos. 8,673,307; 9,493,549; 9,783,594; and WO 2012/154312; WO2012/158948; WO 2013/086533; WO 2013/142324; WO2014/063059; WO 2014/089152, WO 2015/048462; WO 2015/103549; WO 2015/117008; WO2016/014484; WO 2016/154003; WO 2016/196975; WO 2016/149710; WO2017/096221; WO 2017/133639; WO 2017/133640. Additional examples include those described in Sajadi, et al., Cell. (2018) 173(7):1783-1795; Sajadi, et al., J Infect Dis. (2016) 213(1):156-64; Klein et al., Nature, 492(7427): 118-22 (2012), Horwitz et al., Proc Natl Acad Sci U S A, 110(41): 16538-43 (2013), Scheid, et al., Science, 333: 1633-1637 (2011), Scheid, et al., Nature, 458:636-640 (2009), Eroshkin et al, Nucleic Acids Res., 42 (Database issue):Dl 133-9 (2014), Mascola et al., Immunol Rev., 254(l):225-44 (2013), such as 2F5, 4E10, M66.6, CAP206-CH12, 10E8, 10E8v4, 10E8-5R-100cF, DH511.11P, 7b2, and LN01 (all of which bind the MPER of gp41).

Examples of additional antibodies include bavituximab, UB-421, BF520.1, CH01, CH59, C2F5, C4E10, C2F5+C2G12+C4E10, 3BNC117, 3BNC117-LS, 3BNC60, DH270.1, DH270.6, D1D2, 10-1074-LS, Cl3hmAb, GS-9722 (elipovimab), DH411-2, BG18, GS-9721, PGT145, PGT121, PGT-121.60, PGT-121.66, PGT122, PGT-123, PGT-124, PGT-125, PGT-126, PGT-151, PGT-130, PGT-133, PGT-134, PGT-135, PGT-128, PGT-136, PGT-137, PGT-138, PGT-139, MDX010 (ipilimumab), DH511, DH511-2, N6, N6LS, N49P6, N49P7, N49P7.1, N49P9, N49P11, N60P1.1, N60P25.1, N60P2.1, N60P31.1, N60P22, NIH 45-46, PGC14, PGG14, PGT-142, PGT-143, PGT-144, PGDM1400, PGDM12, PGDM21, PCDN-33A, 2Dm2m, 4Dm2m, 6Dm2m, PGDM1400, MDX010 (ipilimumab), VRC01, VRC-01-LS, A32, 7B2, 10E8, VRC-07-523, VRC07-523LS, VRC24, VRC41.01, 10E8VLS, 3810109, 10E8v4, IMC-HIV, iMabm36, eCD4-Ig, IOMA, CAP256-VRC26.25, DRVIA7,VRC-HIVMAB080-00-AB, VRC-HIVMAB060-00-AB, P2G12, VRC07, 354BG8, 354BG18, 354BG42, 354BG33, 354BG129, 354BG188, 354BG411, 354BG426, VRC29.03, CAP256, CAP256-VRC26.08, CAP256-VRC26.09, CAP256-VRC26.25, PCT64-24E and VRC38.01, PGT-151, CAP248-2B, 35O22, ACS202, VRC34 and VRC34.01, 10E8, 10E8v4, 10E8-5R-100cF, 4E10, DH511.11P, 2F5, 7b2, and LN01.

Example of HIV bispecific and trispecific antibodies include MGD014, B12BiTe, BiIA-SG, TMB-bispecific, SAR-441236, VRC-01/PGDM-1400/10E8v4, 10E8.4/iMab, 10E8v4/PGT121-VRC01.

### Pharmacokinetic Enhancers

In certain embodiments, the agents described herein are combined with a pharmacokinetic enhancer. Examples of pharmacokinetic enhancers that can be combined with an agent of this disclosure include cobicistat and ritonavir.

### Additional Therapeutic Agents

Examples of additional therapeutic agents that can be combined with an agent of this disclosure include the compounds disclosed in WO 2004/096286 (Gilead Sciences), WO 2006/015261 (Gilead Sciences), WO 2006/110157 (Gilead Sciences), WO 2012/003497 (Gilead Sciences), WO 2012/003498 (Gilead Sciences), WO 2012/145728 (Gilead Sciences), WO 2013/006738 (Gilead Sciences), WO 2013/159064 (Gilead Sciences), WO 2014/100323 (Gilead Sciences), US 2013/0165489 (University of Pennsylvania), US 2014/0221378 (Japan Tobacco), US 2014/0221380 (Japan Tobacco), WO 2009/062285 (Boehringer Ingelheim), WO 2010/130034 (Boehringer Ingelheim), WO 2013/006792 (Pharma Resources), US 20140221356 (Gilead Sciences), US 20100143301 (Gilead Sciences) and WO 2013/091096 (Boehringer Ingelheim).

### HIV Vaccines

In certain embodiments, the agents described herein are combined with an HIV vaccine. Examples of HIV vaccines that can be combined with an agent of this disclosure include peptide vaccines, recombinant subunit protein vaccines, live vector vaccines, DNA vaccines, HIV MAG DNA vaccine, CD4-derived peptide vaccines, vaccine combinations, adenoviral vector vaccines (an adenoviral vector such as Ad5, Ad26 or Ad35), simian adenovirus (chimpanzee, gorilla, rhesus, i.e., rhAd), adeno-associated virus vector vaccines, Chimpanzee adenoviral vaccines (e.g., ChAdOX1, ChAd68, ChAd3, ChAd63, ChAd83, ChAd155, ChAd157, Pan5, Pan6, Pan7, Pan9), Coxsackieviruses based vaccines, enteric virus based vaccines, Gorilla adenovirus vaccines, lentiviral vector based vaccine, arenavirus vaccines (such as LCMV, Pichinde), bi-segmented or tri-segmented arenavirus based vaccine, trimer-based HIV-1 vaccine, measles virus based vaccine, flavivirus vector based vaccines, tobacco mosaic virus vector based vaccine, Varicella-zoster virus based vaccine, Human parainfluenza virus 3 (PIV3) based vaccines, poxvirus based vaccine (modified vaccinia virus Ankara (MVA), orthopoxvirus-derived NYVAC, and avipoxvirus-derived ALVAC (canarypox virus) strains); fowlpox virus based vaccine, rhabdovirus-based vaccines, such as VSV and marabavirus; recombinant human CMV (rhCMV) based vaccine, alphavirus-based vaccines, such as semliki forest virus, venezuelan equine encephalitis virus and sindbis virus; (see Lauer, Clinical and Vaccine Immunology, 2017, DOI: 10.1128/CVI.00298-16); LNP formulated mRNA based therapeutic vaccines; LNP-formulated self-replicating RNA/self-amplifying RNA vaccines.

Examples of vaccines include: anti-CD40.Env-gp140 vaccine, Ad4-EnvC150, BG505 SOSIP.664 gp140 adjuvanted vaccine, BG505 SOSIP.GT1.1 gp140 adjuvanted vaccine, Chimigen HIV vaccine, ConM SOSIP.v7 gp140, rgp120 (AIDSVAX), ALVAC HIV (vCP1521)/AIDSVAX B/E (gp120) (RV144), monomeric gp120 HIV-1 subtype C vaccine, MPER-656 liposome subunit vaccine, Remune, ITV-1, Contre Vir, Ad5-ENVA-48, DCVax-001 (CDX-2401), Vacc-4x, Vacc-C5, VAC-3S, multiclade DNA recombinant adenovirus-5 (rAd5), rAd5 gag-pol env A/B/C vaccine, Pennvax-G, Pennvax-GP, Pennvax-G/MVA-CMDR, HIV-TriMix-mRNA vaccine, HIV-LAMP-vax, Ad35, Ad35-GRIN, NAcGM3/VSSP ISA-51, poly-ICLC adjuvanted vaccines, TatImmune, GTU-multiHIV (FIT-06), ChAdV63.HIVconsv, gp140[delta]V2.TV1+MF-59, rVSVIN HIV-1 gag vaccine, SeV-EnvF, SeV-Gag vaccine, AT-20, DNK-4, ad35-Grin/ENV, TBC-M4, HIVAX, HIVAX-2, N123-VRC-34.01 inducing epitope-based HIV vaccine, NYVAC-HIV-PT1, NYVAC-HIV-PT4, DNA-HIV-PT123, rAAV1-PG9DP, GOVX-B11, GOVX-B21, GOVX-C55, TVI-HIV-1, Ad-4 (Ad4-env Clade C+Ad4-mGag), Paxvax, EN41-UGR7C, EN41-FPA2, ENOB-HV-11, PreVaxTat, AE-H, MYM-V101, CombiHIVvac, ADVAX, MYM-V201, MVA-CMDR, MagaVax, DNA-Ad5 gag/pol/nef/nev (HVTN505), MVATG-17401, ETV-01, CDX-1401, DNA and Sev vectors vaccine expressing SCaVII, rcAD26.MOS1.HIV-Env, Ad26.Mod.HIV vaccine, Ad26.Mod.HIV + MVA mosaic vaccine + gp140, AGS-004, AVX-101, AVX-201, PEP-6409, SAV-001, ThV-01, TL-01, TUTI-16, VGX-3300, VIR-1111, IHV-001, and virus-like particle vaccines such as pseudovirion vaccine, CombiVICHvac, LFn-p24 B/C fusion vaccine, GTU-based DNA vaccine, HIV gag/pol/nef/env DNA vaccine, anti-TAT HIV vaccine, conjugate polypeptides vaccine, dendritic-cell vaccines (such as DermaVir), gag-based DNA vaccine, GI-2010, gp41 HIV-1 vaccine, HIV vaccine (PIKA adjuvant), i-key/MHC class II epitope hybrid peptide vaccines, ITV-2, ITV-3, ITV-4, LIPO-5, multiclade Env vaccine, MVA vaccine, Pennvax-GP, pp71-deficient HCMV vector HIV gag vaccine, rgp160 HIV vaccine, RNActive HIV vaccine, SCB-703, Tat Oyi vaccine, TBC-M4, UBI HIV gp120, Vacc-4x + romidepsin, variant gp120 polypeptide vaccine, rAd5 gag-pol env A/B/C vaccine, DNA.HTI and MVA.HTI, VRC-HIVDNA016-00-VP + VRC-HIVADV014-00-VP, INO-6145, JNJ-9220, gp145 C.6980; eOD-GT8 60mer based vaccine, PD-201401, env (A, B, C, A/E)/gag (C) DNA Vaccine, gp120 (A,B,C,A/E) protein vaccine, PDPHV-201401, Ad4-EnvCN54, EnvSeq-1 Envs HIV-1 vaccine (GLA-SE adjuvanted), HIV p24gag prime-boost plasmid DNA vaccine, HIV-1 iglb12 neutralizing VRC-01 antibody-stimulating anti-CD4 vaccine, arenavirus vector-based vaccines (Vaxwave, TheraT), MVA-BN HIV-1 vaccine regimen, UBI HIV gp120, mRNA based prophylactic vaccines, VPI-211, and TBL-1203HI.

### Birth control (contraceptive) combination therapy

In certain embodiments, the agents described herein are combined with a birth control or contraceptive regimen. Therapeutic agents used for birth control (contraceptive) that can be combined with an agent of this disclosure include cyproterone acetate, desogestrel, dienogest, drospirenone, estradiol valerate, ethinyl Estradiol, ethynodiol, etonogestrel, levomefolate, levonorgestrel, lynestrenol, medroxyprogesterone acetate, mestranol, mifepristone, misoprostol, nomegestrol acetate, norelgestromin, norethindrone, noretynodrel, norgestimate, ormeloxifene, segestersone acetate, ulipristal acetate, and any combinations thereof.

In some embodiments, an agent disclosed herein, or a pharmaceutical composition thereof, is combined with a first additional therapeutic agent (a contraceptive) selected from the group consisting of cyproterone acetate, desogestrel, dienogest, drospirenone, estradiol valerate, ethinyl Estradiol, ethynodiol, etonogestrel, levomefolate, levonorgestrel, lynestrenol, medroxyprogesterone acetate, mestranol, mifepristone, misoprostol, nomegestrol acetate, norelgestromin, norethindrone, noretynodrel, norgestimate, ormeloxifene, segestersone acetate, ulipristal acetate, and any combinations thereof.

### Gene Therapy and Cell Therapy

In certain embodiments, the agents described herein are combined with a gene or cell therapy regimen. Gene therapy and cell therapy include without limitation the genetic modification to silence a gene; genetic approaches to directly kill the infected cells; the infusion of immune cells designed to replace most of the patient's own immune system to enhance the immune response to infected cells, or activate the patient's own immune system to kill infected cells, or find and kill the infected cells; genetic approaches to modify cellular activity to further alter endogenous immune responsiveness against the infection. Examples of cell therapy include LB-1903,ENOB-HV-01, GOVX-B01, and SupT1 cell based therapy. Examples of dendritic cell therapy include AGS-004. CCR5 gene editing agents include SB-728T. CCR5 gene inhibitors include Cal-1. In some embodiments, C34-CCR5/C34-CXCR4 expressing CD4-positive T-cells are co-administered with one or more multi-specific antigen binding molecules. In some embodiments, the agents described herein are co-administered with AGT-103-transduced autologous T-cell therapy or AAV-eCD4-Ig gene therapy.

### Gene Editors

In certain embodiments, the agents described herein are combined with a gene editor, e.g., an HIV targeted gene editor. In various embodiments, the genome editing system can be selected from the group consisting of: a CRISPR/Cas9 complex, a zinc finger nuclease complex, a TALEN complex, a homing endonucleases complex, and a meganuclease complex. An illustrative HIV targeting CRISPR/Cas9 system includes without limitation EBT-101.

### CAR-T-cell therapy

In some embodiments, the agents described herein can be co-administered with a population of immune effector cells engineered to express a chimeric antigen receptor (CAR), wherein the CAR comprises an HIV antigen binding domain. The HIV antigen include an HIV envelope protein or a portion thereof, gp120 or a portion thereof, a CD4 binding site on gp120, the CD4-induced binding site on gp120, N glycan on gp120, the V2 of gp120, the membrane proximal region on gp41. The immune effector cell is a T-cell or an NK cell. In some embodiments, the T-cell is a CD4+ T-cell, a CD8+ T-cell, or a combination thereof. Cells can be autologous or allogeneic. Examples of HIV CAR-T include convertible CAR-T, VC-CAR-T, CMV-N6-CART, anti-CD4 CART-cell therapy, CD4 CAR+C34-CXCR4+CCR5 ZFN T-cells, autologous hematopoietic stem cells genetically engineered to express a CD4 CAR and the C46 peptide.

### TCR-T-cell therapy

In certain embodiments, the agents described herein are combined with a population of TCR-T-cells. TCR-T-cells are engineered to target HIV derived peptides present on the surface of virus-infected cells, for example ImmTAV.

### B-cell therapy

In certain embodiments, the antibodies or antigen-binding fragments described herein are combined with a population of B cells genetically modified to express broadly neutralizing antibodies, such as 3BNC117 (Hartweger et al, J. Exp. Med. 2019, 1301, Moffett et al., Sci. Immunol. 4, eaax0644 (2019) 17 May 2019).

It will be appreciated by one of skill in the art that the additional therapeutic agents listed above may be included in more than one of the classes listed above. The particular classes are not intended to limit the functionality of those compounds listed in those classes.

### V. Kits

The pharmaceutical compositions provided herein can be formed from components provided a kit. In one aspect, provided herein are a kit comprising the components of a pharmaceutical composition described herein in a suitable packaging. In some embodiments, the kit further comprises instructions for making and using the pharmaceutical compositions.

Accordingly, provided herein is a kit comprising:
i) a first container comprising bictegravir drug substance in solid form; and
ii) a second container comprising a liquid vehicle.

In some embodiments, provided herein is a kit comprising:
i) a first container comprising bictegravir free acid; and
ii) a second container comprising an aqueous suspending vehicle.

In some embodiments, the aqueous suspending vehicle has a pH of from about 7.0 to about 7.8. In some embodiments, the aqueous suspending vehicle has a pH of about 7.4.

In some embodiments of any of the kits disclosed herein, the bictegravir free acid is crystalline.

In some embodiments, the crystalline bictegravir free acid is of form III, wherein form III is characterized by an x-ray powder diffraction (XRPD) pattern having peaks at about 9.6°, 14.0°, and 18.5° 2θ ± 0.2° 2θ. In some embodiments, crystalline bictegravir free acid is of form III wherein the x-ray powder diffraction (XRPD) pattern has further peaks at about 20.0° and 22.5° 2θ ± 0.2° 2θ.

In some embodiments of any of the kits provided herein, the crystalline bictegravir free acid has a particle size distribution with a d₉₀ value from about 1 µm to about 90 µm. In some embodiments of any of the kits provided herein, the crystalline bictegravir free acid has a particle size distribution with a d₉₀ value from about 5 µm to about 30 µm. In some embodiments of any of the kits provided herein, the crystalline bictegravir free acid has a particle size distribution with a d₉₀ value of ≤ about 30 µm. In some embodiments of any of the kits provided herein, the crystalline bictegravir free acid has a particle size distribution with a d₉₀ value of ≤ about 20 µm. In some embodiments of any of the kits provided herein, the crystalline bictegravir free acid has a particle size distribution with a d₉₀ value of ≤ about 15 µm. In some embodiments of any of the kits provided herein, the crystalline bictegravir free acid has a particle size distribution with a d₉₀ value of ≤ about 10 µm. In some embodiments of any of the kits provided herein, the crystalline bictegravir free acid has a particle size distribution with a d₉₀ value from about 20 µm to about 25 µm. In some embodiments of any of the kits provided herein, the crystalline bictegravir free acid has a particle size distribution with a d₉₀ value from about 15 µm to about 20 µm. In some embodiments of any of the kits provided herein, the crystalline bictegravir free acid has a particle size distribution with a d₉₀ value from about 10 µm to about 15 µm. In some embodiments of any of the kits provided herein, the crystalline bictegravir free acid has a particle size distribution with a d₉₀ value from about 1 µm to about 10 µm. In some embodiments of any of the kits provided herein, the crystalline bictegravir free acid has a particle size distribution with a d₉₀ value from about 1 µm to about 5 µm.

The invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results.

### EXAMPLES

Example compositions which fall outside the scope of the claims are provided for reference only.

### Example 1. Long Acting Injectable Formulations Comprising Bictegravir

### (I) Formulations

Bictegravir for injection is a sterile, white to colored powder that is reconstituted prior to administration as either a suspension or solution formulation for SC or IM injection. Examples of these formulations and their associated pharmacokinetic profiles are provided below.

### A. Suspension Formulation

One suspending vehicle for BIC suspension for injection is a sterile, clear solution containing povidone, polysorbate 20, sodium chloride, sodium phosphate dibasic heptahydrate, sodium phosphate monobasic monohydrate, hydrochloric acid, sodium hydroxide, and sterile water for injection.

Another suspending vehicle for BIC suspension is a sterile, clear solution containing hydroxyl propyl methyl cellulose (HPMC), polysorbate 20, and 99% phosphate buffered saline.

Another suspending vehicle for BIC suspension is sterile Miglyol 812N.

Another suspending vehicle for BIC suspension is sterile sesame oil.

### B. Solution Formulation

The solution vehicle for BIC solution for injection is a sterile, clear solution containing N-methyl-2-pyrrolidone (NMP) prepared prior to use.

### (II). Pharmacokinetics

Pharmacokinetics (PK) following a single BIC SC injection of solution and suspension formulations was characterized in male Sprague-Dawley rat, beagle dog, and cynomolgus monkey. The BIC injectable solution and both suspension formulations display extended-release PK following SC administration in all examined species and support a potential monthly or less frequent dosing regimen. Pharmacokinetics modeling showed the extended duration of exposure was due to a flip-flop PK where BIC absorption rate from the SC compartment (kᵣₑₗₑₐₛₑ; **Table 1)** was much slower than the systemic elimination rate following IV administration (kₑ₁ = CL/Vₛₛ) in each nonclinical species tested (mean kₑ₁: ~0.0162 h⁻¹ in rat; ~0.302 h⁻¹ in dog; ~0.253 h⁻¹ in monkey).

**Table 1. BIC Plasma Pharmacokinetic Parameters Following a Single Subcutaneous (SC) or Intramuscular (IM) Administration**

| **Formulation** | | **Mean ± Standard Deviation (n=3)** | | | **Mean** | | |
|---|---|---|---|---|---|---|---|
| | **Species (Dose)^{d}** | **Cₘₐₓ (µM)** | **AUCₗₐₛₜ (µM×h)** | **F%^{b}** | **C_{28d} (µM)** | **Tₗₐₛₜ (day)** | **kᵣₑₗₑₐₛₑ (h⁻¹) ^{c} (range)** |
| NMP Solution SC (300 mg/mL) | Rat (30 mg/kg) | 41.4 ± 14.3 | 29100 ± 1550 | 78.7 ± 6.1 | 15.9 | 112 | 0.00144 |
| | Dog (10 mg/kg) | 0.837 ± 0.316 | 411 ± 149 | 62.2 ± 22.0 | 0.176 | 140 | 0.00111 |
| | Monkey^{a} (100 mg-fixed) | 3.98 ± 2.67 | 1935 | 62.3 | 0.594 | 252 | 0.00174 |
| HPMC Suspension SC (250 mg/mL) | Rat (30 mg/kg) | 36.9 ± 4.60 | 24500 ± 6600 | 88.4 ± 13.1 | 19.3 | 168 | 0.00172 |
| | Dog (100 mg-fixed) | 1.26 ± 0.799 | 504 ± 155 | 90.5 ± 22.7 | 0.186 | 70 | 0.00321 |
| | Monkey^{b} (100 mg-fixed) | 2.66 ± 0.875 | 1740 ± 267 | 58.2 ± 10.9 | 0.619 | 238 | 0.00141 |
| PVP Suspension SC (250 mg/mL) | Rat^{b} (30 mg/kg) | 44.2 ± 16.3 | 23700 ± 7600 | 87.1 ± 30.0 | 16.7 | 98 | 0.00309 |
| | Dog^{b} (100 mg-fixed) | 1.28 ± 0.534 | 532 ± 146 | 94.4 ± 26.6 | 0.373 | 84 | 0.00416 |
| | Monkey^{b} (100 mg-fixed) | 1.52 ± 0.80 | 1370 ± 329 | 54.0 ± 8.7 | 0.788 | 112 | 0.000594 |
| Miglyol Suspension SC (446 mg/mL) | Rat (53.5 mg/kg) | 73.5 ± 2.5 | 49800 + 3890 | 76.8 ± 5.9 | 28.9 | 224 | 0.00194 |
| | Monkey^{e} (446 mg-fixed) | 6.86 ± 2.62 | 9980 ± 4080 | 69.9 ± 26.4 | 3.97 | 196 | 0.000588 |
| Miglyol Suspension IM (450 mg/mL) | Monkey (450 mg-fixed) | 50.2 ± 16.9 | 8660 ± 668 | 59.8± 5.6 | 3.90 | 84 | 0.000951 |

| | | | | | | | |
|---|---|---|---|---|---|---|---|
| AUCₗₐₛₜ = area under time-concentration-time curve to the last quantifiable time-point; Cₘₐₓ = maximum concentration; C_{28d} = concentration on day 28; kᵣₑₗₑₐₛₑ = BIC release rate from the SC depot; Tₗₐₛₜ = last quantifiable time-point a AUCₗₐₛₜ represents the AUC to the last observed time-point b F% calculated following SC administration with AUC_{inf} for completed studies and AUCₗₐₛₜ for on-going studies c kᵣₑₗₑₐₛₑ mean value from modeling the mean PK data; kᵣₑₗₑₐₛₑ range from modeling terminal slope of the individual animal PK d nominal dose e AUCₗₐₛₜ represents the AUC to the last observed time-point | | | | | | | |

### A. Pharmacokinetics Following Subcutaneous Administration of NMP Solution Formulation

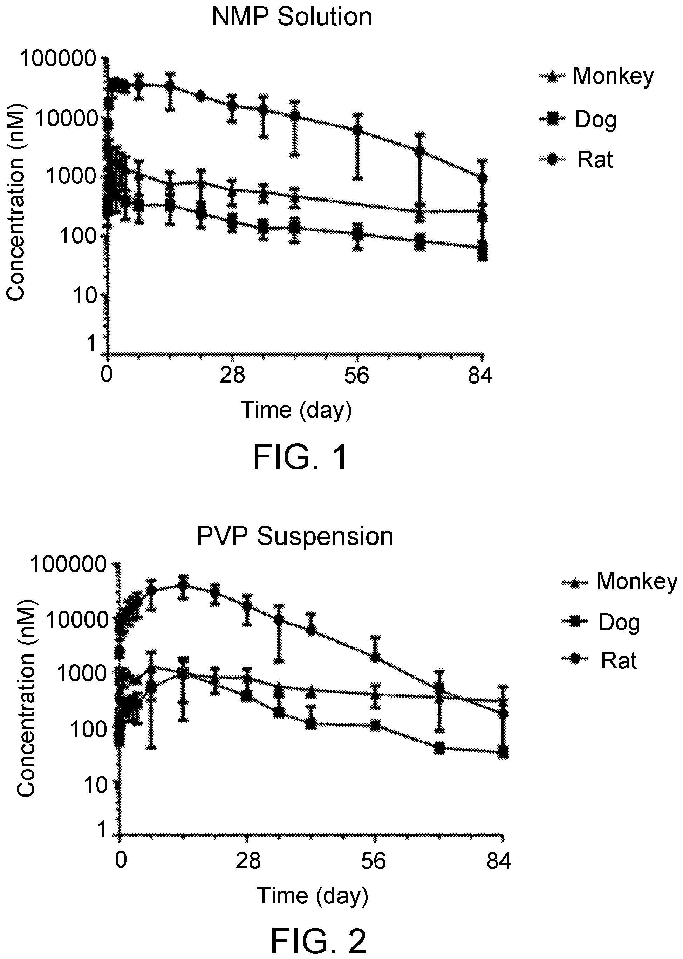

The solution was formulated as BIC free acid (300 mg/mL or ~250 mg/mL, e.g., 251 mg/mL) in 100% NMP. The PK profiles of BIC in plasma following a single SC administration of the NMP solution formulation at 300 mg/mL in rat (30 mg/kg), dog (10 mg/kg), and cynomolgus monkey (100 mg fixed; ~29.2 mg/kg) are shown in **FIG. 1****;** the PK parameters are presented in **Table 1.** The BIC plasma concentrations increased, reaching mean maximal concentration at 2, 1, and 0.125 days postdose in rat, dog, and monkey, respectively. The PK profiles showed no unintended (burst) release. After reaching maximal concentrations in plasma, a sustained slow elimination phase was observed with concentrations above the detection limit for at least 90 days postdose in all 3 species. The comparison of total exposure (AUCₗₐₛₜ) across species was consistent with the systemic clearance (CL) of BIC and body weight-corrected doses. The PK study in monkey is ongoing with extended sample collection; mean BIC plasma concentrations (> 200 nM) were well above the limit of quantitation (~2 nM) in monkey on Day 112. The mean bioavailability (F%; estimated based on AUCₗₐₛₜ; **Table 1)** was 79%, 62%, and 62% in rat, dog and monkey, respectively.

### B. Pharmacokinetics Following SC Administration of PVP Suspension Formulation

The suspension formulation consisted of BIC free acid (form III; particle size d₉₀ = 6.9 to 17 µm; 250 mg/mL) in 2.0% povidone (PVP) K12, 0.5% polysorbate 20, 0.76% sodium chloride in 10 mM sodium phosphate buffer (pH 7.4) and is henceforth referred to as PVP suspension. The PK studies in rat (30 mg/kg), dog (100 mg fixed; ~10 mg/kg) and cynomolgus monkey (100 mg fixed; ~32 mg/kg). The PK profiles of BIC in plasma following the PVP suspension formulation administration are shown in **FIG. 2****;** the PK parameters are presented in **Table 1.** The BIC plasma concentrations increased slowly (compared to the NMP solution formulation) reaching mean maximal concentration at 14, 16.3, and 2.5 days postdose in rat, dog, and monkey, respectively. The PK profiles showed no unintended (burst) release. After reaching maximal concentrations in plasma a sustained slow elimination phase was observed with concentrations above the detection limit for at least 70 days postdose in all 3 nonclinical species. The mean bioavailability (F%; **Table 1)** was 87%, 94%, and 54% in rat, dog, and monkey, respectively.

### C. Pharmacokinetics Following SC Administration of HPMC Suspension Formulation

The suspension was formulated as BIC free acid (250 mg/mL; form III; particle size d₉₀ = 12.9 µM) in 0.5% hydroxyl propyl methyl cellulose (HPMC); 0.5% polysorbate 20; 99% phosphate buffered saline (pH 7.4) and is referred to in this Example (Example 1) as HPMC suspension.

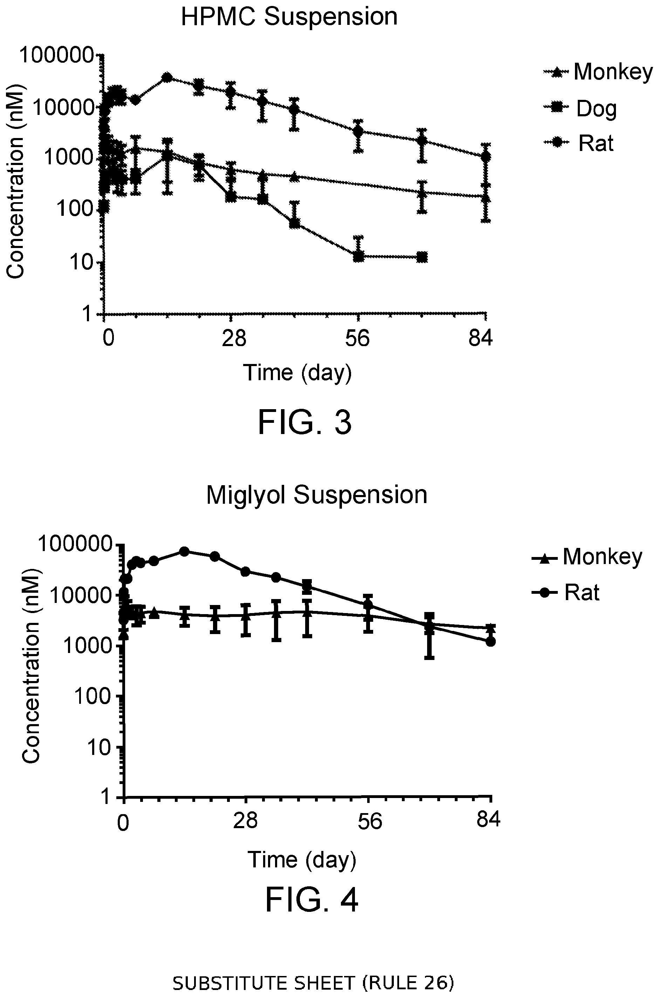

PK profiles of BIC in plasma following a single SC administration of the HPMC suspension formulation in rat (30 mg/kg), dog (100 mg fixed; ~11.3 mg/kg) and cynomolgus monkey (100 mg fixed; ~38.1 mg/kg) are shown in **FIG. 3****;** the PK parameters are presented in 1. BIC plasma concentrations increased slowly (compared to the NMP solution formulation) reaching mean maximal concentration at 14, 14, and 0.33 days post dose in rat, dog, and monkey, respectively. The PK profiles showed no unintended (burst) release. After reaching maximal concentrations in plasma a sustained slow elimination phase was observed with concentrations above the detection limit for at least 70 days post dose in all three nonclinical species. The mean bioavailability (F%, **Table 1)** was 88, 91, and 58% in rat, dog and monkey, respectively.

The PVP suspension had improved viscosity and a reduced applied force to the syringe plunger during administration compared to the HPMC suspension was chosen for further development along with the NMP solution.

### D. Pharmacokinetics Following SC Administration of Miglyol Suspension Formulation

The suspension was formulated as BIC free acid (form III; particle size d₉₀ = 16 µm; 446 mg/mL) in 100% Miglyol^{®} 812N and is henceforth referred to as Miglyol suspension.

PK profiles of BIC in plasma following the Miglyol suspension formulation administration is shown in **FIG. 4****;** the PK parameters are presented in Table 1. BIC plasma concentrations increased slowly (compared to the NMP solution formulation) reaching mean maximal concentration at 15.8 and 0.26 days post dose in rat and monkey, respectively. The PK profiles showed no unintended (burst) release. After reaching maximal concentrations in plasma a sustained slow elimination phase was observed with concentrations above the detection limit for at least 196 days post dose in both nonclinical species. The mean bioavailability (F%; **Table 1)** was 77, and 70% in rat and monkey, respectively.

### D. Methods

### a) Male Sprague Dawley Rat:

This study investigated the pharmacokinetics of three formulations of bictegravir (BIC) by subcutaneous administration to determine the potential to deliver a long acting (LA) pharmacokinetic (PK) profile. BIC was administered by a 30-min intravenous (IV) infusion at 0.5 mg/kg and by subcutaneous (SC) injection of three LA formulations (e.g., NMP solution, HPMC suspension, and PVP suspension) at 30 mg/kg to male Sprague Dawley rats. The concentrations of BIC in plasma were determined with a liquid chromatography-tandem mass spectrometry (LC-MS/MS) method.

At dosing, the animals (n=3 or 4) weighed between 0.25 to 0.32 kg. The animals were fasted overnight and food was returned at approximately 4 hours post dose.

The intravenous dose was administered as a 30-minute infusion via a catheter implanted animals. After dose administration, the dose apparatus (i.e., the indwelling catheter) was flushed with approximately 1 mL of saline. The subcutaneous injection was administered via injection into the dorsal surface between the shoulders and point of the hip. Individual dose was calculated based on body weights recorded on the day of dose administration.

Serial blood samples (approximately 0.3 mL each) were collected from the jugular vein via syringe and needle at specified time points from each animal into single tubes containing K₂EDTA as the anticoagulant and were immediately placed on wet ice prior to centrifugation for plasma. For the IV group, the blood samples were collected at pre-dose and at 0.25, 0.48, 0.58, 0.75, 1.5, 3, 6, 8, 12, 24, 48, 72, 96, 120, 144, 168, 192, 216, 240, 264, 288, 312 and 336 hours after the start of infusion. For the SC groups, the blood samples were collected at pre-dose and at 1, 3, 8, 24, 48, 72, 96, 168, 336, 504, 672, 840, 1008, 1344, 1680, 2016, 2352, 2688, 3024, 3360, 3696 and 4032 hours after dose administration. For PVP SC group, blood collection occurred up to 2352h. The blood samples were centrifuged at approximately 5 °C. The plasma was separated, frozen, and maintained at approximately -80 °C until sample analysis.

An LC-MS/MS method was used to measure the concentration of BIC in rat plasma. Generally, a 10 µL aliquot of each plasma sample with the exception of the matrix blanks, 60 µL of an internal standard solution (carbamazepine, 100 ng/mL) in acetonitrile was added. The matrix blank samples received 60 µL of acetonitrile only. The precipitated proteins were removed by centrifugation and 50 µL of supernatant was transferred into a clean 96 short-well plate. A 50 µL aliquot of water was added to each sample. An aliquot of 6 µL was injected into an Applied Biosystems API-5500 triple quadrupole LC-MS/MS system. The standard curve and quality control (QC) samples were prepared by spiking an appropriate amount of BIC solution, prepared in DMSO, into blank (undosed) rat plasma, then further diluting in blank rat plasma to complete the calibration line.

### HPLC Conditions

*Column:* Waters HSS T3 column (50 X 2.1 mm, 2.5 µm)
*Mobile phases:*
Mobile phase A: 100:0.1 Water: Formic Acid
Mobile phase B: 100:0.1 Acetonitrile: Formic Acid

*HPLC pumps:* Waters Acquity UPLC system with sample organizer
*HPLC elution program:*

| **Time (min)** | **Flow Rate (mL/min)** | **Mobile Phase A (%)** | **Mobile Phase B (%)** |
|---|---|---|---|
| 0.00-1.00 | 0.80 | 75 to 55 | 25 to 45 |
| 1.00-1.05 | 0.80 | 55 to 5 | 45 to 95 |
| 1.05-1.30 | 0.80 | 5 | 95 |
| 1.30-1.50 | 0.80 | 5 to 75 | 95 to 25 |

For estimation of pharmacokinetic parameters, concentrations reported as below the lower limit of quantification (BLQ) were assigned a value of zero if pre-dose and treated as missing thereafter.

Non-compartmental analysis (NCA) was performed on plasma concentration data in order to estimate pharmacokinetic parameters **(Table 1).**

### b) Male Beagle Dogs

This study investigated the pharmacokinetics of three formulations of bictegravir (BIC) by subcutaneous administration to determine the potential to deliver a long acting (LA) pharmacokinetic (PK) profile. BIC was administered by a 30-min intravenous (IV) infusion at 0.5 or 1 mg/kg and by subcutaneous (SC) injection of three LA formulations (e.g., NMP solution, HPMC suspension, and PVP suspension) at 10 mg/kg or 100 mg fixed dose to Male Beagle Dogs. The concentrations of BIC in plasma were determined with a liquid chromatography-tandem mass spectrometry (LC-MS/MS) method.

At dosing, the animals weighed between 7.98 and 11.6 kg. The animals from the SC leg with the PVP suspension were not fasted. The other animals were fasted overnight, and food was returned at approximately 4 hours post dose.

The intravenous dose was administered as a 30-minute infusion via a saphenous vein. After administration, the dose apparatus (i.e., the indwelling catheter) was flushed with approximately 1 mL of saline. The subcutaneous (SC) dose was administered into the dorsal surface between the shoulders and point of the hip. Individual dose was calculated based on body weights recorded on the day of dose administration.

Serial blood samples (approximately 1 mL each) were collected from the cephalic or jugular vein via syringe and needle at specified time points from each animal into single tubes containing K₂EDTA as the anticoagulant and were immediately placed on wet ice prior to centrifugation for plasma. For the IV studies, the blood samples were collected at pre-dose and nominally at 0.25, 0.48, 0.58, 0.75, 1, 1.5, 3, 6, 12, 24, 48, 72, 96, 120, 144, 168, 192, 216, 240, 264, 288, 312 and 336 hours after the start of infusion. For the SC NMP solution and HPMC suspension groups, the blood samples were collected at pre-dose and nominally at 1, 3, 8, 24, 48, 72, 96, 168, 336, 504, 672, 840, 1008, 1344, 1680, 2016, 2352, 2688, 3024 and 3360 hours after dose administration. For the PVP suspension group, the blood samples were collected at pre-dose and at 1, 3, 8, 24, 48, 72, 96, 168, 336, 672, 840, 1008, 1344, 1680 and 2016 hours; additional time-points will be collected up to 4032 hours after dose administration. The blood samples were centrifuged at approximately 5 °C. The plasma was separated, frozen, and maintained at approximately -80 °C until sample analysis.

The pharmacokinetic analysis of samples was performed according to the methods described for the Male Sprague Dawley Rat.

### c) Male Cynomolgus Monkeys

This study investigated the pharmacokinetics of long acting formulations of bictegravir (BIC) by subcutaneous administration to determine the potential to deliver a long acting (LA) pharmacokinetic (PK) profile. BIC was administered by subcutaneous (SC) injection of three LA formulations - NMP solution, HPMC suspension, and PVP suspension - to non-naïve male cynomolgus monkeys. The LA formulations were administered via syringe and needle in the mid-scapular to mid-back region. The NMP solution (BIC 300 mg/mL; Group 1) and HPMC suspension (BIC 250 mg/mL; Group 2) were administered as a single injection at 100 mg/animal. The PVP suspension formulation (BIC 250 mg/mL) was administered at 100 mg/animal (Group 3), at 200 mg/animal (Group 5) or as two equal-volume 100 mg/animal injections for a total of 200 mg/animal (Group 6). The PVP suspension formulation (BIC 125 mg/mL) was also administered at 100 mg/animal (Group 4). BIC plasma concentrations were determined by a liquid chromatography-tandem mass spectrometry (LC-MS/MS) method.

The PK studies were conducted in non-naive animals; select dosings were also done in animals previously exposed to BIC. At dosing, the cynomolgus monkey weighed between 2.5 and 4.0 kg. The animals in all groups were fasted overnight and food was returned at approximately 4 hours post dose.

The subcutaneous dose was administered via a syringe and needle in the mid-scapular region. The dose sites were shaved prior to dose administration and marked and maintained throughout the study as applicable. Individual doses were calculated based on body weights recorded on the day of dose administration.

Serial blood samples (approximately 1 mL each) were collected from jugular vein via syringe and needle at specified time points after dosing from each animal into Vacutainer^{™} tubes containing K₂EDTA as the anti-coagulant and were immediately placed on wet ice prior to centrifugation for plasma. The blood samples were collected predose and at 1, 3, 8, 24, 48, 72, 96, 168, 336, 504, 672, 840, 1008, 1344, 1680, 2016, 2352, 2688, 3024, 3360, 3696, 4032, 4368, 4704, 5040, 5376, 5712, 6048, 6384, 6720, 7056, 7392, 7728, 8064, 8400, and 8736 hours after dosing (as appropriate). The blood samples were centrifuged at approximately 5 °C. The plasma was separated, frozen, and maintained at approximately -70 °C until sample analysis. The pharmacokinetic analysis of samples was performed in a similar manner to the methods described for the Male Sprague Dawley Rat.

### Example 2. Evaluation of BIC Plasma Pharmacokinetic Parameters in Healthy Human Subjects in Phase 1 Clinical Studies

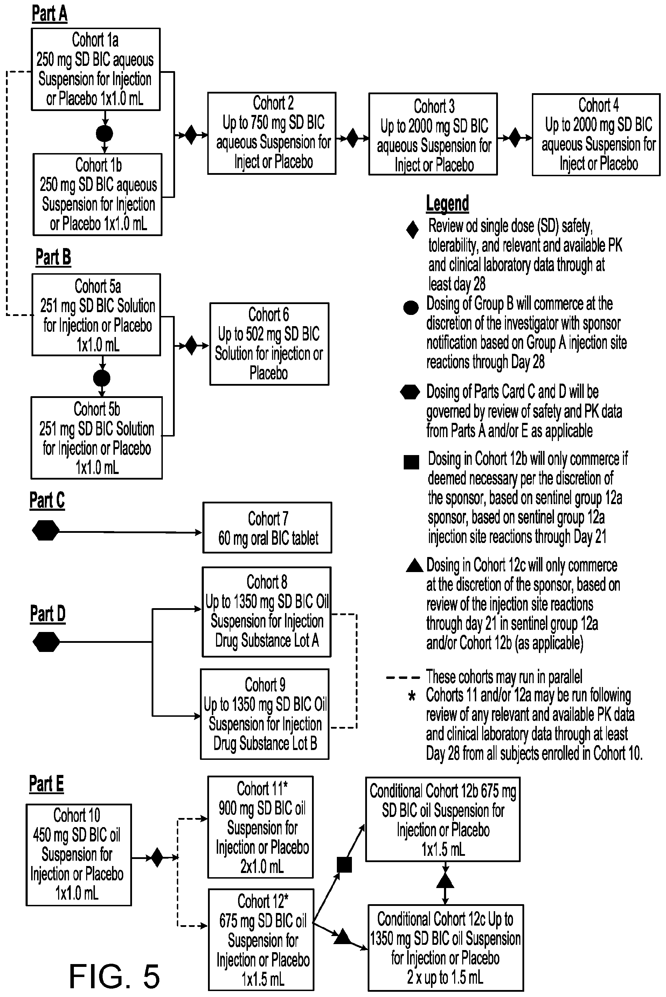

A Phase 1 study is designed to evaluate the safety, tolerability, and PK of escalating single doses of BIC aqueous suspension for injection, BIC solution for injection, and BIC oil suspension for injection in healthy normal subjects. The study also evaluates the PK and safety of single oral doses of a BIC tablet in healthy subjects. A high-level schema of the Ph 1 study is shown in **FIG. 5****.**

### (I) Formulations

Bictegravir for injection is a sterile, white to colored powder that is reconstituted prior to administration as either an aqueous suspension, oil suspension, or solution formulation for SC injection at a final concentration of 250, 450, and 251 mg/mL, respectively. Examples of three formulations and methods are provided below.

### A. Suspension Formulations

One suspending vehicle for BIC suspension for injection is a sterile, clear solution containing povidone (PVP), polysorbate 20, sodium chloride, sodium phosphate dibasic heptahydrate, sodium phosphate monobasic monohydrate, hydrochloric acid, sodium hydroxide, and sterile water for injection.

Another suspending vehicle for BIC suspension is sterile, clear solution containing a medium chain triglyceride for injection.

### B. Solution Formulation

The solution vehicle for BIC solution for injection is a sterile, clear solution containing N-methyl-2-pyrrolidone (NMP) prepared prior to use.

### (II) Methods

### a) Healthy Male and Nonpregnant, Nonlactating Female Human Subjects, Aged 18 to 45, Inclusive

This study investigates the pharmacokinetics of long acting formulations of bictegravir (BIC) by subcutaneous administration to determine the potential to deliver a long acting (LA) pharmacokinetic (PK) profile. BIC is administered by subcutaneous (SC) injection of three LA formulations - NMP solution, PVP suspension and Miglyol suspension to healthy male and nonpregnant, nonlactating female human subjects, aged 18 to 45, inclusive. The LA formulations are administered via syringe and needle in the abdomen region. The NMP solution (BIC 251 mg/mL) and PVP suspension (BIC 250 mg/mL) are administered as a single injection at 251 mg/subject (NMP solution) or at 250 mg/subject (PVP suspension).

### (III) Dose Escalation

Cohort 1 and Cohort 5 of Parts A and B, respectively, are dosed in 2 groups, the sentinel group (4 active and 1 placebo, randomly assigned) and the remainder of the cohort. Dosing of the remainder of the cohort (8 active and 2 placebo, randomly assigned) commences at the discretion of the investigator with sponsor notification, based on review of the sentinel group injection site reactions through Day 28.

Initiation of a single-dose administration in Cohorts 2 to 4 in Part A and Cohort 6 in Part B is determined upon evaluation of safety and any relevant and available PK and clinical laboratory data through at least Day 28 from all subjects enrolled in the previous dosing cohorts.

For Cohorts 2 to 4, selected doses (up to 2000 mg) are either lower than or no more than 3-fold higher than the highest previously evaluated dose in Part A. For Cohort 6, selected doses (up to 502 mg) are either lower than or no more than 2-fold higher than the highest previously evaluated dose in Part B. Escalation to a dose higher than previously studied occurs only in the absence of dose-limiting toxicity (DLT) and/or meeting any pre-specified stopping criteria.

Cohorts may be initiated in parallel only if the dose under evaluation is at or below a dose previously evaluated.

Cohort 10 of Part E proceeds in two groups: 4 active and 1 placebo (first group) and 8 active and 2 placebo (second group). Dosing in the second group is staggered by a minimum of 10 days relative to dosing in the first group, remaining subjects are randomized based on the review of available safety and tolerability data from the preceding group. A starting dose of 450 mg BIC oil suspension for injection or placebo are administered as a single 1.0 mL SC injection in the abdomen on Day 1.

Cohort 12 is dosed in three groups: a sentinel cohort (Cohort 12a; 4 active and 1 placebo, randomly assigned), Conditional Cohort 12b (8 active and 2 placebo, randomly assigned), and Cohort 12c (12 active and 3 placebo, randomly assigned). Cohort 12b is a conditional cohort which only initiates if deemed necessary per the discretion of the sponsor, based on review of the injection site reactions through Day 21 in sentinel cohort 12a. In addition, cumulative safety data from prior cohort(s) are assessed, as applicable. Cohort 12c commences at the discretion of the sponsor, based on review of the injection site reactions through Day 21 in sentinel cohort 12a and/or Cohort 12b (as applicable). Additional cumulative safety data from prior cohort(s) may also be assessed.

For any cohort within Part E, the maximum injection volume is no more than 1.5 mL per injection. Escalation to a dose higher than previously studied occurs only in the absence of DLT and/or meeting any pre-specified stopping criteria.

For any given cohort, the sponsor may elect to hold dosing, select an intermediate dose, or stop study enrollment at any time based on review of the preliminary safety data.

Based on review of relevant safety and PK data by the Safety Review Team (SRT), and in discussion with the investigator, escalation to a higher dose occurs only in the absence of DLT and/or meeting any pre-specified stopping criteria as specified in the preceding paragraph. The decision to dose escalate and the dose to be studied in each cohort (if applicable) is made by the SRT.

### (IV) Subject Population

Up to 186 unique subjects are enrolled in the study, with an approximately even distribution of healthy male and nonpregnant, nonlactating female subjects of 18 through 45 years of age, inclusive.

### (V) Dosage and Administration of BIC

Following completion of screening and admission assessments, eligible subjects are enrolled to receive study treatments as shown below.

### Part A. Single Ascending Doses BIC aqueous suspension for injection (Cohorts 1 to 4):

Part A characterizes the PK of single ascending doses of SC BIC aqueous suspension for injection. Progression within this part is governed by review of safety and PK data, and application of stopping rules. Following completion of screening and Day -1 procedures for Cohort 1a, eligible subjects are randomized 4:1 to receive a single dose of either SC BIC aqueous suspension for injection (250 mg/mL; N = 4) or placebo (N = 1). Following completion of screening and Day -1 procedures for Cohort 1b, eligible subjects are randomized 8:2 to receive a single dose of either BIC aqueous suspension for injection (250 mg/mL; N = 8) or placebo (N = 2).

Following completion of screening and Day -1 procedures for Cohorts 2 to 4, eligible subjects are randomized 12:3 per cohort to receive single doses of either BIC aqueous suspension for injection (250 mg/mL; N = 12) or placebo (N = 3), with staggered dose escalations. In Cohort 1, single dose of 250 mg BIC aqueous suspension for injection or placebo is administered as a single 1.0 mL SC injection in the abdomen on Day 1. For Cohorts 2 to 4, BIC aqueous suspension for injection or placebo is administered as single or multiple SC injections at different abdominal sites, as appropriate. In the case where multiple injections are administered for a given dose, the first and last injections is no more than 15 minutes apart. For any cohort, the maximum injection volume is no more than 2.0 mL per injection.

The treatments for each cohort are as follows:

| **Cohort** | **Subjects** | **Dose of BIC Aqueous Suspension for Injection or Placebo** |
|---|---|---|
| 1a | 4 active / 1 placebo | 250 mg |
| 1b | 8 active / 2 placebo | 250 mg |
| 2 | 12 active / 3 placebo | Up to 750 mg |
| 3 | 12 active / 3 placebo | Up to 2000 mg |
| 4 | 12 active / 3 placebo | Up to 2000 mg |

### Part B. Single Ascending Doses BIC solution for injection (Cohorts 5 and 6):

Part B characterizes the PK of single ascending doses of BIC solution for injection. Cohort 5 runs in parallel with Part A and progression to Cohort 6 is governed by review of safety and PK data from Cohort 5, and application of stopping rules.

Following completion of screening and Day -1 procedures for Cohort 5a, eligible subjects are randomized 4:1 to receive a single dose of either BIC solution for injection (251 mg/mL; N = 4) or placebo (N = 1).

Following completion of screening and Day -1 procedures for Cohort 5b, eligible subjects are randomized 8:2 to receive a single dose of either BIC solution for injection (251 mg/mL; N = 8) or placebo (N = 2).

Following completion of screening and admission procedures for Cohort 6, eligible subjects are randomized 12:3 to receive a single dose of either BIC solution for injection (251 mg/mL; N = 12) or placebo (N = 3).

In Cohort 5, single dose of 251 mg BIC solution for injection or placebo is administered as a single 1.0 mL SC injection in the abdomen on Day 1.

In Cohort 6, BIC solution for injection or placebo is administered as a single or multiple SC injections at different abdominal sites, as appropriate. The first and last injections is no more than 15 minutes apart if multiple injections are administered.

For any cohort, the maximum injection volume is no more than 2.0 mL per injection.

The treatments for each cohort are as follows:

| **Cohort** | **Subjects** | **Dose of BIC Aqueous Suspension for Injection or Placebo** |
|---|---|---|
| 5a | 4 active / 1 placebo | 251 mg |
| 5b | 8 active / 2 placebo | 251 mg |
| 6 | 12 active / 3 placebo | Up to 502 mg |

### Part C. Oral BIC Tablet (60 mg; Cohort 7):

Initiation of dosing within this cohort is determined upon evaluation of relevant and available PK data from Parts A and E.

The treatment within this part is as follows:

| **Cohort (Subjects)** | **Period** | **1** | | **2** |
|---|---|---|---|---|
| 7 (12 active) | Study Day | Day 1 | Days 2 to 8 | Day 9 |
| | Treatment | 60 mg (1 × 60 mg) oral BIC tablet under fasted conditions | Washout | 60 mg (1 × 60 mg) oral BIC tablet under fed conditions with a high-fat meal |

### Part D. BIC oil suspension for injection, BIC drug substance Lots A and B (Cohorts 8 and 9):

Part characterizes the PK of single doses of BIC oil suspension for injection, 450 mg/mL, containing two distinct lots of BIC drug substance (Lots A and B) having different particle size distributions. Initiation of dosing within this part is governed by review of available and relevant safety and PK data from Part E. This part includes two Cohorts (8 and 9) that run in parallel.

In Cohorts 8 and 9, single dose of BIC oil suspension for injection is administered as a single or multiple SC injections at different abdominal sites, as appropriate. The first and last injections is no more than 15 minutes apart if multiple injections are administered.

The treatments for each cohort are as follows:

| **Cohort** | **Subjects** | **Dose of BIC Oil Suspension for Injection** |
|---|---|---|
| 8 | 12 active | Up to 1350 mg (Lot A) |
| 9 | 12 active | Up to 1350 mg (Lot A) |

### Part E. Single Ascending Doses BIC oil suspension for Injection (Cohorts 10, 11, and/or 12):

Part E characterizes the PK of single ascending doses of BIC oil (medium chain triglyceride) suspension for injection. Progression within this part is governed by review of safety and any available PK data, and application of stopping rules.

### Cohort 10

Cohort 10 is an adaptive cohort and can be run anytime during the study. Following completion of screening and Day -1 procedures for Cohort 10, a total of 15 eligible subjects are randomized as 12:3 to receive either BIC oil suspension for injection (450 mg/mL; N = 12) or placebo (N = 3). Cohort 10 proceeds in two groups: 4 active and 1 placebo (first group) and 8 active and 2 placebo (second group). Dosing in the second group is staggered by a minimum of 10 days relative to dosing in the first group, remaining subjects are randomized based on the review of available safety and tolerability data from the preceding group. A starting dose of 450 mg BIC oil suspension for injection or placebo is administered as a single 1.0 mL SC injection in the abdomen on Day 1.

### Cohort 11

Following completion of screening and Day -1 procedures for Cohort 11, eligible subjects are randomized 12:3 to receive either BIC oil suspension for injection (450 mg/mL; N = 12) or placebo (N = 3). 900 mg BIC oil suspension for injection or placebo is administered as two 1.0 mL SC injections at different abdominal sites. The first and the last injections is no more than15 minutes apart.

### Cohort 12

Cohort 12 is dosed in three groups (as applicable): a sentinel cohort (Cohort 12a; 4 active and 1 placebo, randomly assigned), Conditional Cohort 12b (8 active and 2 placebo, randomly assigned), and Cohort 12c (12 active and 3 placebo, randomly assigned).

*Sentinel Cohort 12a* - Following completion of screening and Day -1 procedures for Cohort 12a, eligible subjects are randomized 4:1 to receive either BIC oil suspension for injection (450 mg/mL; N = 4) or placebo (N = 1). 675 mg BIC oil suspension for injection or placebo is administered as a single 1.5 mL SC injection in the abdomen on Day 1.

*Conditional Cohort 12b* - Following completion of screening and Day -1 procedures for Cohort 12b, eligible subjects are randomized 8:2 to receive either BIC oil suspension for injection (450 mg/mL; N = 8) or placebo (N = 2). 675 mg BIC oil suspension for injection or placebo is administered as a single 1.5 mL SC injection in the abdomen on Day 1.

*Cohort 12c* - Following completion of screening and Day -1 procedures for Cohort 12c, eligible subjects are randomized 12:3 to receive either BIC oil suspension for injection (450 mg/mL; N = 12) or placebo (N = 3). Up to 1350 mg BIC oil suspension for injection or placebo is administered as two SC injections of up to 1.5 mL at different abdominal sites. The first and the last injections is no more than 15 minutes apart.

For any cohort within Part E, the maximum injection volume is no more than 1.5 mL per injection.

The treatments for each cohort are as follows:

| **Cohort** | **Subjects** | **Dose of BIC Oil Suspension for Injection or Placebo (# of injs. x inj. volume** |
|---|---|---|
| 10 ^{a} | 12 active / 3 placebo | 450 mg (1 x 1.0 mL) |
| 11 ^{b,c} | 12 active / 3 placebo | 900 mg (2 x 1.0 mL) |
| 12a ^{b,c} | 4 active / 1 placebo | 675 mg (1 x 1.5 mL) |
| 12b ^{d} | 8 active / 2 placebo | 675 mg (1 x 1.5 mL) |
| 12c^{e} | 12 active / 3 placebo | Up to 1350 mg (2 x up to 1.5 mL) |

| | | |
|---|---|---|
| ^{a} Cohort 10 proceeds in two groups, dosing in the second group is staggered by a minimum of 10 days relative to dosing in the first group and commences following review of available safety from the preceding group. ^{b} Based on the review of safety, tolerability and relevant and available PK/clinical laboratory data through at least Day 28 from all subjects in Cohort 10, dosing in Cohort 11 and/or 12a is initiated (as described above). ^{c} If a decision is made to run both Cohorts 11 and 12a, they may proceed in parallel. ^{d} Conditional cohort which will only initiate if deemed necessary per the discretion of the sponsor, based on review of the injection site reactions through Day 21 in sentinel cohort 12a. ^{e} Dosing in Cohort 12c initiates per the discretion of the sponsor, based on review of the injection site reactions through Day 21 in sentinel cohort 12a and/or Cohort 12b (as applicable). | | |

### (VI) Fasting and Meals

Parts A, B, D, and E (Cohorts 1 to 6, and 8 to 12):
All study treatments are administered in the morning without regards to fasting status.

### Part C (Cohort 7):

### Period 1 (fasted state dosing):

Following an overnight fast (no food or drinks except water for at least 10 hours), study treatment is administered in the morning on Day 1 with 240 mL of water. Subjects continue to fast until after collection of the 4-hour PK sample on Day 1, relative to the study drug dosing. Additionally, on Day 1, subjects are restricted from water consumption 1 hour before and until 2 hours after study drug dosing, except for the 240 mL given with the study treatment. A meal (standardized lunch) is provided to subjects after the 4-hour postdose blood draw.

### Period 2 (fed state dosing):

Following an overnight fast (no food or drinks except water for at least 10-hours), the study treatment is administered with food and 240 mL of water in the morning on Day 9. A meal is initiated 30 minutes prior to study drug administration. The doses are administered at or within 5 minutes of subjects completing a high-fat meal containing ~ 1000 kcal and ~ 50% fat. Subjects fast for 4-hours after study drug administration. Other than the water provided with dosing and beverages provided with the standardized meal, water and other fluids are withheld for 1 hour before and until 2-hours after study drug dosing. Water may be consumed by subjects following the 2-hours blood draw for the remainder of the collection period. A meal (standardized lunch) is provided to subjects after the 4-hours postdose blood draw. In both the periods, on days with no study drug administration, there is no restriction on food and water intake.

All meals and/or snacks given to subjects during their stay in the clinical study facility are standardized for all subjects and are similar in calorie and fat content and taken at approximately the same time each day (e.g., 07:30, 12:00, and 18:00). All meals provided must be approved by the sponsor. Components of meals (e.g., margarine, jelly, bread) are given to subjects in individual portions (e.g., 1 tablespoon) per the approved meal schedule. The provision of meal components in bulk (e.g., a jar of jelly for subjects to share) is not practiced.

### (VII) Plasma Pharmacokinetic Collection

### Parts A, B, D, and E (Cohorts 1 to 6, and 8 to 12):

Immediately following dosing on Day 1, a sample collection period of up to 84 days begins, during which time blood samples for plasma PK are collected at predetermined timepoints. The duration of sample collection may be extended up to Day 126 [up to Day 182 in Parts D and E (Cohorts 8 through 12)], based upon available PK data. The duration of each sample collection period is dependent on monitoring of the plasma concentrations and stops when BIC levels in all evaluable subjects in a given cohort are below the limit of quantitation (BLQ) for at least 2 consecutive visits. If PK sample collection is stopped for this reason, the subjects are discontinued from all further follow-up.

Serial blood samples for plasma PK assessments of BIC occurs relative to the morning dosing of BIC for injection on Day 1 at the time points below.
i) 0 (predose, ≤ 5 min before dose), 1, 2, 4, 6, 8, 12, 24, 48, 72, 96, 120, 144, and 168 hours postdose
ii) Single PK samples are drawn in the morning at approximately the same time as the predose sample on Days 10, 14, 17, 21, 24, 28, 31, 35, 38, 42, 49, 56, 63, 70, 77, 84, and at the ET visit (if applicable)
iii) Additional PK samples (if applicable) are drawn anytime on Days 98, 112, and 126
iv) Applicable to Parts D and E (Cohorts 8 through 12) only: based on the emerging PK data, anytime PK samples may be drawn on Days 140, 154, 168, and 182

### Part C (Cohort 7):

Serial blood samples for plasma PK assessments occurs at the following timepoints relative to the morning dosing of oral BIC tablet on Days 1 and 9: 0 (predose, ≤ 5 min before dose), 0.5, 1, 2, 4, 6, 8, 12, 24, 48, 72, 96, and 120 hours postdose. Plasma concentrations of BIC are determined and PK evaluated.

The above procedure may be appropriately modified for IM injection of the formulations described herein.

### Example 3: Development of Long-Acting Injectable Bictegravir (BIC)

### Physiochemical Properties of BIC Free Acid Form III and BIC Sodium

BIC can exist (among other forms) as a free acid and sodium salt (see, e.g., U.S. Patent Nos. 9,682,084 and 9,708,342). Multiple crystalline forms of BIC free acid are known. Free acid Form III has suitable physicochemical properties (e.g., physically and chemically stable and has the lowest aqueous solubility and intrinsic dissolution rate at physiological pH) for long-acting injectable (LAI) formulations and was selected for further development.

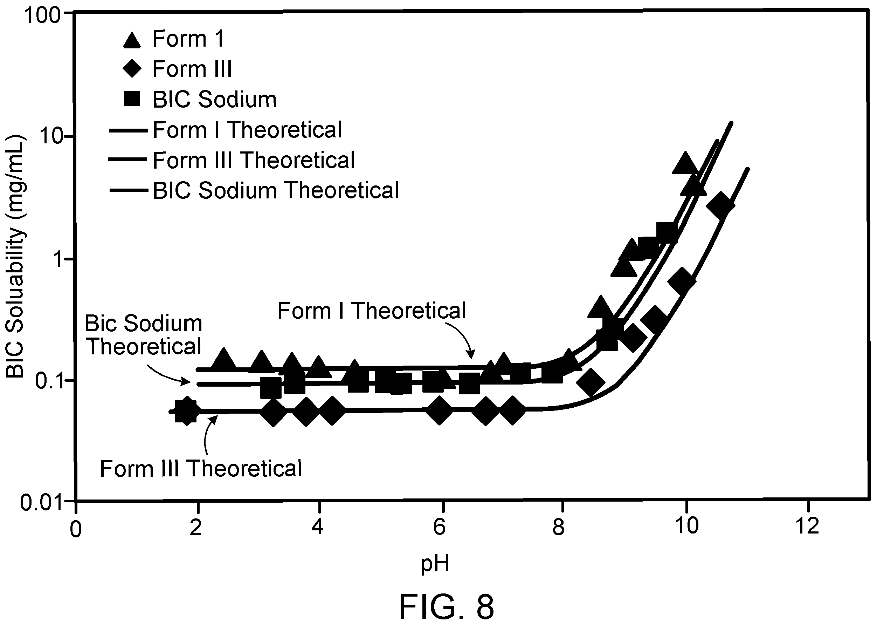

BIC sodium rapidly disproportionates to free acid Form I in aqueous media. **FIG. 8** shows the thermodynamic solubility profiles for BIC sodium and BIC free acid forms I and III.

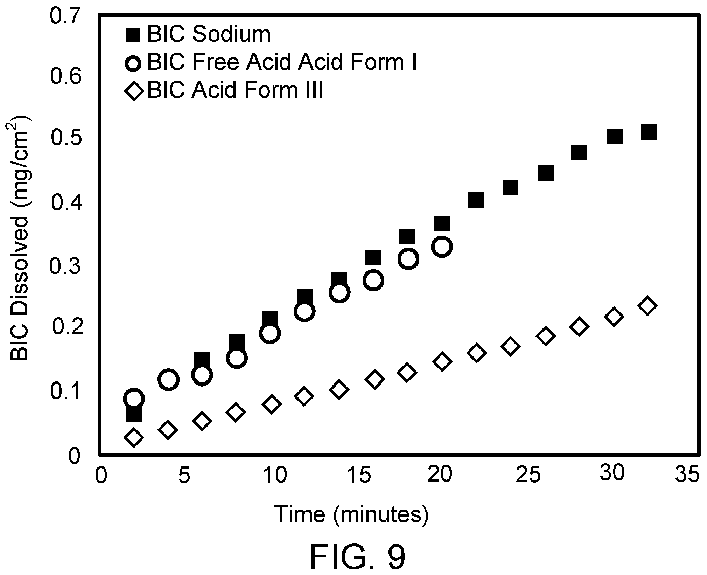

Free acid Form III exhibits a slower dissolution rate than both the free acid Form I and the sodium salt **(****FIG. 9****).**

The solubility of BIC free acid Form III as compared to BIC sodium in a variety of organic solvents and excipients is shown below in **Table 2.**

**Table 2. Solubility of BIC free acid Form III and BIC sodium**

| **Media** | **BIC Sodium Solubility (mg/mL)** | **BIC Form III Solubility (mg/mL)** |
|---|---|---|
| **Organic Solvents** | | |
| N-methylpyrrolidone | 13 | 310-325 |
| Dimethyl sulfoxide (DMSO) | 4.74 | 248 |
| Acetonitrile (ACN) | 0.002 | 34.4 |
| Ethanol (EtOH) | 0.06 | 15.2 |
| Dichloromethane (DCM) | 0.06 | 263 |
| Methanol (MeOH) | 0.60 | 16.8 |
| 75% NMP: 25% Water | 1.8 | 1.8 |
| 75% NMP: 25% Glycofurol | 62.6 | 4.3 |
| 75% NMP: 25% Propylene glycol | 61.4 | 2.6 |
| 75% NMP: 25% Ethanol | 166.9 | 2.0 |
| 50% NMP: 50%: Ethanol | 91.7 | 0.7 |
| 25% NMP: 75% Ethanol | 65.1 | 0.3 |

| **Excipients** | | |
|---|---|---|
| Octanoic acid (C8:0) | 41 | 9.3 |
| Miglyol 812 N | 0.004 | 0.87 |
| Sesame oil | 0.03 | 0.29 |
| Polyethylene glycol 300 | 0.23 | 1.42 |
| Propylene glycol | 0.73 | 4.2 |
| Glycofurol | 4.3 | 7.7 |
| Polysorbate 20 | 0.78 | 4.99 |
| Polysorbate 80 | 0.23 | 0.11 |
| 30% Captisol in water, pH 6 | 0.48 | 4.4 |

### Example 4. Suspension and Solution Formulations of Bictegravir

### (I) Suspension Formulations

One suspension formulation comprises 250 mg/mL micronized BIC free acid Form III in 2% Povidone K12 (PVP), 0.5% Polysorbate 20, 0.8% sodium chloride in 10 mM sodium phosphate buffer, pH 7.4. The formulation is provided as a two-component product (i.e., a kit): 1) terminally sterilized micronized BIC Form III in glass vials (e.g., with a micronized d₉₀ < 30 µm), and 2) aqueous vehicle (e.g., see **Table 3)** sterilized by filtration and filled aseptically in glass vials. Upon mixing, the formulation comprises a withdrawable volume of ≥ 2.0 mL for up to 500 mg dose per injection site to be administered through, e.g., a 18-25G needle. The mixed suspension composition is shown in **Table 4** and **Table 5.** Pharmacokinetic data for PVP suspensions of BIC free acid form III are found in **FIG. 2****.**

**Table 3. Sample Aqueous Vehicle Composition**

| **Component** | **% w/w per Vial** |
|---|---|
| Povidone K12 | 2.00 |
| Polysorbate 20 | 0.50 |
| Sodium Chloride | 0.76 |
| Sodium Phosphate Monobasic Monohydrate (Na₂HPO₄ · 7H₂O) | 0.03 |
| Sodium Phosphate Dibasic Heptahydrate (Na₂HPO₄ · 7H₂O) | 0.20 |
| Sterile Water for Injection | 96.50 |
| Total | 100.0 |

**Table 4. Sample BIC LAI suspension upon mixing**

| **Component** | **Function** | **% w/w** |
|---|---|---|
| BIC FA III | API | 22.97 |
| Povidone K12 | Suspending Agent | 1.54 |
| Tween 20 | Wetting Agent | 0.39 |
| Sodium Chloride | Tonicity To achieve 300 mOsm | 0.59 |
| Sodium Phosphate Monobasic Monohydrate (NaH₂PO₄ · H2O) | Buffer (20 mM, pH 7.4) | 0.03 |
| Sodium Phosphate Dibasic Heptahydrate (Na₂HPO₄ · 7H₂O) | | 0.15 |
| Hydrochloric Acid | pH Adjustment | N/A |
| Sodium Hydroxide | | N/A |
| Sterile Water for Injection | Vehicle | 74.33 |
| **Total** | | **100.00** |

**Table 5. Sample BIC LAI suspension upon mixing**

| **Component** | **Function** | **% w/w** |
|---|---|---|
| BIC FA III* | API | 23.60 |
| Povidone K12 | Suspending Agent | 1.53 |
| Tween 20 | Wetting Agent | 0.38 |
| Sodium Chloride | Tonicity To achieve 300 mOsm | 0.58 |
| Sodium Phosphate Monobasic Monohydrate (NaH₂PO₄ · H₂O) | Buffer (20 mM, pH 7.4) | 0.02 |
| Sodium Phosphate Dibasic Heptahydrate (Na₂HPO₄ · 7H₂O) | | 0.15 |
| Sterile Water for Injection | Vehicle | 73.73 |
| **Total** | | **100.00** |

| | | |
|---|---|---|
| *d₉₀: 17 µm | | |

Further contemplated suspension formulations comprise BIC free acid form III in mixtures of NMP and DMSO, e.g., 50% DMSO/50% NMP. Such suspensions provide for increased BIC loading. For example, a 50% DMSO/50% NMP solution can achieve a BIC drug substance concentration of about 700 mg/mL with suitable injectability properties.

### (II) Solution Formulations

A sample solution formulation comprises about 250 mg/mL (or, e.g., 300 mg/mL) BIC in NMP. This formulation is supplied as a two-component product (i.e., a kit): 1) terminally sterilized micronized BIC free acid form III in one or more glass vials (e.g., with a particle size distribution, e.g., d₉₀ < 30 µm), and 2) NMP filled aseptically in one or more glass vials. The solution is compounded at the time of use and sterilized by filtration. The solution can be administered via, e.g., a 26G needle. Pharmacokinetic data for a NMP solution of BIC free acid form III (300 mg/mL) are found in **FIG. 1****.** A further example of a solution formulation consists of 31.38wt% BIC free acid and the 68.72 wt% NMP, wherein the concentration of BIC free acid is 251 mg/mL.

### Example 5. Development of Additional LAI Suspension Formulations

Single component suspensions of BIC for injection were also investigated, examples of which are provided below.

### (I) Aqueous Nanosuspension of BIC Sodium Salt

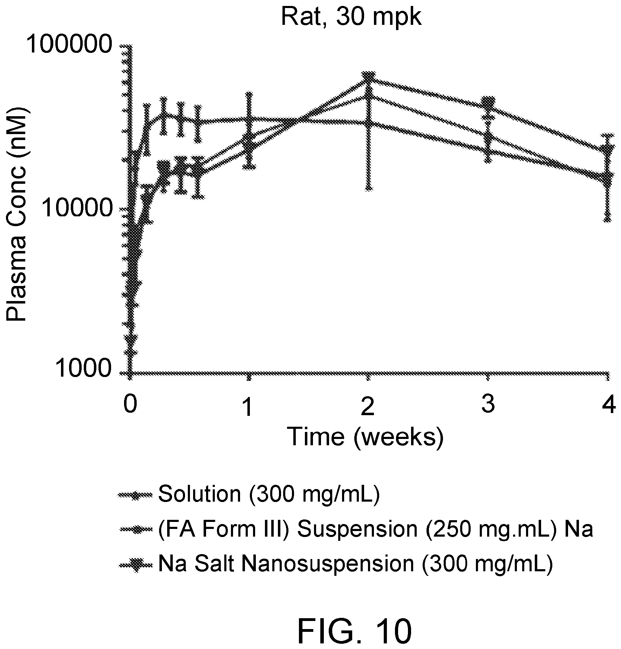

Nanosuspensions of BIC sodium salt can be achieved in aqueous media comprising 2% poloxamer 188 in water. The nanosuspension can be obtained on both Netzsch mill and microfluidizer, resulting in particle size d₉₀ of 427 nm (z-average on DLS), 807 nm (Malvern). Pharmacokinetic data in a rat for an exemplary 300 mg/mL BIC sodium salt nanosuspension (e.g., 2% poloxamer 188 in water) as compared to the BIC free acid form III solution (NMP) at 300 mg/mL and the BIC free acid form III PVP suspension at 250 mg/mL are shown in FIG. 10.

### (II) Triglyceride or Oil Suspensions of BIC Sodium Salt or BIC Free Acid

Suspensions of BIC sodium salt (BIC Na) and BIC free acid (BIC FA) can be achieved in a triglyceride based media, e.g., Miglyol 812N, a triglyceride ester of saturated coconut/palm kernel oil derived caprylic and capric fatty acids and plant derived glycerol (60/40 caprylic acid (C8:0)/capric acid (C10:0). Sample formulations of bictegravir suspensions with Miglyol 812N and sesame oil are shown in **Table 6.**

**Table 6. Sample BIC suspensions**

| | **No.** | **Formulation** |
|---|---|---|
| Nano-suspension*, bead milling | 1 | BIC Na in Miglyol 812N, 250 mg/mL |
| | 2 | BIC Na in Miglyol 812N, high DL |
| Micro-suspension API, jet milled: d₉₀: 17 µM | 3 | BIC FA Form III in 2% w/w PVP K12, 0.5% w/w Polysorbate 20, 0.76% NaCl in 10 mM sodium phosphate buffer, pH 7.4, 250 mg/mL |
| | 4 | BIC FA Form III in Miglyol 812N, 250 mg/mL |
| | 5 | BIC FA Form III in Miglyol 812N, 450 mg/mL |
| HSWM^{a} Micro-suspension API: d₉₀: 32 µM | 6 | BIC FA Form III in 2% w/w PVP K12, 0.5% w/w Polysorbate 20, 0.76% NaCl in 10 mM sodium phosphate buffer, pH 7.4, 250 mg/mL |
| | 7 | BIC FA Form III in Miglyol 812N, 250 mg/mL |
| | 8 | BIC FA Form III in Miglyol 812N, 450 mg/mL |
| Micro-suspension API, jet milled: d₉₀ 14 µM | 9 | BIC FA Form III, 250 mg/mL in Miglyol 812N, (40.69 wt% of BIC FA Form III and 59.31 wt% of Miglyol 812N) |

| | | |
|---|---|---|
| ^{a}HSWM = High Shear Wet Milling | | |

### Example 6: Pharmacokinetics Parameters of Micro-suspensions of BIC FA form III in Sesame Oil versus in Miglyol 812N in Rats

A micro-suspension of BIC FA Form III was explored to determine if such formulations would enhance the long acting nature of the formulations based on the expected slower absorption of sesame oil at the injection site. A 250 mg/mL suspension was formed by suspending BIC FA Form III (d₉₀: 14 µm) in 100% sesame oil. The mixture was sonicated for vortexed for 90 seconds, sonicated for 20 min, and vortexed for an additional 10 seconds to homogenize the mixture. Formulations up to 450 mg/ml were prepared using similar methods. A rat PK study was performed according to methods described previously, where the 250 mg/ml micro-suspension was dosed at 30 mg/kg. The PK data is summarized in **Table** 7. Ultimately the sesame oil formulation had plasma concentration comparable to the Miglyol formulation, the sesame oil formulation was not carried forward due to lower Cmax when compared to the Miglyol formulation.

**Table 7. Pharmacokinetics Parameters of Micro-suspensions of BIC FA form III in Sesame Oil versus in Miglyol 812N in Rats**

| | **BIC FA form III in Miglyol 812N** | **BIC FA form III in Sesame Oil** |
|---|---|---|
| Cₘₐₓ (nM) | 55300 | 36100 |
| Tₘₐₓ (h) | 336 | 168 |
| AUC 0-3360 (µM*h) | 32300 ± 1310 | -- |
| F% | 88.4 ± 4.4 | -- |
| AUC 0-1680 (µM*h) | 32200 ± 1390 | 20300 ± 10000 |
| F% | 88.1 ± 4.3 | 44.2 ± 24.2 |
| AUC 0-1008 (µM*h) | 30900 ± 2700 | 20200 ± 9950 |
| F% | 84.3 ± 6.8 | 44.0 ± 24.1 |
| AUC 0-672 (µM*h) | 27700 ± 4770 | 19000 ± 9320 |
| F% | 75.6 ± 11.8 | 41.3 ± 22.6 |
| AUC 0-168 (µM*h) | 7430 ± 1600 | 5520 ± 2410 |
| F% | 20.2 ± 3.7 | 11.8 ± 5.2 |

### Example 7. Preparation and Characterization of BIC Free FA Form IX and Form X

BIC free acid Form IX can be prepared from BIC free acid as shown in Formula (I) according to the procedures as described herein. To a 100 mL jacketed glass reactor equipped with overhead retreat curve agitation was charged 38.5 g of a 1.075 mg/mL solution of BIC free acid in NMP. The agitation rate was set to 200 rpm and the reactor was heated to 55 °C. The reactor was then rapidly charged (within about 5 seconds) with 50 mL water. Upon the addition of water, the internal temperature of the reaction mixture dropped by about 10 °C and the solution became cloudy (white), then an oily, gummy residue formed rapidly. After about 45 minutes, the mixture returned to a cloudy white appearance. The mixture was aged at 55 °C for about one additional hour and then cooled to 20 °C in about 1 hour, then aged for an additional 1 hour. The suspension was filtered, and the filtered cake was washed with 77 mL of 8:2 (v/v) H₂O: NMP followed by 77 mL of water. The wet cake was dried under vacuum and N₂ sweep at 55 °C for about 19 hours.

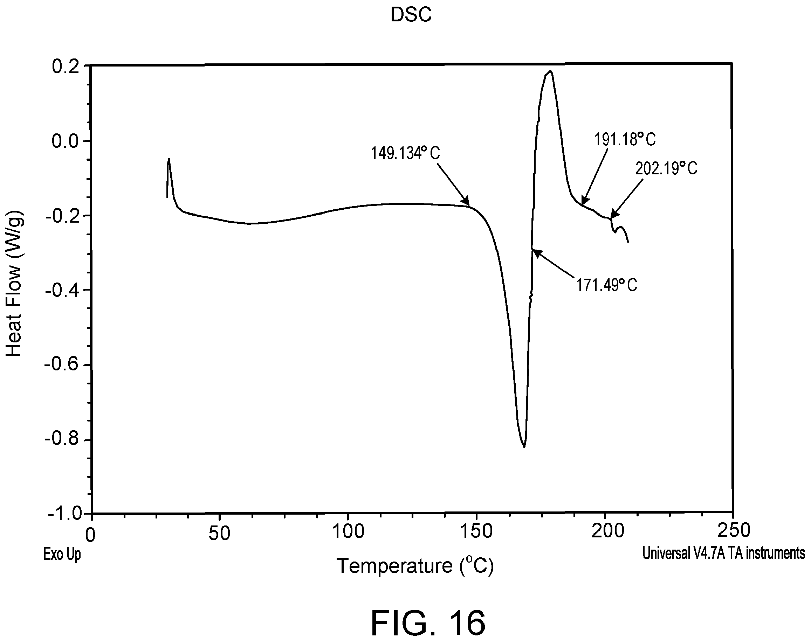

BIC FA Form X can be prepared by heating BIC FA Form IX to about 210 °C using the DSC as shown in **FIG. 16****.** In this case a broad endothermic event with onset at about 149 °C is followed by a broad exothermic event at about 172 °C. A small endothermic event at about 202 °C is taken as the melt of Form III. To prepare sufficient solids for testing, a number of DSC experiments were performed and the solids were combined for analysis by XRPD and DSC.

BIC free acid Form IX and Form X were characterized by X-ray powder diffraction (XRPD), differential scanning calorimetry (DSC), and thermogravimetric analysis (TGA). X-ray powder diffraction (XRPD) analysis was conducted on a diffractometer (PANalytical XPERT-PRO, PANalytical B. V., Almelo, Netherlands) using copper radiation (Cu Kα, λ = 1.541874 Å). Samples were spread evenly on a zero-background sample plate. The generator was operated at a voltage of 45 kV and amperage of 40 mA. Slits were Soller 0.02 rad, antiscatter 1.0°, and divergence of 7mm. Scans were performed from 2 to 40° 2θ with a 0.0167 step size. Data analysis was performed using X'Pert Data Viewer V1.2d (PANalytical B.V., Almelo, Netherlands). X-ray powder diffraction analysis was also conducted on a diffractometer (Rigaku MiniFlex, Rigaku Corporation, Tokyo, Japan) using copper radiation (Cu Kα, λ= 1.541874 Å). Samples were spread evenly on a zero-background sample plate. The generator was operated at a voltage of 40 kV and amperage of 15 mA. Scans were performed from 2 to 40° 2θ with a 0.050-degree step size and a speed of 2-8 degrees/minute. Data analysis was also performed using X'Pert Data Viewer V1.2d (PANalytical B.V., Almelo, Netherlands).

DSC analyses were performed by loading 1-5 mg of material into a crimped Tzero standard aluminum pan and heating the sample at 10 °C/min from 20 to 300 °C or above. The sample and reference pans were under a 50 mL/min nitrogen purge. Data analysis was completed using Universal Analysis 2000 Version 4.7A (TA Instruments, New Castle, DE).

TGA analyses were performed by loading 1-10 mg of material onto an aluminum weigh pan (TA Instruments, New Castle, DE) and heated the sample to 350 °C or above at a rate of 10 °C/min. The sample and reference pans were under a 60 mL/min and 40 mL/min nitrogen purge, respectively. Data analysis was completed using Universal Analysis 2000 Version 4.7A (TA Instruments, New Castle, DE).

The XRPD pattern of BIC free acid Form IX is characterized by sharp reflections, indicating crystallinity **(****FIG. 12****).** Characteristic peaks from the XRPD diffractogram are summarized in **Table 8.** The DSC **(****FIG. 13****)** shows a broad endothermic event with onset at about 156 °C which is followed by sharp endothermic event at about 200 °C and a much weaker endothermic event at about 213 °C. The endothermic event at about 156 °C is believed to represent a transformation of Form IX to a mixture of two forms. The event at about 200 °C is believed to correspond to the melt of Form III and the event at about 213 °C is believed to correspond to the melt of Form X. The TGA traces show variable mass losses on heating from different samples (0.15, 0.25, and 0.74 wt% by 150 °C). Form IX has been formed in pure phase at the mg scale, and the XRPD is shown in **FIG. 12****.**

**Table 8. Characteristic XRPD peaks observed in the XRPD Diffractogram of BIC FA Form IX.**

| Position [°2Theta] | Relative Intensity [%] |
|---|---|
| 16.7 | 100.0 |
| 28.3 | 89.0 |
| 6.7 | 78.7 |
| 14.4 | 66.4 |
| 14.3 | 47.5 |
| 6.5 | 41.4 |
| 12.5 | 39.8 |
| 10.9 | 39.2 |
| 29.7 | 35.2 |
| 10.6 | 23.1 |

BIC free acid Form X was characterized by XRPD and DSC. The XRPD of BIC free acid Form X is characterized by sharp reflections, indicating crystallinity **(****FIG. 14****).** Characteristic peaks from the XRPD diffractogram are summarized in **Table 9.** The DSC of the solid shows a sharp endotherm at about 213 °C **(****FIG. 15****).** BIC free acid Form X is a polymorph isolated by heating BIC free acid Form IX to 210 °C. This heating step may also produce BIC free acid Form III, but Form III melts at about 203 °C and Form X melts at about 213 °C. In one experiment, the DSC was used to prepare BIC free acid Form X by heating BIC FA Form IX to about 210 °C **(****FIG. 16****).** In this case a broad endothermic event with onset at about 149 °C is followed by a broad exothermic event at about 172 °C. A small endothermic event at about 202 °C is taken as the melt of BIC free acid Form III.

**Table 9. Characteristic XRPD peaks observed in the XRPD Diffractogram of BIC Form X.**

| Tier One | | Tier Two | | Tier Three | |
|---|---|---|---|---|---|
| Position [°2Theta] | Relative Intensity [%] | Position [°2Theta] | Relative Intensity [%] | Position [°2Theta] | Relative Intensity [%] |
| 5.3 | 100.0 | 17.9 | 11.3 | 23.5 | 4.9 |
| 10.6 | 18.1 | 18.6 | 6.1 | 24.1 | 4.9 |
| 14.3 | 9.5 | 18.9 | 8.0 | 24.6 | 4.8 |
| 15.9 | 12.4 | 19.5 | 11.0 | | |
| 22.1 | 12.5 | | | | |

### Example 8. Development of BIC LAI Formulations using PLGA as Hydrophilic Carrier

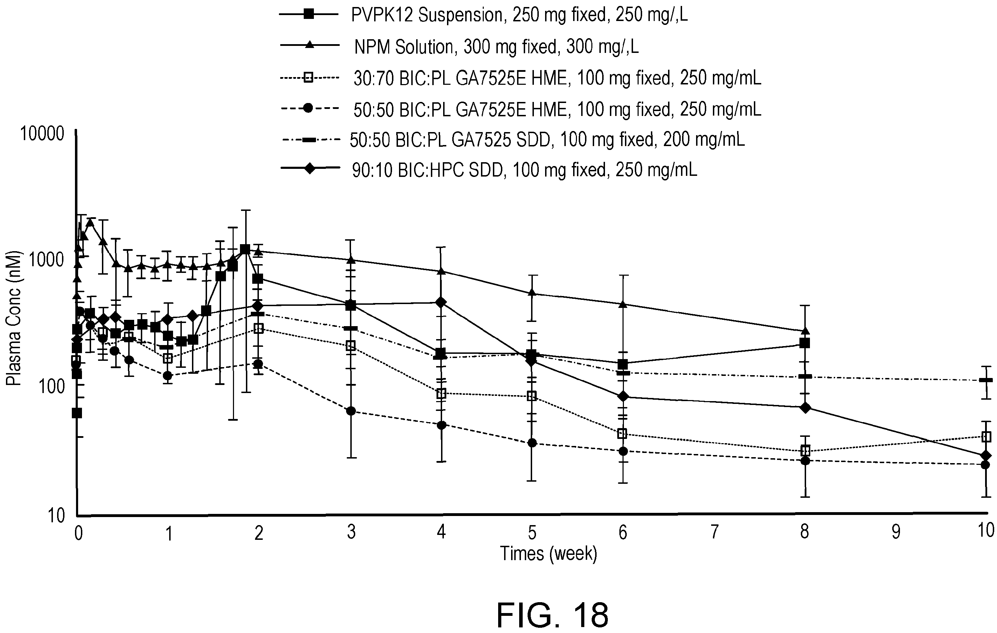

PLGA was evaluated as the hydrophilic carrier in the preparation of long-acting injectable (LAI) formulation of BIC FA Form III. Four separate approaches were considered for formulating BIC FA Form III with PLGA: oil in water emulsion (O/W), solid in oil in water emulsion (S/O/W), hot melt extrusion (HME), and spray dry dispersion (SDD). The formulations were characterized by methods including XRPD, DSC, TGA, and *in vitro* dissolution. A subset of the formulations were evaluated for their pharmacokinetics in a dog animal model as described in Example 1, and the PK profile curves in dog are shown in **FIG. 18****.** The experimental parameters of the XRPD and DSC methods can be found under Example 7. The experimental parameters of the *in vitro* dissolution method are listed below.

*In vitro* dissolution testing was carried out using an automated Distek Model 2500 or Distek Symphony 7100 USP 2 (Paddle) dissolution apparatus. The dissolution media was either 0.5% (wt/wt) SLS in 50 mM sodium citrate buffer, pH 5.5 or 1% (wt/wt) SLS in 50 mM sodium citrate buffer, pH 5.5, and 1000 mL of the dissolution media was used for each run. The speed of the paddle was set at 50 rpm, and the temperature of the dissolution vessels was maintained at 37 °C. The liquid suspension formulations containing BIC were filled into an appropriately sized (size 2, size 1, or larger) gelatin (Povidone K12, PLGA7525E HME 30% DL and PLGA7525E SDD 50% DL formulations) or HPMC (all other formulations) capsules for dissolution testing. 3 mL aliquots were sampled at 0 (initial), 15 min, 30 min, 45 min, 60 min, 90 min, 120 min, 150 min, 180 min, 210 min, and 240 min. The dissolution samples were filtered using 0.2 µm nylon filters prior to analysis. The concentration of BIC in the dissolution samples was determined using the HPLC method as described below.

### HPLC Conditions

*Column:* Waters Acquity BEH C18 column (50 X 2.1 mm, 1.7 µm)
*Mobile phases:*
Mobile phase A: 100:0.1 Water: Formic Acid
Mobile phase B: 100:0.1 Acetonitrile: Formic Acid

*HPLC pumps:* Waters Acquity UPLC system with sample organizer
Injection volume: 5 µL
*Detection:* UV at 260 nm
*Column temperature:* 60 °C
*HPLC elution program:*

| **Time (min)** | **Flow Rate (mL/min)** | **Mobile Phase A (%)** | **Mobile Phase B (%)** |
|---|---|---|---|
| 0.00-0.50 | 0.60 | 95 | 5 |
| 0.50-3.00 | 0.60 | 95 to 0 | 5 to 100 |
| 3.00-3.50 | 0.60 | 0 | 100 |
| 3.50-4.00 | 0.60 | 0 to 95 | 100 to 5 |
| 4.00-6.00 | 0.60 | 95 | 5 |

### a) Oil in Water (O/W) Emulsion

O/W emulsions were formed by combining dichloromethane (DCM), PLGA7525 or PLGA5050 (Resomer RG 753 S or Resomer RG 503 S), and BIC Form III to form a solvent phase (4 mL) that was combined with an aqueous phase (1% polyvinyl alcohol in water, 16 mL). The combination was mixed via vortex mixing for 60 seconds at 2000 rpm and excess solvent was removed by rotovap at 35 °C. The particle size of BIC used when preparing the emulsions was 2 µm. A summary of the composition components is provided in **Table 10.** Phase separation and batch-to-batch inconsistency during scale-up were observed for the BIC / PGLA O/W emulsion formulations.

**Table 10. Composition of BIC III FA in PLGA Oil in Water Emulsion Formulation**

| **Formulation No.** | **PLGA L/G ratio** | **BIC wt% in formulation** | **wt% solids in DCM** | **Final Particle Size (Dᵥₒₗ, µm)** |
|---|---|---|---|---|
| 1 | 75/25 | 30 wt% | 20 | 101 |
| 2 | 75/25 | 32 wt% | 10 | 79 |
| 3 | 75/25 | 32 wt% | 5 | 50 |
| 4 | 75/25 | 32 wt% | 2.5 | 30 |
| 5 | 75/25 | 48 wt% | 10 | 73 |
| 6 | 75/25 | 72 wt% | 10 | 60 |
| 7 | 50/50 | 30 wt% | 10 | 78 |

### b) Solids in Oil in Water (S/O/W) Emulsions

S/O/W suspensions were formed by combining micronized BIC Form III (~2µm), ethyl acetate (amount), PLGA (amount/type), and 1 wt% Pluronic F127 (Poloxamer 407). The combination was mixed using vortex mixer for 60 seconds at 2000 rpm and excess solvent was removed using a rotovap at 35 °C. The target concentration of solids in these compositions was 100 mg/ml. The wt% of BIC in the formulation obtained in three S/O/W emulsion preparations is summarized in **Table 11.**

The crystallinity of the BIC drug substance was conserved in these formulations, but low encapsulation efficiency (particularly at high concentrations) was the limiting factor. In addition, the formulations demonstrated burst release at all concentrations as shown by *in vitro* dissolution testing, limiting their applicability as a long-acting product.

**Table 11. Final concentration of BIC (wt%) in PILGA S/O/W Emulsions**

| **Formulation No.** | **PLGA L/G ratio** | **BIC wt% in final formulation** |
|---|---|---|
| 1 | 75/25 | 28 |
| 2 | 75/25 | 41 |
| 3 | 75/25 | 50 |

### c) Spray Dry Dispersion (SDD)

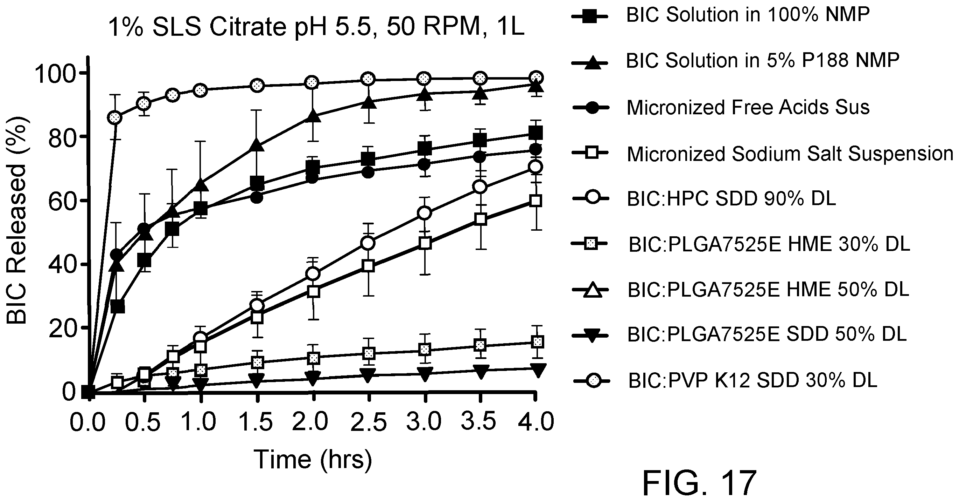

Spray dried emulsions of BIC in PLGA, povidone (PVP), and hydroxypropylcellulose (HPC) were prepared using a Buchi spray dryer. The solvent stream had a concentration of 50 mg/mL of total solids in the stream with a target of 30% BIC loading. The feed rate was 4.5 mL/min (6 g/min for DCM solvent, 4 g/min for ethyl acetate solvent, and 4-6 g/min for acetone). The inlet temperature was 60 °C and the outlet temperature was 38 °C. DCM or acetone as the solvent resulted in a dissolved BIC where ethyl acetate resulted in a suspension of micronized BIC. The resulting formulations were largely amorphous. The formulations demonstrated significant burst release in *in vitro* dissolution testing **(****FIG. 17****).** The spray dried BIC alone or BIC-PLGA formulations were shown to be amorphous by XRPD. The spray drying process produced a phase-separated BIC:PLGA 50:50 dispersion, as indicated by two glass transition events in the DSC thermograms. Several vehicles were evaluated for dosing BIC:PLGA7525 SDD formulations **(Table 12),** where the 0.1% HPMC, E4M with 0.1% Tween 80 in 99.8% PBS provided a homogeneous suspension for up to 2 hours upon preparation, and was selected as the vehicle for dosing BIC:PLGA SDD in a dog animal model. PK data from the dog study are shown in **FIG. 18****.**

**Table 12. Vehicles evaluated for dosing BIC:PLGA7525 SDD formulations**

| Formulation | Vehicle Tested | Visual Observations |
|---|---|---|
| A | 0.5% HPMC E4M, 0.5% Tween 80, 99% PBS | "chunky", inhomogeneous |
| B | 0.5% HPMC E4M, 0.75% Tween 80, 98.75% PBS | "chunky", inhomogeneous |
| C | 0.5% HPMC E4M, 0.5% Tween 20, 99% PBS | "chunky", inhomogeneous |
| D | 0.1% HPMC E4M, 0.1% Tween 80, 99.8% PBS | Homogeneous for 2 h at room temperature |
| E | 0.25% HPMC E4M, 0.25% Tween 80, 99.5% PBS | "chunky", inhomogeneous |

Additional BIC SDD prototype formulations containing three polymers: Povidone K12, Povidone K17, and HPC were also prepared and evaluated. All three SDD formulations were prepared at 30% drug loading (DL), where the Povidone K12 and Povidone K17 formulations were prepared at 5% solids in acetone and the HPC formulation was prepared at 3.4% (wt/wt) solids in acetone. The SDD formulations were characterized by XRPD, TGA and DSC, and all three spray dried SDD were shown to be amorphous by XRPD. Two glass transition events were observed in the DSC thermograms of all three SDD formulations.

Several vehicles were evaluated for dosing BIC:Povidone K12, BIC:Povidone K17, and BIC:HPC SDD formulations **(Table 13).** Based on the observations, the following vehicles were selected for dosing the SDD formulations in animal studies: 5% Poloxamer P188 in 1X PBS for HPC SDD formulation, 1X PBS for Povidone K12 SDD formulation, and 0.5% HPMC E4M, 0.5% Tween 80, 1X PBS for the Povidone K17 SDD formulation.

**Table 13. Vehicles evaluated for dosing BIC:Povidone K12, BIC:Povidone K17, and BIC:HPC SDD formulations**

| Formulation | Polymer | Vehicle Tested | Observations |
|---|---|---|---|
| A | HPC | 250 mg/mL SDD in 5% Poloxamer P188, 1X PBS | Poor flow with some clumps initially; easily syringeable through 18G needle; not syringeable through 21G needle |
| B | HPC | 250 mg/mL SDD in 0.5% HPMC E4M, 0.5% Tween 80, 1X PBS | Poor flow with some clumps initially; more viscous than Formulation A |
| C | HPC | 250 mg/mL SDD in 1% Tween 80 in water | Poor flow with some clumps initially; similar viscosity as Formulation B |
| D | HPC | 200 mg/mL SDD in 5% Poloxamer P188 in 1X PBS | Good flow, less viscous than Formulation A; easily syringeable through 21G needle |
| E | Povidone K12 | 250 mg/mL SDD in 1X PBS | Slow flow with some clumps initially; viscous, foamy; easily syringeable through 21G needle |
| F | Povidone K17 | 250 mg/mL SDD in 1X PBS | Very viscous and forms gel; foamy and clumpy; not tested for syringeability due to viscosity |
| G | Povidone K17 | 250 mg/mL SDD in 0.5% HPMC E4M, 0.5% Tween 80, 1X PBS | Flows slowly, but less foamy and viscous than Formulation F; easily syringeable through 21G needle |

The BIC-HPC SDD, 90% drug load did not show high burst release **(****FIG. 17****)** and was further evaluated in dog model *in vivo* **(****FIG. 18****).**

### d) Hot Melt Extrusion (HME)

BIC drug substance was incorporated into various PLGAs through hot melt extrusion using a Haake hot melt extruder. Experiments were performed at a 5-g scale where BIC FA Form III was intimately mixed with PLGA prior to HME. Extrusion was performed at 110 °C (below BIC Tₘ) and 210 °C (above BIC Tₘ) using various PLGAs where the ratios of the lactic acid and glycolic acid monomer (L/G ratio) were varied. The resulting material was milled in an Ika^{®} tube mill at 25,000 rpm, 10s interval, 2 min at a time and then passed through a 100-mesh sieve (150 µm). A summary of the BIC FA Form III-PLGA HME formulations is provided in **Table 14.** As characterized using XRPD, the BIC drug substance was determined to be amorphous in all of the resulting HME formulations.

The BIC/PLGA7525 HME, 30 and 50% drug load were evaluated by *in vitro* dissolution **(****FIG. 17****)** and did not show fast release and therefore were further evaluated in dog PK model **(****FIG. 18****).**

**Table 14. BIC FA Form III-PLGA HME Formulations**

| **Formulation No.** | **PLGA L/G ratio** | **BIC wt% in final formulation** | **Extrusion Temp (°C)** |
|---|---|---|---|
| 1 | 75/25 | 30% | 110 |
| 2 | 75/25 | 30% | 210 |
| 3 | 50/50 | 30% | 210 |
| 4 | 75/25 | 50% | 210 |
| 5 | 75/25 | 70% | 210 |

It should be appreciated that certain features of the invention, which are, for clarity, described in the context of separate embodiments, can also be provided in combination in a single embodiment. Conversely, various features of the invention which are, for brevity, described in the context of a single embodiment, can also be provided separately or in any suitable subcombination.

## Claims

1. A pharmaceutical composition consisting of:
| **Component** | **% w/w** |
|---|---|
| bictegravir, free acid | 18-28 |
| povidone K-12 | 1.0-2.0 |
| polysorbate 20 | 0.3-0.5 |
| sodium chloride | 0.5-0.7 |
| sodium phosphate buffer | 0.1-0.3 |
| water | 70-80 |

2. The pharmaceutical composition of claim 1, consisting of:
| **Component** | **% w/w** |
|---|---|
| bictegravir, free acid | 23.6 |
| povidone K-12 | 1.5 |
| polysorbate 20 | 0.4 |
| sodium chloride | 0.6 |
| sodium phosphate buffer | 0.2 |
| water | 73.7 |

3. The pharmaceutical composition of any one of claims 1-2, wherein the pharmaceutical composition is for injection.

4. The pharmaceutical composition of any one of claims 1-3, wherein the pharmaceutical composition is for subcutaneous or intramuscular administration.

5. A pharmaceutical composition of any one of claims 1-4 for use in a method for treating or preventing an HIV infection.

6. A pharmaceutical composition of any one of claims 1-4, for use in a method for treating or preventing an HIV infection in a human, wherein the method comprises administering to the human the pharmaceutical composition of any one of claims 1-4 by injection.

7. The pharmaceutical composition for use according to claim 6, wherein the pharmaceutical composition is administered not more frequently than once every four weeks (Q4W).

8. The pharmaceutical composition for use according to claim 6 or 7, wherein the pharmaceutical composition is administered once every four weeks (Q4W).

9. The pharmaceutical composition for use according to claim 6 or 7, wherein the pharmaceutical composition is administered once every eight weeks (Q8W).

10. The pharmaceutical composition for use according to claim 6 or 7, wherein the pharmaceutical composition is administered once every twelve weeks (Q12W).

11. The pharmaceutical composition for use according to any one of claims 6-10, wherein
(a) the pharmaceutical composition comprises from about 100 mg to about 2500 mg of bictegravir; or
(b) the pharmaceutical composition comprises from about 200 mg to about 1000 mg of bictegravir; or
(c) the pharmaceutical composition comprises from about 400 mg to about 600 mg of bictegravir; or
(d) the pharmaceutical composition comprises about 550 mg of bictegravir.

12. The pharmaceutical composition for use according to any one of claims 6-10, wherein the human is infected with HIV.

13. The pharmaceutical composition for use according to claim 12, wherein the human has an HIV-1 RNA copy number of less than about 50 copies/mL.

## Patentansprüche

1. Pharmazeutische Zusammensetzung, bestehend aus:
| **Komponente** | **Gew.-%** |
|---|---|
| Bictegravir, freie Säure | 18-28 |
| Povidon K-12 | 1,0-2,0 |
| Polysorbat 20 | 0,3-0,5 |
| Natriumchlorid | 0,5-0,7 |
| Natriumphosphatpuffer | 0,1-0,3 |
| Wasser | 70-80 |

2. Pharmazeutische Zusammensetzung nach Anspruch 1, bestehend aus:
| **Komponente** | **Gew.-%** |
|---|---|
| Bictegravir, freie Säure | 23, 6 |
| Povidon K-12 | 1, 5 |
| Polysorbat 20 | 0,4 |
| Natriumchlorid | 0, 6 |
| Natriumphosphatpuffer | 0, 2 |
| Wasser | 73, 7 |

3. Pharmazeutische Zusammensetzung nach einem der Ansprüche 1-2, wobei die pharmazeutische Zusammensetzung für die Injektion bestimmt ist.

4. Pharmazeutische Zusammensetzung nach einem der Ansprüche 1-3, wobei die pharmazeutische Zusammensetzung für die subkutane oder intramuskuläre Verabreichung bestimmt ist.

5. Pharmazeutische Zusammensetzung nach einem der Ansprüche 1-4 zur Verwendung bei einem Verfahren zur Behandlung oder Vorbeugung einer HIV-Infektion.

6. Pharmazeutische Zusammensetzung nach einem der Ansprüche 1-4, zur Verwendung bei einem Verfahren zur Behandlung oder Vorbeugung einer HIV-Infektion bei einem Menschen, wobei das Verfahren Verabreichen der pharmazeutischen Zusammensetzung nach einem der Ansprüche 1-4 an den Menschen per Injektion umfasst.

7. Pharmazeutische Zusammensetzung zur Verwendung gemäß Anspruch 6, wobei die pharmazeutische Zusammensetzung nicht häufiger als einmal alle vier Wochen (Q4W) verabreicht wird.

8. Pharmazeutische Zusammensetzung zur Verwendung gemäß Anspruch 6 oder 7, wobei die pharmazeutische Zusammensetzung einmal alle vier Wochen (Q4W) verabreicht wird.

9. Pharmazeutische Zusammensetzung zur Verwendung gemäß Anspruch 6 oder 7, wobei die pharmazeutische Zusammensetzung einmal alle acht Wochen (Q8W) verabreicht wird.

10. Pharmazeutische Zusammensetzung zur Verwendung gemäß Anspruch 6 oder 7, wobei die pharmazeutische Zusammensetzung einmal alle zwölf Wochen (Q12W) verabreicht wird.

11. Pharmazeutische Zusammensetzung zur Verwendung gemäß einem der Ansprüche 6-10, wobei
(a) die pharmazeutische Zusammensetzung etwa 100 mg bis etwa 2500 mg Bictegravir umfasst; oder
(b) die pharmazeutische Zusammensetzung etwa 200 mg bis etwa 1000 mg Bictegravir umfasst; oder
(c) die pharmazeutische Zusammensetzung etwa 400 mg bis etwa 600 mg Bictegravir umfasst; oder
(d) die pharmazeutische Zusammensetzung etwa 550 mg Bictegravir umfasst.

12. Pharmazeutische Zusammensetzung zur Verwendung gemäß einem der Ansprüche 6-10, wobei der Mensch mit HIV infiziert ist.

13. Pharmazeutische Zusammensetzung zur Verwendung gemäß Anspruch 12, wobei der Mensch eine HIV-1-RNA-Kopienzahl von weniger als etwa 50 Kopien/ml aufweist.

## Revendications

1. Composition pharmaceutique constituée de :
| **Composant** | **% p/p** |
|---|---|
| bictégravir, acide libre | 18-28 |
| povidone K-12 | 1,0-2,0 |
| polysorbate 20 | 0,3-0,5 |
| chlorure de sodium | 0,5-0,7 |
| tampon phosphate de sodium | 0,1-0,3 |
| eau | 70-80 |

2. Composition pharmaceutique selon la revendication 1, constituée de :
| **Composant** | **% p/p** |
|---|---|
| bictégravir, acide libre | 23, 6 |
| povidone K-12 | 1,5 |
| polysorbate 20 | 0,4 |
| chlorure de sodium | 0, 6 |
| tampon phosphate de sodium | 0, 2 |
| eau | 73, 7 |

3. Composition pharmaceutique selon l'une quelconque des revendications 1 à 2, dans laquelle la composition pharmaceutique est pour injection.

4. Composition pharmaceutique selon l'une quelconque des revendications 1 à 3, dans laquelle la composition pharmaceutique est destinée à une administration sous cutanée ou intramusculaire.

5. Composition pharmaceutique selon l'une quelconque des revendications 1 à 4 destinée à être utilisée dans un procédé de traitement ou de prévention d'une infection par le VIH.

6. Composition pharmaceutique selon l'une quelconque des revendications 1 à 4, destinée à être utilisée dans un procédé de traitement ou de prévention d'une infection par le VIH chez un humain, dans laquelle le procédé comprend l'administration à l'humain de la composition pharmaceutique selon l'une quelconque des revendications 1 à 4 par injection.

7. Composition pharmaceutique pour utilisation selon la revendication 6, dans laquelle la composition pharmaceutique est administrée pas plus fréquemment qu'une fois toutes les quatre semaines (Q4W).

8. Composition pharmaceutique pour utilisation selon la revendication 6 ou 7, dans laquelle la composition pharmaceutique est administrée une fois toutes les quatre semaines (Q4W).

9. Composition pharmaceutique pour utilisation selon la revendication 6 ou 7, dans laquelle la composition pharmaceutique est administrée une fois toutes les huit semaines (Q8W).

10. Composition pharmaceutique pour utilisation selon la revendication 6 ou 7, dans laquelle la composition pharmaceutique est administrée une fois toutes les douze semaines (Q12W).

11. Composition pharmaceutique destinée à être utilisée selon l'une quelconque des revendications 6 à 10, dans laquelle
(a) la composition pharmaceutique comprend d'environ 100 mg à environ 2 500 mg de bictégravir ; ou
(b) la composition pharmaceutique comprend d'environ 200 mg à environ 1 000 mg de bictégravir ; ou
(c) la composition pharmaceutique comprend d'environ 400 mg à environ 600 mg de bictégravir ; ou
(d) la composition pharmaceutique comprend environ 550 mg de bictégravir.

12. Composition pharmaceutique pour utilisation selon l'une quelconque des revendications 6 à 10, dans laquelle l'humain est infecté par le VIH.

13. Composition pharmaceutique destinée à être utilisée selon la revendication 12, dans laquelle l'humain a un nombre de copies d'ARN du VIH-1 inférieur à environ 50 copies/mL.
